# EUROPEAN PATENT SPECIFICATION

(11) **EP 2 892 910 B1**
(45) Date of publication and mention of the grant of the patent: **07.09.2016**
(21) Application number: 13763430.9
(22) Date of filing: 10.09.2013
(51) Int. Cl.: C07J 63/00, A61K 31/56, A61P 29/00

(54) **GLYCYRRHETINIC ACID DERIVATIVES WITH ANTI-INFLAMMATORY ACTIVITY**
GLYCYRRHETINSÄUREDERIVATE MIT ENTZÜNDUNGSHEMMENDER WIRKUNG
DÉRIVÉS D'ACIDE GLYCYRRHÉTINIQUE AVEC ACTIVITÉ ANTI-INFLAMMATOIRE

(30) Priority: 10.09.2012 US 201261699123 P
(43) Date of publication of application: 15.07.2015
(73) Proprietor: AbbVie Inc., North Chicago, IL 60064 (US)
(72) Inventor: WAGNER, Rolf, Antioch, Illinois 60002 (US); CHEN, Hui-Ju, Grayslake, Illinois 60030 (US); SHANLEY, Jason, Chicago, Illinois 60622 (US); BOGDAN, Andrew, Evanston, Illinois 60202 (US); MARJANOVIC, Jasmina, Chicago, Illinois 60640 (US); WANG, Xiu, Green Oaks, Illinois 60048 (US); DONNELLY-ROBERTS, Diana, Gurnee, Illinois 60031 (US)
(74) Representative: Modiano, Micaela Nadia
(86) International application number: PCT/US2013/059008
(87) International publication number: WO 2014/040052

(56) References cited:
- WO-A1-2009/129545
- WO-A1-2009/129546
- WO-A1-2009/129548
- WO-A1-2012/125488
- WO-A2-2004/064723
- RAWINDRA GAWARE ET AL: "Synthesis of new glycyrrhetinic acid derived ring A azepanone, 29-urea and 29-hydroxamic acid derivatives as selective 11-hydroxysteroid dehydrogenase 2 inhibitors", BIOORGANIC & MEDICINAL CHEMISTRY, PERGAMON, GB, vol. 19, no. 6, 3 February 2011 (2011-02-03), pages 1866-1880, XP028176790, ISSN: 0968-0896, DOI: 10.1016/J.BMC.2011.02.005 [retrieved on 2011-02-11]
- YUAN GAO ET AL: "The Synthesis of Glycyrrhetinic Acid Derivatives Containing A Nitrogen Heterocycle and Their Antiproliferative Effects in Human Leukemia Cells", MOLECULES, vol. 15, no. 6, 21 June 2010 (2010-06-21), pages 4439-4449, XP55108542, DOI: 10.3390/molecules15064439
- CHADALAPAKA G ET AL: "Structure-dependent inhibition of bladder and pancreatic cancer cell growth by 2-substituted glycyrrhetinic and ursolic acid derivatives", BIOORGANIC & MEDICINAL CHEMISTRY LETTERS, PERGAMON, AMSTERDAM, NL, vol. 18, no. 8, 15 April 2008 (2008-04-15) , pages 2633-2639, XP022606362, ISSN: 0960-894X, DOI: 10.1016/J.BMCL.2008.03.031 [retrieved on 2008-03-14]
- HONDA TADASHI ET AL: "Novel synthetic oleanane and ursane triterpenoids with various enone functionalities in ring A as inhibitors of nitric oxide production in mouse macrophages", JOURNAL OF MEDICINAL CHEMISTRY, AMERICAN CHEMICAL SOCIETY, US, vol. 43, no. 9, 4 May 2000 (2000-05-04), pages 1866-1877, XP002541099, ISSN: 0022-2623, DOI: 10.1021/JM000008J [retrieved on 2000-04-14]
- DATABASE CA [Online] CHEMICAL ABSTRACTS SERVICE, COLUMBUS, OHIO, US; ZHAO, YU ET AL: "Oleanane type pentacyclic triterpene derivatives, their preparation and application", XP002721905, retrieved from STN Database accession no. 2006:669587 & CN 1 796 401 A (ZHEJIANG HISUN NATURELITE PHARMACEUTICAL RESEARCH AND DEVELOPMENT CO.,) 5 July 2006 (2006-07-05)
- RAN YOU ET AL: "Discovery of a Potential Anti-Inflammatory Agent: 3-Oxo-29-noroleana-1,9(11),12-trien-2,20-d icarbonitrile", JOURNAL OF MEDICINAL CHEMISTRY, vol. 56, no. 5, 14 March 2013 (2013-03-14) , pages 1984-1995, XP55108604, ISSN: 0022-2623, DOI: 10.1021/jm301652t

## Description

### FIELD OF THE INVENTION

The present invention relates generally to the fields of biology and medicine. More particularly, it concerns compounds, compositions and methods for the treatment and prevention of diseases such as those associated with oxidative stress and inflammation.

### BACKGROUND

The anti-inflammatory and anti-proliferative activity of the naturally occurring triterpenoid, oleanolic acid, has been improved by chemical modifications. For example, 2-cyano-3,12-diooxooleana-1,9(11)-dien-28-oic acid (CDDO) and related compounds have been developed (Honda *et al.,* 1997; Honda *et al.,* 1998; Honda *et al.,* 1999; Honda *et al.,* 2000a; *Honda et al.,* 2000b; Honda, *et al.,* 2002; Suh *et al.* 1998; Suh *et al.,* 1999; Place *et al.,* 2003; Liby *et al.,* 2005) and evaluated in clinical trials for the treatment of cancer, diabetic nephropathy and chronic kidney disease.

Synthetic triterpenoid analogs of oleanolic acid have also been shown to be inhibitors of cellular inflammatory processes, such as the induction by IFN-γ of inducible nitric oxide synthase (iNOS) and of COX-2 in mouse macrophages. See Honda *et al.* (2000a); Honda *et al.* (2000b), and Honda *et al.* (2002). Synthetic derivatives of another triterpenoid, betulinic acid, have also been shown to inhibit cellular inflammatory processes, although these compounds have been less extensively characterized (Honda *et al.,* 2006). The pharmacology of these synthetic triterpenoid molecules is complex. Compounds derived from oleanolic acid have been shown to affect the function of multiple protein targets and thereby modulate the activity of several important cellular signaling pathways related to oxidative stress, cell cycle control, and inflammation *(e.g.,* Dinkova-Kostova *et al.,* 2005; Ahmad *et al.,* 2006; Ahmad *et al.,* 2008; Liby *et al.,* 2007a). Derivatives of betulinic acid, though they have shown comparable anti-inflammatory properties, also appear to have significant differences in their pharmacology compared to OA-derived compounds (Liby *et al.,* 2007b). Given that the biological activity profiles of known triterpenoid derivatives vary, and in view of the wide variety of diseases that may be treated or prevented with compounds having potent antioxidant and anti-inflammatory effects, and the high degree of unmet medical need represented within this variety of diseases, it is desirable to synthesize new compounds with diverse structures that may have improved biological activity profiles for the treatment of one or more indications.

While oleanolic, ursolic and betulinic acid derivatives have been described as useful antioxidants, glycyrrhetinic acid derived compounds have never been regarded as useful agents to ameliorate oxidative stress, particularly through the Keap-1/Nrf-2 pathway. This unique triterpenoid core has now been found to yield a wide variety of potent compounds that are useful in this regard, and it is the object of this disclosure to describe this discovery and invention in detail.

### SUMMARY OF THE INVENTION

The present disclosure provides novel synthetic triterpenoid derivatives, with anti-inflammatory and/or antioxidant properties, pharmaceutical compositions, and methods for their manufacture, and methods for their use.

In one aspect, there are provided compounds of the Formula: 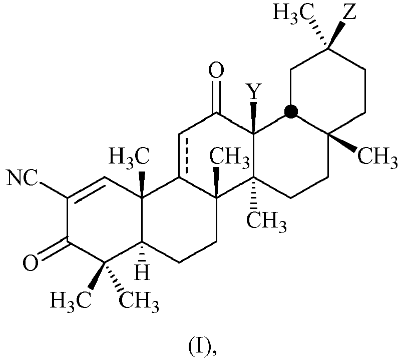 or a pharmaceutically acceptable salt thereof, wherein
----- is a single or double bond;
Y is -OR¹; and
Z is -CO₂H, -CO₂R² , -C(O)NR³R⁴, -C(O)G¹, -NR³C(O)R⁵, -NR³S(O)₂R⁵, -CH₂NR³C(O)R⁵, -CH₂NR³S(O)₂R⁵, -NR³R⁶, or -CH₂NR³R⁶;
   or
Y is hydrogen; and
Z is -C(O)NR³R¹⁴, -C(O)G¹, -NR³C(O)R⁵, -NR³S(O)₂R⁵, -CH₂NR³C(O)R⁵, -CH₂NR³S(O)₂R⁵, -NR³R⁶, or -CH₂NR³R⁶;
R¹ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, -G^{2b}, or C(O)R^{1a};
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁴ is hydrogen, C₁-C₈-alkyl, C₁-C₈-alkenyl, C₁-C₈-alkynyl, C₁-C₈-haloalkyl,
   C₁-C₆-alkoxy-C₁-C₆-alkyl, di(C₁-C₆-alkoxy)-C₁-C₆-alkyl, C₁-C₆-cyanoalkyl,
   C₁-C₆-hydroxyalkyl, C₁-C₆-di(hydroxy)alkyl, -(CR^{4a}R^{4b})ₘ-N(R³)₂,
   -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b},
   -(CR^{4a}R^{4b})ₘ-C(O)OR², -(CR^{4a}R^{4b})ₘ-CO₂H, -(CR^{4a}R^{4b})ₘ-C(O)N(R³)₂,
   -(CR^{4a}R^{4b})ₘ-C(O)N(OH)R³, or -SO₂R^{1a};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R⁵⁶)ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G⁵⁶, -N(R^{1b})-G^{5b}, -(CR^{5a}R^{5b})ₙ-G^{5b}, or -(CR^{5a}R^{5b})ₙ-NHC(O)G^{5b};
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR^{4a}R^{4b}₎ₘ-G^{4b}, -(CR^{4a}R^{4b})ₘ-C(O)OR², or -(CR^{4a}R^{4b})ₘ-C(O)N(R³)₂;
R¹⁴ is C₁-C₈-alkenyl, C₁-C₈-alkynyl, C₁-C₈-haloalkyl, C₁-C₆-alkoxy-C₁-C₆-alkyl, C₁-C₆-alkoxy-C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR4^{a}R4^{b})ₘ-C(O)N(R³)₂, -SO₂R^{1a}, or
   -(CR^{4a}R^{4b})ₘ-SO₂R^{1a};
R^{1a} is, at each occurrence, independently C₁-C₆-alkyl, C₁-C₆-haloalkyl, C₃-C₈-cycloalkyl, or C₁-C₈-cycloalkyl-C₁-C₃-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, cyano, hydroxy, oxo, C₁-C₆-alkoxy, C₁-C₈-alkyl, C₁-C₈-alkenyl, C₁-C₈-alkynyl, C₁-C₈-haloalkyl, C₁-C₆-alkoxy-C₁-C₆-alkyl, C₁-C₆-cyanoalkyl, C₁-C₆-hydroxyalkyl, -CO₂R^{1a}, or -C(O)N(R³)₂;
G^{2a} is C₁-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(OMR^{4d}), C₁-C₄-cyanoalkyl, C₁-C₄-haloalkyl, -G^{a}, -(CR^{4a}R^{4b})ₘ-G^{a}, or -O-G^{a};
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen;
m, at each occurrence, independently is 1, 2, 3, 4, or 5; and
n, at each occurrence, independently is 1, 2, 3, 4, or 5.

In some aspects, there are provided pharmaceutical compositions comprising one or more of the above described compounds and an excipient. In other aspects there are provided compounds for use in methods of treating and/or preventing a disease or a disorder in patients in need thereof, comprising administering to such patients one or more of the above described compounds in an amount sufficient to treat and/or prevent the disease or disorder. In some embodiments, the disease has an inflammatory component. In some aspects, there are provided uses of one of more of the above described compounds in the manufacture of a medicament for the treatment of a disease with an inflammatory component. In some aspects, there are provided compounds as described above for the use in the treatment of a disease with an inflammatory component. In some aspects, there are provided compositions comprising one or more of the compounds described above for the use in the treatment of a disease with an inflammatory component.

The compounds, compositions comprising the compounds, and methods for treating or preventing conditions and disorders by administering the compounds are further described herein.

### DETAILED DESCRIPTION OF THE INVENTION

The present invention includes compounds of Formula (I): 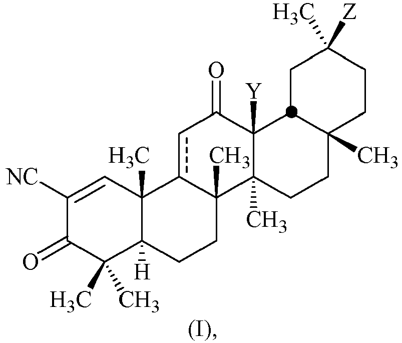 and salts thereof, wherein Y and Z are as defined above in the Summary of the Invention. The invention further includes compositions comprising such compounds and such compounds for use in methods for treating conditions and disorders using such compounds and compositions.

In various embodiments, the present invention provides at least one variable that occurs more than one time in any substituent or in the compound of the invention or any other formulae herein. Definition of a variable on each occurrence is independent of its definition at another occurrence. Further, combinations of substituents are permissible only if such combinations result in stable compounds. Stable compounds are compounds, which can be isolated from a reaction mixture.

### Definitions

As used in the specification and the appended claims, unless specified to the contrary, the following terms have the meaning indicated:

The term "alkenyl" as used herein, means a straight or branched hydrocarbon chain containing from 2 to 10 carbons and containing at least one carbon-carbon double. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

The term "alkenylene" denotes a divalent group derived from a straight or branched chain hydrocarbon of 2 to 4 carbon atoms and contains at least one carbon-carbon double bond. Representative examples of alkenylene include, but are not limited to, -CH=CH- and -CH₂CH=CH-.

The term "alkoxy" refers to an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkoxyalkoxy" means an alkoxy group, as defined herein, appended to the parent molecular moiety through another alkoxy group, as defined herein. Representative examples of alkoxyalkoxy include, but are not limited to, tert-butoxymethoxy, 2-ethoxyethoxy, 2-methoxyethoxy, and methoxymethoxy.

The term "alkoxyalkoxyalkyl" means an alkoxyalkoxy group, as defined herein, appended to the parent molecular moiety through an alkyl group, as defined herein. Representative examples of alkoxyalkoxyalkyl include, but are not limited to, tert-butoxymethoxymethyl, ethoxymethoxymethyl, (2-methoxyethoxy)methyl, and 2-(2-methoxyethoxy)ethyl.

The term "alkoxyalkyl" refers to an alkoxy group, as defined herein, appended to the parent molecular moiety through an alkyl group, as defined herein. Representative examples of alkoxyalkyl include, but are not limited to, tert-butoxymethyl, 2-ethoxyethyl, 2-methoxyethyl, and methoxymethyl.

The term "di(alkoxy)alkyl" refers to two independent alkoxy groups, as defined herein, appended to the parent molecular moiety through a single alkyl group, as defined herein, wherein each alkoxy group is attached to a different carbon atom of the alkyl group. Representative examples of di(alkoxy)alkyl include, but are not limited to, 1,3-dimethoxypropan-2-yl, 2,3-dimethoxypropanyl, 2,4-dimethoxybutanyl.

The term "alkyl" as used herein, means a straight or branched, saturated hydrocarbon chain containing from 1 to 10 carbon atoms. The term "lower alkyl" or "C₁₋₆ alkyl" means a straight or branched chain hydrocarbon containing 1 to 6 carbon atoms. The term "C₁₋₃-alkyl" means a straight or branched chain hydrocarbon containing 1 to 3 carbon atoms. Representative examples of alkyl include, but are not limited to, methyl, ethyl, n-propyl, iso-propyl, n-butyl, sec-butyl, iso-butyl, *tert*-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

The term "alkylene" denotes a divalent group derived from a straight or branched chain hydrocarbon 1 to 10 carbon atoms. Representative examples of alkylene include, but are not limited to, -CH₂-, -CH₂CH₂-, -CH₂CH₂CH₂-, -CH₂CH₂CH₂CH₂-, and -CH₂CH(CH₃)CH₂-.

The term "alkynyl" as used herein, means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "aryl" as used herein, means phenyl or a bicyclic aryl. The bicyclic aryl is naphthyl, or a phenyl fused to a monocyclic cycloalkyl, or a phenyl fused to a monocyclic cycloalkenyl. Representative examples of the biaryl groups include, but are not limited to, dihydroindenyl, indenyl, naphthyl, dihydronaphthalenyl, and tetrahydronaphthalenyl. The bicyclic aryl is attached to the parent molecular moiety through any carbon atom contained within the bicyclic ring system. The aryl groups of the present invention can be unsubstituted or substituted.

The term "arylalkyl" as used herein, means an aryl group, as defined herein, appended to the parent molecular moiety through an alkylene group, as defined herein. Representative examples of arylalkyl include, but are not limited to, benzyl, 2-phenylethyl, 3-phenylpropyl, and 2-naphth-2-ylethyl.

The term "cyano" as used herein, means a -CN group.

The term "cyanoalkyl" as used herein, means a cyano group, as defined herein, appended to the parent molecular moiety through an alkyl group, as defined herein. Representative examples of cyanoalkyl include, but are not limited to, cyanomethyl, 2-cyanoethyl, and 3-cyanopropyl.

The term "cycloalkyl" or "cycloalkane" as used herein, means a monocyclic, a bicyclic, or a tricyclic cycloalkyl. The monocyclic cycloalkyl is a carbocyclic ring system containing three to eight carbon atoms, zero heteroatoms and zero double bonds. Examples of monocyclic ring systems include cyclopropyl, cyclobutyl, cyclopentyl, cyclohexyl, cycloheptyl, and cyclooctyl. The bicyclic cycloalkyl is a monocyclic cycloalkyl fused to a monocyclic cycloalkyl ring, or a bridged monocyclic ring system in which two non-adjacent carbon atoms of the monocyclic ring are linked by an alkylene bridge containing one, two, three, or four carbon atoms. Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Tricyclic cycloalkyls are exemplified by a bicyclic cycloalkyl fused to a monocyclic cycloalkyl, or a bicyclic cycloalkyl in which two non-adjacent carbon atoms of the ring systems are linked by an alkylene bridge of 1, 2, 3, or 4 carbon atoms. Representative examples of tricyclic-ring systems include, but are not limited to, tricyclo[3.3.1.0^{3,7}]nonane (octahydro-2,5-methanopentalene or noradamantane), and tricyclo[3.3.1.1^{3,7} ]decane (adamantane). The monocyclic, bicyclic, and tricyclic cycloalkyls can be unsubstituted or substituted, and are attached to the parent molecular moiety through any substitutable atom contained within the ring system.

The term "cycloalkylalkyl" as used herein, means a cycloalkyl group appended to the parent molecular moiety through an alkyl group, as defined herein.

The term "cycloalkenyl" or "cycloalkene" as used herein, means a monocyclic or a bicyclic hydrocarbon ring system. The monocyclic cycloalkenyl has four-, five-, six-, seven-or eight carbon atoms and zero heteroatoms. The four-membered ring systems have one double bond, the five-or six-membered ring systems have one or two double bonds, and the seven- or eight-membered ring systems have one, two or three double bonds. Representative examples of monocyclic cycloalkenyl groups include, but are not limited to, cyclobutenyl, cyclopentenyl, cyclohexenyl, cycloheptenyl and cyclooctenyl. The bicyclic cycloalkenyl is a monocyclic cycloalkenyl fused to a monocyclic cycloalkyl group, or a monocyclic cycloalkenyl fused to a monocyclic cycloalkenyl group. The monocyclic or bicyclic cycloalkenyl ring can contain one or two alkylene bridges, each consisting of one, two or three carbon atoms, each linking two non-adjacent carbon atoms of the ring system. Representative examples of the bicyclic cycloalkenyl groups include, but are not limited to, 4,5,6,7-tetrahydro-3a*H*-indene, octahydronaphthalenyl and 1,6-dihydro-pentalene. The monocyclic and bicyclic cycloalkenyl can be attached to the parent molecular moiety through any substitutable atom contained within the ring systems, and can be unsubstituted or substituted.

The term "halo" or "halogen" as used herein, means Cl, Br, I, or F.

The term "haloalkyl" as used herein, means an alkyl group, as defined herein, in which one, two, three, four, five or six hydrogen atoms are replaced by halogen. Representative examples of haloalkyl include, but are not limited to, chloromethyl, 2-fluoroethyl, 2,2,2-trifluoroethyl, trifluoromethyl, difluoromethyl, pentafluoroethyl, 2-chloro-3-fluoropentyl, and trifluoropropyl such as 3,3,3-trifluoropropyl.

The term "heterocycle" or "heterocyclic" as used herein, means a monocyclic heterocycle, a bicyclic heterocycle, or a tricyclic heterocycle. The monocyclic heterocycle is a three-, four-, five-, six-, seven-, or eight-membered ring containing at least one heteroatom independently selected from the group consisting of O, N, and S. The three- or four-membered ring contains zero or one double bond, and one heteroatom selected from the group consisting of O, N, and S. The five-membered ring contains zero or one double bond and one, two or three heteroatoms selected from the group consisting of O, N and S. The six-membered ring contains zero, one or two double bonds and one, two, or three heteroatoms selected from the group consisting of O, N, and S. The seven- and eight-membered rings contains zero, one, two, or three double bonds and one, two, or three heteroatoms selected from the group consisting of O, N, and S. Representative examples of monocyclic heterocycles include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyranyl, pyrazolinyl, pyrazolidinyl, pyrrolinyl, pyrrolidinyl, tetrahydrofuranyl, tetrahydropyranyl, tetrahydropyridinyl, tetrahydrothienyl, thiadiazolinyl, thiadiazolidinyl, 1,2-thiazinanyl, 1,3-thiazinanyl, thiazolinyl, thiazolidinyl, thiomorpholinyl, 1,1-dioxidothiomorpholinyl (thiomorpholine sulfone), thiopyranyl, and trithianyl. The bicyclic heterocycle is a monocyclic heterocycle fused to a phenyl group, or a monocyclic heterocycle fused to a monocyclic cycloalkyl, or a monocyclic heterocycle fused to a monocyclic cycloalkenyl, or a monocyclic heterocycle fused to a monocyclic heterocycle, or a bridged monocyclic heterocycle ring system in which two non adjacent atoms of the ring are linked by an alkylene bridge of 1, 2, 3, or 4 carbon atoms, or an alkenylene bridge of two, three, or four carbon atoms. Representative examples of bicyclic heterocycles include, but are not limited to, benzopyranyl, benzothiopyranyl, chromanyl, 2,3-dihydrobenzofuranyl, 3,4-dihydrobenzothienyl, 2,3-dihydroisoquinolinyl or indolinyl, 2,3-dihydroisoquinolinyl, 1,1-dioxidoisothiazolidinyl, azabicyclo[2.2.1]heptyl (including 2-azabicyclo[2.2.1]hept-2-yl), 2,3-dihydro-1*H-*indolyl, isoindolinyl, octahydro-1*H*-indolyl, octahydrocyclopenta[*c*]pyrrolyl, octahydropyrrolopyridinyl, and tetrahydroisoquinolinyl. Tricyclic heterocycles are exemplified by a bicyclic heterocycle fused to a phenyl group, or a bicyclic heterocycle fused to a monocyclic cycloalkyl, or a bicyclic heterocycle fused to a monocyclic cycloalkenyl, or a bicyclic heterocycle fused to a monocyclic heterocycle, or a bicyclic heterocycle in which two non adjacent atoms of the bicyclic ring are linked by an alkylene bridge of 1, 2, 3, or 4 carbon atoms, or an alkenylene bridge of two, three, or four carbon atoms. Examples of tricyclic heterocycles include, but are not limited to, octahydro-2,5-epoxypentalene, hexahydro-2*H*-2,5-methanocyclopenta[*b*]furan, hexahydro-1*H*-1,4-methanocyclopenta[*c*]furan, aza-adamantane (1-azatricyclo[3.3.1.1^{3,7}]decane), and oxa-adamantane (2-oxatricyclo[3.3. 1.1^{3,7}]decane). The monocyclic, bicyclic, and tricyclic heterocycles are connected to the parent molecular moiety through any carbon atom or any nitrogen atom contained within the rings, and can be unsubstituted or substituted.

The term "heteroaryl" as used herein, means a monocyclic heteroaryl or a bicyclic heteroaryl. The monocyclic heteroaryl is a five- or six-membered ring. The five-membered ring contains two double bonds. The five-membered ring can contain one heteroatom selected from O or S; or one, two, three, or four nitrogen atoms and optionally one oxygen or sulfur atom. The six-membered ring contains three double bonds and one, two, three or four nitrogen atoms. Representative examples of monocyclic heteroaryl include, but are not limited to, furanyl, imidazolyl, isoxazolyl, isothiazolyl, oxadiazolyl, 1,2-oxazolyl, 1,3-oxazolyl, pyridinyl, pyridazinyl, pyrimidinyl, pyrazinyl, pyrazolyl, pyrrolyl, tetrazolyl, thiadiazolyl, 1,3-thiazolyl, thienyl, triazolyl, and triazinyl. The bicyclic heteroaryl consists of a monocyclic heteroaryl fused to a phenyl, or a monocyclic heteroaryl fused to a monocyclic cycloalkyl, or a monocyclic heteroaryl fused to a monocyclic cycloalkenyl, or a monocyclic heteroaryl fused to a monocyclic heteroaryl, or a monocyclic heteroaryl fused to a monocyclic heterocycle. Representative examples of bicyclic heteroaryl groups include, but are not limited to, benzofuranyl, benzothienyl, benzoxazolyl, 2,1,3-benzothiadiazolyl, benzimidazolyl, benzoxadiazolyl, 6,7-dihydro-1,3-benzothiazolyl, furo[3,2-*b*]pyrrolyl, imidazo[1,2-*a*]pyridinyl, indazolyl, indolyl, isoindolyl, isoquinolinyl, naphthyridinyl, pyridoimidazolyl, pyrrolopyridinyl, quinolinyl, thiazolo[5,4-*b*]pyridin-2-yl, thiazolo[5,4-*d*]pyrimidin-2-yl, thienopyridinyl and 5,6,7,8-tetrahydroquinolin-5-yl. The monocyclic and bicyclic heteroaryl groups of the present invention can be substituted or unsubstituted and are connected to the parent molecular moiety through any carbon atom or any nitrogen atom contained within the ring systems.

The term "heteroarylalkyl," as used herein, means a heteroaryl group appended to the parent molecular moiety through an alkyl group, as defined herein.

The term "heteroatom" as used herein, means a nitrogen, oxygen, or sulfur atom.

The term "hydroxyl" or "hydroxy" as used herein, means an -OH group.

The term "hydroxyalkyl" as used herein, means at least one hydroxy group, as defined herein, is appended to the parent molecular moiety through an alkylene group, as defined herein. Representative examples of hydroxyalkyl include, but are not limited to, hydroxymethyl, 2-hydroxyethyl, 3-hydroxypropyl, 2,3-dihydroxypentyl, and 2-ethyl-4-hydroxyheptyl.

The term "di(hydroxy)alkyl" as used herein, means two hydroxy group, as defined herein, are appended to the parent molecular moiety through a single alkylene group, as defined herein, wherein each hydroxy group is attached to different carbon atom of the alkylene group. Representative examples of di(hydroxy)alkyl include, but are not limited to, 2,3-dihydroxypentyl, 2,3-dihydroxypropyl, and 2,4-dihydroxybutyl.

The term "nitro" as used herein, means a -NO₂ group.

The term "nitrogen protecting group" as used herein means those groups intended to protect a nitrogen atom against undesirable reactions during synthetic procedures. Nitrogen protecting groups comprise carbamates, amides, *N*-benzyl derivatives, and imine derivatives. Preferred nitrogen protecting groups are acetyl, benzoyl, benzyl, benzyloxycarbonyl (Cbz), formyl, phenylsulfonyl, pivaloyl, *tert*-butoxycarbonyl (Boc), *tert*-butylacetyl, trifluoroacetyl, and triphenylmethyl (trityl). Nitrogen-protecting groups are appended onto primary or secondary amino groups by reacting the compound that contains the amine group with base, such as triethylamine, and a reagent selected from an alkyl halide, an alkyl triflate, a dialkyl anhydride, for example as represented by an alkyl anhydride (alkyl-OC=O)₂O, a diaryl anhydride, for example as represented by (aryl-OC=O)₂O, an acyl halide, an alkylchloroformate, or an alkylsulfonylhalide, an arylsulfonylhalide, or halo-CON(alkyl)₂, for example acetyl chloride, benzoyl chloride, benzyl bromide, benzyloxycarbonyl chloride, formylfluoride, phenylsulfonyl chloride, pivaloyl chloride, (*tert*-butyl-O-C=O)₂O, trifluoroacetic anhydride, and triphenylmethyl chloride.

The term "oxo" as used herein, means a =O group.

When cycloalkyl, heterocycle, heteroaryl, aryl, and the like are "substituted", it means there are one or more substituents other than hydrogen on the respective ring. "Unsubstituted" rings have no substituents other than hydrogen.

In some instances, the number of carbon atoms in a hydrocarbon substituent (e.g., alkyl, alkenyl, alkynyl, cycloalkyl or cycloalkenyl) is indicated by the prefix "Cₓ-Cy-", wherein x is the minimum and y is the maximum number of carbon atoms in the substituent. Thus, for example, "C₁-C₆-alkyl" refers to an alkyl substituent containing from 1 to 6 carbon atoms. Illustrating further, C₃-C₆-cycloalkyl means a saturated hydrocarbon ring containing from 3 to 6 carbon ring atoms.

### COMPOUNDS

Compounds of the invention have the Formula (I) as described above.

Particular values of variable groups in compounds of Formula (I) are as follows. Such values can be used where appropriate with any of the other values, definitions, claims or embodiments defined hereinbefore or hereinafter.

In one embodiment, the compounds are further defined as: 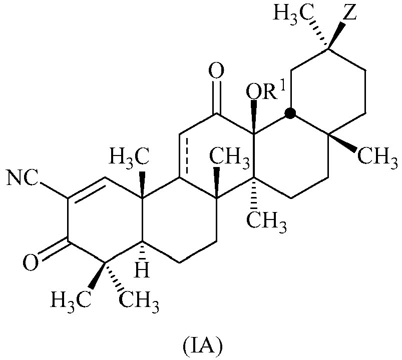 wherein,
----- is a single or double bond;
Z is -CO₂H, -CO₂R², -C(O)NR³R⁴, -C(O)G¹, -NR³C(O)R⁵, -NR³S(O)₂R⁵, -CH₂NR³C(O)R⁵, -CH₂NR³S(O)₂R⁵, -NR³R⁶, or -CH₂NR³R⁶;
R¹ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, -G^{2b}, or C(O)R^{1a};
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or ₋G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁴ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, or -SO₂R^{1a};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R⁵⁶)ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², or -(CR^{4a}R^{4b})ₘ-C(O)N(R³)₂;
R^{1a} is, at each occurrence, independently C₁-C₆-alkyl, C₁-C₆-haloalkyl, C₃-C₈-cycloalkyl, or C₁-C₈-cycloalkyl-C₁-C₃-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, cyano, hydroxy, oxo, C₁-C₆-alkoxy, C₁-C₈-alkyl, C₁-C₈-alkenyl, C₁-C₈-alkynyl, C₁-C₈-haloalkyl, C₁-C₆-alkoxy-C₁-C₆-alkyl, C₁-C₆-cyanoalkyl, C₁-C₆-hydroxyalkyl, -CO₂R^{1a}, or -C(O)N(R³)₂;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{4b})ₘ-G^{a}, or -O-G^{a};
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, ₋N(R^{5c})C(O)R^{5d}, -N(R^{5c})C(O)O(R^{5d}), -N(R^{5c})S(O)2(R^{5d}), C₁-C₄-cyanoalkyl, C₁-C₄-haloalkyl, -G^{a}, or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen;
m, at each occurrence, independently is 1, 2, 3, 4, or 5; and
n, at each occurrence, independently is 1, 2, 3, 4, or 5;
or a pharmaceutically acceptable salt thereof.

In another embodiment, the compounds are further defined as: 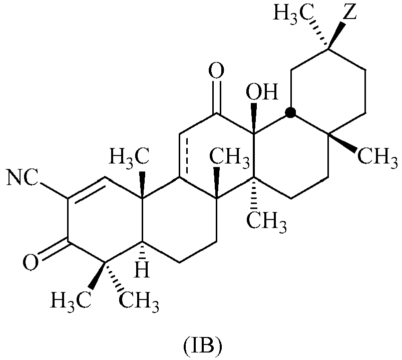 wherein,
----- is a single or double bond;
Z is -CO₂H, -CO₂R², -C(O)NR³R⁴, -C(O)G¹, -NR³C(O)R⁵, -NR³S(O)₂R⁵, -CH₂NR³C(O)R⁵, -CH₂NR³S(O)₂R⁵, -NR³R⁶, or -CH₂NR³R⁶;
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b}
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁴ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, or -SO₂R^{1a};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², or -(CR^{4a}R^{4b})ₘ-C(O)N(R³)₂;
R^{1a} is, at each occurrence, independently C₁-C₆-alkyl, C₁-C₆-haloalkyl, C₁-C₈-cycloalkyl, or C₁-C₈-cycloalkyl-C₁-C₃-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, cyano, hydroxy, oxo, C₁-C₆-alkoxy, C₁-C₈-alkyl, C₁-C₈-alkenyl, C₁-C₈-alkynyl, C₁-C₈-haloalkyl, C₁-C₆-alkoxy-C₁-C₆-alkyl, C₁-C₆-cyanoalkyl, C₁-C₆-hydroxyalkyl, -CO₂R^{1a}, or -C(O)N(R³)₂;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{4b})ₘ-G^{a}, or -O-G^{a};
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a} or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen;
m, at each occurrence, independently is 1, 2, 3, 4, or 5; and
n, at each occurrence, independently is 1, 2, 3, 4, or 5;
or a pharmaceutically acceptable salt thereof.

In another embodiment, the compounds are further defined as a compound of Formula (IB) wherein, Z is -CO₂H or -CO₂R², wherein R² is C₁-C₆-alkyl, C₁-C₆-haloalkyl, -CH²CR^{2a}=CR^{2b}R^{2c}, -CH₂G^{2a}, -G^{2a}, -CH₂G^{2b}, or -G^{2b};
R^{2a}, R^{2b}, and R^{2c} are independently hydrogen, C₁-C₆-alkyl or C₁-C₆-haloalkyl;
G^{2a} is C₃-C₈-cycloalkyl; and
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
or a pharmaceutically acceptable salt thereof.

In another embodiment, the compounds are further defined as a compound of Formula (IB) wherein, Z is -CO₂H or -CO₂R², wherein R² is C₁-C₆-alkyl;
or a pharmaceutically acceptable salt thereof.

In another embodiment, the compounds are further defined as a compound of Formula (IB) wherein, Z is -C(O)NR³R⁴ or -C(O)G¹;
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁴ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, or -SO₂R^{1a};
R^{1a} is, at each occurrence, independently C₁-C₆-alkyl, C₁-C₆-haloalkyl, C₃-C₈-cycloalkyl, or C₁-C₈-cycloalkyl-C₁-C₃-alkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4C})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{4b})ₘ-G^{a}, or -O-G^{a}; and
m, at each occurrence, independently is 1, 2, 3, 4, or 5;
or a pharmaceutically acceptable salt thereof.

In another embodiment, the compounds are further defined as a compound of Formula (IB) wherein, Z is -C(O)NR³R⁴ or -C(O)G¹, wherein
R² is C₁-C₆-alkyl;
R³ is hydrogen or C₁-C₆-alkyl;
R⁴ is hydrogen, C₁-C₈-alkyl, C₁-C₈-alkynyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -(CR^{4a}R^{4b})ₘ-N(R³)₂, -(CR^{4a}R^{4b})ₘ-N(R³)C(O)R³, -G^{4a}, -(CR^{4a}R^{4b})_{m-G}^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR4^{a}R4^{b})ₘ-C(O)N(R³)₂, -(CR^{4a}R^{4b})ₘ-C(O)N(OH)R³, or -SO₂R^{1a};
R^{1a} is, at each occurrence, independently C₁-C₆-alkyl;
R^{4a} and R^{4b} are, at each occurrence, independently hydrogen or C₁-C₆-alkyl;
R^{4c} is, at each occurrence, independently hydrogen or C₁-C₄-alkyl;
R^{4d} is, at each occurrence, independently C₁-C₄-alkyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, hydroxy, C₁-C₆-alkoxy, C₁-C₈-alkyl, C₁-C₈-haloalkyl, C₁-C₆-hydroxyalkyl, -CO₂R^{1a}, or -C(O)N(R³)₂;
G^{4a} is cycloalkyl or heterocycle, wherein the cycloalkyl and heterocycle are unsubstituted or substituted with C₁-C₄-alkyl, halogen, cyano, oxo, -OR^{4c}, -C(O)R^{4d}, -C(O)OR^{4c}, -C(O)N(R^{4c})₂, -N(R^{4c})C(O)R^{4d} -N(R^{4c})C(O)O(R^{4d}), -N(R^{4c})S(O)₂(R^{4d}), C₁-C₄-haloalkyl, C₁-C₄-hydroxyalkyl or -(CR^{4a}R^{4b})ₘ-G^{a};
G^{4b} is aryl or heteroaryl, wherein the aryl and heteroaryl are unsubstituted or substituted with C₁-C₄-alkyl, halogen, cyano, -OR^{4c}, C₁-C₄-haloalkyl or -O-G^{a};
G^{a} is aryl, wherein the aryl is unsubstituted or substituted with 1, 2, 3, 4 or 5 C₁-C₆-alkyl, cyano or halogen; and
m, at each occurrence, independently is 1, 2, 3 or 4;
or a pharmaceutically acceptable salt thereof.

In another embodiment, the compounds are further defined as a compound of Formula (IB) wherein, Z is -C(O)NR³R⁴ or -C(O)G¹, wherein
R³ is hydrogen or C₁-C₆-alkyl;
R⁴ is C₁-C₈-alkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, -G^{4a}, -(CR^{4a}R^{4b})m-G^{4a}, -G^{4b} or -(CR^{4a}R^{4b})ₘ-G^{4b};
R^{1a} is, at each occurrence, independently C₁-C₆-alkyl;
R^{4a} and R^{ab} are, at each occurrence, independently hydrogen or C₁-C₆-alkyl;
R^{4c} is, at each occurrence, independently C₁-C₄-alkyl;
G¹ is a nitrogen containing heterocycle or heteroaryl, wherein the heterocycle or heteroaryl is attached to the carbonyl of Z at a nitrogen atom and is unsubstituted or substituted with one or more -CO₂R^{1a};
G^{4a} is cycloalkyl or heterocycle;
G^{4b} is aryl or heteroaryl, wherein the aryl and heteroaryl are unsubstituted or substituted with C₁-C₄-alkyl, or -OR^{4c}; and
m, at each occurrence, independently is 1 or 2;
or a pharmaceutically acceptable salt thereof.

In another embodiment, the compounds are further defined as a compound of Formula (IB) wherein, Z is -NR³C(O)R⁵, -NR³S(O)₂R⁵, -CH₂NR³C(O)R⁵, or -CH₂NR³S(O)₂R⁵, wherein,
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b} or -(CR^{5a}R^{5b})ₙ-NHC(O)G^{5b};
R^{1a} is, at each occurrence, independently C₁-C₆-alkyl, C₁-C₆-haloalkyl, C₃-C₈-cycloalkyl, or C₁-C₈-cycloalkyl-C₁-C₃-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G^{2a} is C₁-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)²(R^{5d}), C₁-C₄-cyanoalkyl, C₁-C₄-haloalkyl, -G^{a}, or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen; and
n, at each occurrence, independently is 1, 2, 3, 4, or 5;
or a pharmaceutically acceptable salt thereof.

In another embodiment, the compounds are further defined as a compound of Formula (IB) wherein, Z is -NR³C(O)R⁵, -NR³S(O)₂R⁵, -CH₂NR³C(O)R⁵, or -CH₂NR³S(O)₂R⁵, wherein,
R² is C₁-C₆-alkyl;
R³ is hydrogen or C₁-C₆-alkyl;
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R^{1a} is, at each occurrence, independently C₁-C₆-alkyl;
R^{1b} is, at each occurrence, independently hydrogen or C₁-C₆-alkyl;
R^{5a} and R^{5b} are, at each occurrence, independently hydrogen or C₁-C₆-alkyl;
R^{5c} is, at each occurrence, independently hydrogen or C₁-C₄-alkyl;
G^{5a} is cycloalkyl or heterocycle, wherein the cycloalkyl and heterocycle are unsubstituted or substituted with C₁-C₄-alkyl, oxo, -OR^{5c} or -C(O)OR^{5c};
G^{5b} is aryl or heteroaryl, wherein the aryl and heteroaryl are unsubstituted or substituted with C₁-C₄-alkyl, halogen, -OR^{5c}, -C(O)N(R^{5c})₂ or C₁-C₄-haloalkyl;
G^{5c} is aryl, wherein the aryl is unsubstituted or substituted with 1, 2, 3, 4 or 5 C₁-C₆-alkyl, C₁-C₆-alkoxy, cyano or halogen; and
n, at each occurrence, independently is 1, 2, 3 or 4;
or a pharmaceutically acceptable salt thereof.

In another embodiment, the compounds are further defined as a compound of Formula (IB) wherein, Z is -NR³R⁶, or -CH₂NR³R⁶;
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G²b;
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², or -(CR^{4a}R^{4b})ₘ-C(O)N(R³)₂;
R^{2a},R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4C})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4C})₂, -N(R^{4c})₂, -N(R^{4c})C(O)R^{4d}, -N(R^{4c})C(O)O(R^{4d}), -N(R^{4c})S(O)²(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-Cₐ-cyanoalkyl, C₁-C₄-haloalkyl, -G^{a}, -(CR^{4a}R^{4b})ₘ-G^{a}, or -O-G^{a};
G^{a} is aryl or heteroaryl, wherein the aryl or heteroaryl is unsubstituted or substituted with 1, 2, 3, 4 or 5 C₁-C₆-alkyl, cyano or halogen; and
m, at each occurrence, independently is 1, 2, 3, 4, or 5;
or a pharmaceutically acceptable salt thereof.

In another embodiment, the compounds are further defined as: 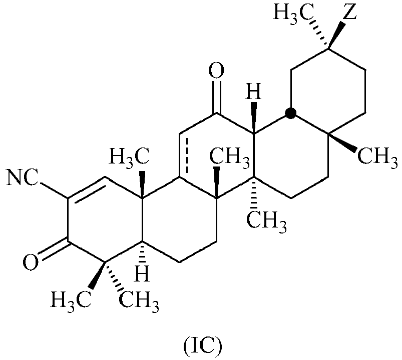 wherein,
----- is a single or double bond;
Z is -C(O)NR³R¹⁴, -C(O)G¹, -NR³C(O)R⁵, -NR³S(O)₂R⁵, -CH₂NR³C(O)R⁵, -CH₂NR₃S(O)₂R⁵, -NR³R⁶, or -CH₂NR³R⁶;
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5a})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO²R^{1a}, -(CR^{5a}R^{5b})ₙ-NHSO²R^{1a}, -NHC(O)R^{1a}, -NHC(O)OR^{1a}, -N(R^{1b})-CR^{5a}R^{5b})ₙ-CN, -G^{5a}, -(CR^{5a}R^{5b})n-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², or -(CR^{4a}R^{4b})ₘ-C(O)N(R³)₂;
R¹⁴ is C₁-C₈-alkenyl, C₁-C₈-alkynyl, C₁-C₈-haloalkyl, C₁-C₆-alkoxy-C₁-C₆-alkyl, C₁-C₆-alkoxy-C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -G^{4b}, -(CR^{4a}R^{4b})m-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -SO₂R^{1a}, or -(CR^{4a}R^{4b})ₘ-SO₂R^{1a};
R^{1a} is, at each occurrence, independently C₁-C₆-alkyl, C₁-C₆-haloalkyl, C₁-C₈-cycloalkyl, or C₁-C₈-cycloalkyl-C₁-C₃-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, cyano, hydroxy, oxo, C₁-C₆-alkoxy, C₁-C₈-alkyl, C₁-C₈-alkenyl, C₁-C₈-alkynyl, C₁-C₈-haloalkyl, C₁-C₆-alkoxy-C₁-C₆-alkyl, C₁-C₆-cyanoalkyl, C₁-C₆-hydroxyalkyl, -CO₂R^{1a}, or -C(O)N(R³)₂;
G^{2a} is C₁-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4C}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{4a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4a})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{4b})ₘ-G^{a}, or -O-G^{a};
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)²(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen;
m, at each occurrence, independently is 1, 2, 3, 4, or 5; and
n, at each occurrence, independently is 1, 2, 3, 4, or 5;
or a pharmaceutically acceptable salt thereof.

In another embodiment, the compounds are further defined as a compound of Formula (IC) wherein, Z is -C(O)NR³R¹⁴ or -C(O)G¹, wherein,
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R¹⁴ is C₁-C₈-alkenyl, C₁-C₈-alkynyl, C₁-C₈-haloalkyl, C₁-C₆-alkoxy-C₁-C₆-alkyl, C₁-C₆-alkoxy-C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -SO₂R^{1a}, or -(CR^{4a}R^{4b})ₘ-SO₂R^{1a};
G¹ is a nitrogen containing heterocycle or heteroaryl, wherein the heterocycle or heteroaryl is attached to the carbonyl of Z at a nitrogen atom and is unsubstituted or substituted with one or more halogen, cyano, hydroxy, oxo, C₁-C₆-alkoxy, C₁-C₈-alkyl, C₁-C₈-alkenyl, C₁-C₈-alkynyl, C₁-C₈-haloalkyl, C₁-C₆-alkoxy-C₁-C₆-alkyl, C₁-C₆-cyanoalkyl, C₁-C₆-hydroxyalkyl, -CO₂R^{1a}, or -C(O)N(R³)₂;
R^{1a} is, at each occurrence, independently C₁-C₆-alkyl, C₁-C₆-haloalkyl, C₁-C₈-cycloalkyl, or C₁-C₈-cycloalkyl-C₁-C₃-alkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{4b})ₘ-G^{a}, or -O-G^{a};
G^{a} is aryl or heteroaryl, wherein the aryl or heteroaryl is unsubstituted or substituted with 1, 2, 3, 4 or 5 C₁-C₆-alkyl, cyano or halogen; and
m, at each occurrence, independently is 1, 2, 3, 4, or 5;
or a pharmaceutically acceptable salt thereof.

In another embodiment, the compounds are further defined as a compound of Formula (IC) wherein, Z is -C(O)NR³R¹⁴ or -C(O)G¹, wherein,
R² is C₁-C₆-alkyl;
R³ is hydrogen or C₁-C₆-alkyl;
R¹⁴ is C₁-C₈-alkenyl, C₁-C₈-alkynyl, C₁-C₈-haloalkyl, C₁-C₆-alkoxy-C₁-C₆-alkyl, C₁-C₆-alkoxy-C₁-C₆-alkoxy-C₁-C₆-alky di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -SO₂R^{1a}, or -(CR^{4a}R^{4b})ₘ-SO₂R^{1a};
R^{1a} is, at each occurrence, independently C₁-C₆-alkyl, C₁-C₆-haloalkyl, C₁-C₈-cycloalkyl, or C₁-C₈-cycloalkyl-C₁-C₁-alkyl;
R^{4a} and R^{4b} are, at each occurrence, independently hydrogen or C₁-C₆-alkyl;
R^{4c} is, at each occurrence, independently hydrogen or C₁-C₄-alkyl;
R^{4d} is, at each occurrence, independently C₁-C₄-alkyl or C₁-C₄-haloalkyl;
G^{4a} is cycloalkyl or heterocycle, wherein the cycloalkyl and heterocycle are unsubstituted or substituted with C₁-C₄-alkyl, halogen, cyano, oxo, -OR^{4c}, -C(O)R^{4d}, -C(O)OR^{4c}, -C(O)N(R^{4c})₂, -N(R^{4c})C(O)R^{4d}, -N(R^{4c})C(O)O(R^{4d}), -N(R^{4c})S(O)₂(R^{4d}), C₁-C₄-haloalkyl, C₁-C₄-hydroxyalkyl or -(CR^{4a}R^{4b})ₘ-G^{a};
G^{4b} is aryl or heteroaryl, wherein the aryl and heteroaryl are unsubstituted or substituted with C₁-C₄-alkyl, halogen, cyano, -O-G^{a} or -OR^{4c};
G^{a} is aryl, wherein the aryl is unsubstituted or substituted with 1, 2, 3, 4 or 5 C₁-C₆-alkyl, cyano or halogen; and
m, at each occurrence, independently is 1, 2, 3 or 4;
or a pharmaceutically acceptable salt thereof.

In another embodiment, the compounds are further defined as a compound of Formula (IC) wherein, Z is -NR³C(O)R⁵, -NR³S(O)₂R⁵, -CH₂NR³C(O)R⁵, or -CH₂NR³S(O)₂R⁵, wherein,
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R⁵⁶)ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})n-OR^{1a}, -(CR^{5a}R^{5b})ₙ-SO²R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})n-CN, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b} or -(CR^{5a}R^{5b})ₙ-NHC(O)G^{5b};
R^{1a} is, at each occurrence, independently C₁-C₆-alkyl, C₁-C₆-haloalkyl, C₁-C₈-cycloalkyl, or C₁-C₈-cycloalkyl-C₁-C₁-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G^{2a} is C₁-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen; and
n, at each occurrence, independently is 1, 2, 3, 4, or 5;
or a pharmaceutically acceptable salt thereof.

In another embodiment, the compounds are further defined as a compound of Formula (IC) wherein, Z is -NR³C(O)R⁵, -NR³S(O)₂R⁵, -CH₂NR³C(O)R⁵, or -CH₂NR³S(O)₂R⁵, wherein,
R² is C₁-C₆-alkyl;
R³ is hydrogen or C₁-C₆-alkyl;
R⁵ is C₁C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ₋C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R^{1a} is, at each occurrence, independently C₁-C₆-alkyl;
R^{1b} is, at each occurrence, independently hydrogen or C₁-C₆-alkyl;
R^{5a} and R^{5b} are, at each occurrence, independently hydrogen or C₁-C₆-alkyl;
R^{5c} is, at each occurrence, independently hydrogen, C₁-C₄-alkyl or C₁-C₄-haloalkyl;
G^{5a} is cycloalkyl or heterocycle, wherein the cycloalkyl and heterocycle are unsubstituted or substituted with C₁-C₄-alkyl, oxo, -OR^{5c} or -C(O)OR^{5c};
G^{5b} is aryl or heteroaryl, wherein the aryl and heteroaryl are unsubstituted or substituted with C₁-C₄-alkyl, halogen, -OR^{5c}, -C(O)N(R^{5c})₂ or C₁-C₄-haloalkyl;
G^{5c} is aryl, wherein the aryl is unsubstituted or substituted with 1, 2, 3, 4 or 5 C₁-C₆-alkyl, C₁-C₆-alkoxy, cyano or halogen; and
n, at each occurrence, independently is 1, 2, 3 or 4.

In another embodiment, the compounds are further defined as a compound of Formula (IC) wherein, Z is -NR³R⁶, or -CH₂NR³R⁶, wherein,
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR5^{4a}R^{ab})ₘ-G^{4b}, -(CR^{4a}R^{4b})ₘ-C(O)OR², or -(CR^{4a}R^{4b})ₘ-C(O)N(R³)₂;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{4b})ₘ-G^{a}, or -O-G^{a};
G^{a} is aryl or heteroaryl, wherein the aryl or heteroaryl is unsubstituted or substituted with 1, 2, 3, 4 or 5 C₁-C₆-alkyl, cyano or halogen; and
m, at each occurrence, independently is 1, 2, 3, 4, or 5;
or a pharmaceutically acceptable salt thereof.

In one embodiment, ----- is a single bond.

In another embodiment, ----- is a double bond.

Specific embodiments of compounds contemplated as part of the invention include, but are not limited to:
(2*S*,4a*S*,6a*S*,6b*R*,8a*R*,12a*S*,14a*S*,14b*R*)-methyl 11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*(1,3-oxazol-5-ylmethyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylic acid;
(2*S*,4a*S*,6a*S*,6b*R*,8a*R*,12a*S*,14a*S*,14b*R*)-11-cyano-14a-hydroxy-*N*-(2-hydroxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyridazin-4-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*,2,4a,6a,6b,9,9,12a-octamethyl-10,14-dioxo-*N-*propyl-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(2,2-dimethylpropyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyridazin-3-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-[(1-methyl-1*H*-pyrazol-4-yl)methyl]-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*[2-(morpholin-4-yl)ethyl]-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*(2-methylbenzyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-[(1-methyl-1*H*-pyrazol-3-yl)methyl]-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-methoxybenzyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6aS*,*6b*R*,8a*R*,12a*S*,14a*S*,14b*R*)-11-cyano-14a-hydroxy-*N*-(2-hydroxybutyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(4a*R*,6a*R*,6b*S*,8a*S*,11*S*,12a*R*,12b*S*,14b*S*)-12b-hydroxy-11-(1*H-*imidazol-1-ylcarbonyl)-4,4,6a,6b,8a,11,14b-heptamethyl-3,13-dioxo-3,4,4a,5,6,6a,6b,7,8,8a,9,10,11,12,12a, 12b, 13,14b-octadecahydropicene-2-carbonitrile;
(2*S*,4a*S*,6a*S*,6b*R*,8a*R*,12a*S*,14a*S*,14b*R*)-11-cyano-14a-hydroxy-*N*-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*(3-methylbutyl)-10,14-dioxo-1,2,3,4,4a,5,6,6a,6b,7,8,8a,9,10,12a,14,14a,14b-octadecahydropicene-2-carboxamide;
(2*S*,4aS*,*6a*S*,6b*R*,8a*R*,12a*S*,14a*S,*14b*R*)-11-cyano-14a-hydroxy-2,4a,6a,6b,9,9,12a-heptamethyl-10,14-dioxo-*N*-(tetrahydro-2*H*-pyran-4-yl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-hydroxypropyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N-*[(1*S*)-1-phenylethyl]-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,4b*R*)-11-cyano-*N-*cyclohexyl-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R,*12a*S*,14a*S*,14b*R*)-*N*-benzyl-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N-*(1,3-dimethoxypropan-2-yl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
methyl 1-{[(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl]carbonyl}prolinate;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-propyl-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(2-furylmethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N-*(4-methoxyphenyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N-*(2-methoxyethyl)-*N*,2,4a,6a,6b,9,9,12a-octamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(cyanomethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*(1,2-oxazol-5-ylmethyl)-10,14-dioxo-1,2,3,4,4a,5,6,6a,6b,7,8,8a,9,10,12a,14,14a,14b-octadecahydropicene-2-carboxamide;
(4a*R*,6a*R*,6b*S*,8a*S*,11*S*,12a*R*,12b*S*,14b*S*)-12b-hydroxy-4,4,6a,6b,8a,11,14b-heptamethyl-3,13-dioxo-11-(pyrrolidin-1-ylcarbonyl)-3,4,4a,5,6,6a,6b,7,8,8a,9,10,11,12,12a, 12b, 13,14b-octadecahydropicene-2-carbonitrile;
(2*S*,4a*S*,6a*S*,6b*R*,8a*R*,12a*S*,14a*S*,14b*R*)-11-cyano-14a-hydroxy-*N-*isobutyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*(2-methylbutyl)-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N*-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N*-(2-furylmethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N*-cyclohexyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N-*(1,3-dimethoxypropan-2-yl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-2,4a,6a,6b,9,9,12a-heptamethyl-*N-*[(1-methyl-1*H-*pyrazol-4-yl)methyl]-10,14-dioxo-1,2,3,4,4a,5,6,6a,6b,7,8,8a,9,10,12a,14,14a,14b-octadecahydropicene-2-carboxamide; or
(2*S*,4a*S*,6a*R*,6b*S*,8a*R*,12a*S*,14a*R*,14b*S*)-11-cyano-2,4a,6a,6b,9,9,12a-heptamethyl-*N-*(1,3-oxazol-5-ylmethyl)-10,14-dioxo-1,2,3,4,4a,5,6,6a,6b,7,8,8a,9,10,12a,14,14a,14b-octadecahydropicene-2-carboxamide.

Within the present invention it is to be understood that compounds disclosed herein can exhibit the phenomenon of tautomerism.

Thus, the formulae drawings within this specification can represent only one of the possible tautomeric or stereoisomeric forms. It is to be understood that the invention encompasses any tautomeric or stereoisomeric form, and mixtures thereof, and is not to be limited merely to any one tautomeric or stereoisomeric form utilized within the naming of the compounds or formulae drawings.

A compound that can exhibit tautomerism is Example 2B which can exist in either an enol form or a keto form:

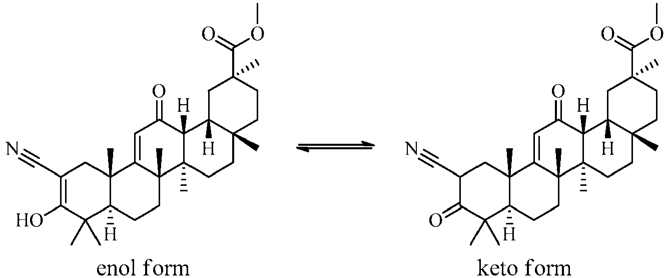

The enol form name is (2*S*,4a*S*,6a*R*,6b*S*,8a*R*,12a*S*,14a*R*,14b*S*)-methyl 11-cyano-10-hydroxy-2,4a,6a,6b,9,9,12a-heptamethyl-14-oxo-1,2,3,4,4a,5,6,6a,6b,7,8,8a,9,12,12a,14,14a,14b-octadecahydropicene-2-carboxylate and the keto form name is (2*S*,4a*S*,6a*R*,6b*S*,8a*R*,12a*S*,14a*R*,14b*S*)-methyl 11-cyano-2,4a,6a,6b,9,9,12a-heptamethyl-10,14-dioxo-1,2,3,4,4a,5,6,6a,6b,7,8,8a,9,10,11,12,12a,14,14a,14b-icosahydropicene-2-carboxylate.

The present invention also includes isotopically-labeled compounds, which are identical to those recited in Formula (I), but for the fact that one or more atoms are replaced by an atom having an atomic mass or mass number different from the atomic mass or mass number usually found in nature. Examples of isotopes suitable for inclusion in the compounds of the invention are hydrogen, carbon, nitrogen, oxygen, phosphorus, fluorine, and chlorine, such as, but not limited to ²H, ³H, ¹³C, ¹⁴C, ¹⁵N, ¹⁸O, ¹⁷O, ³¹P, ³²P, ³⁵S, ¹⁸F, and ³⁶Cl, respectively. Substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Compounds incorporating positron-emitting isotopes are useful in medical imaging and positron-emitting tomography (PET) studies for determining the distribution of receptors. Suitable positron-emitting isotopes that can be incorporated in compounds of Formula (I) are ¹¹C, ¹³N, ¹⁵O, and ¹⁸F. Isotopically-labeled compounds of Formula (I) can generally be prepared by conventional techniques known to those skilled in the art or by processes analogous to those described in the accompanying Examples using appropriate isotopically-labeled reagent in place of non-isotopically-labeled reagent.

### A. BIOLOGICAL DATA

*Tissue Culture:* RAW 264.7, a mouse macrophage cell line, was obtained from American Type Culture Collection (Manassas VA) and maintained in the log phase of growth in Dulbecco's Modified Eagle's Medium (DMEM), 10% heat inactivated fetal calf serum and 100 units/mL antibiotic-antimycotic (AA). Cells were cultured and maintained in a humidified incubator at 37 °C under 5% CO₂ and 95% air. Cells were sub-cultured every 3 days by scraping and were not used beyond passage 20. All cell culture supplies were obtained from Life Technologies (Grand Island, NY).

*Nitric Oxide Suppression Assay.* RAW 264.7 cells were plated 1 day in advance of experiment at a concentration of 80,000 cells/well onto CellBIND® 96 well plates (Corning, NY) in a total volume of 100 µL. The next day, pre-treat cells with compounds (from 3 µM to 0.3 nM serially diluted in a 10 point curve) from a 10× stock by adding 10 µL per well in complete DMEM media containing 10% fetal calf serum. The plates were centrifuged for 3 minutes at 400×g at room temperature followed by 2 hour incubation at 37 °C. The cells were then incubated overnight at 37 °C with 10 µL of the insult, interferon gamma (R&D Systems, Minneapolis, MN), from a 10× stock for a final concentration of 20 ng/mL. The plates were centrifuged for 3 minutes at 400×g at room temperature followed by ∼18 hour incubation at 37 °C. The following day, transfer 50 µL cell culture supernatant from each well into a clear bottom 96 well plate and follow the instructions from Promega's Griess Detection Kit #G2930 (Madison, WI) which involves the addition of 50 µL of the provided sulfanilamide solution for a 5-10 minute incubation at room temperature. Next add 50 µL of the provided *N-*1-napthylethylenediamine dihydrochloride (NED) solution for a 5-10 minute incubation at room temperature and protected from light. If any air bubbles were introduced into the well, the plates need to be centrifuged for 5 minutes at 400×g at room temperature to avoid interference with absorbance readings. The plates were read for absorbance within 30 minutes with a filter between 520 nm and 550 nm.

For the ability of compounds to suppress the increase in nitric oxide release, the percent maximal intensity of nitric oxide detected in each well was normalized to that induced by the peak value for 20 ng/mL of interferon gamma alone and plotted against the compound concentration to calculate IC₅₀ values and to control for plate-to-plate variability. Concentration-response data were analyzed using GraphPad Prism (San Diego, CA); the IC₅₀ values were derived from a single curve fit to the mean data of n=2-3, in duplicates. Selected data is shown in Table 1.

All compounds were dissolved in dimethyl sulfoxide at 10 mM stock solutions and tested at a concentration that the dimethyl sulfoxide levels never exceeded 1%.

**Table 1. Suppression of IFNγ-Induced NO Production.**

| Compound | NO IC₅₀ (µM) |
|---|---|
| bardoxolone methyl | 0.0290 |
| Example 1 | 0.0161 |
| Example 2 | 0.201 |
| Example 3 | 0.0146 |
| Example 3D | 0.301 |
| Example 4 | 0.0554 |
| Example 5 | 0.0500 |
| Example 6 | 0.0355 |
| Example 7 | 0.0224 |
| Example 8 | 0.0279 |
| Example 9 | 0.0128 |
| Example 10 | 0.0239 |
| Example 11 | 0.140 |
| Example 12 | 0.0141 |
| Example 13 | 0.0607 |
| Example 14 | 0.0537 |
| Example 15 | 0.269 |
| Example 16 | 0.0086 |
| Example 17 | 0.0240 |
| Example 18 | 0.0171 |
| Example 19 | 0.0679 |
| Example 20 | 0.0386 |
| Example 21 | 0.0097 |
| Example 22 | 0.0271 |
| Example 23 | 0.0108 |
| Example 24 | 0.0166 |
| Example 25 | 0.0078 |
| Example 26 | 0.0102 |
| Example 27 | 0.0136 |
| Example 28 | 0.0174 |
| Example 29 | 0.0169 |
| Example 30 | 0.0124 |
| Example 31 | 0.0193 |
| Example 32 | 0.0088 |
| Example 33 | 0.0437 |
| Example 34 | 0.0030 |
| Example 35 | 0.0138 |
| Example 36 | 0.142 |
| Example 37 | 0.0158 |
| Example 38 | 0.0068 |
| Example 39 | 0.0057 |

### B. METHODS OF USING THE COMPOUNDS

Inflammation is a biological process that provides resistance to infectious or parasitic organisms and the repair of damaged tissue. Inflammation is commonly characterized by localized vasodilation, redness, swelling, and pain, the recruitment of leukocytes to the site of infection or injury, production of inflammatory cytokines such as TNF-α and IL-1, and production of reactive oxygen or nitrogen species such as hydrogen peroxide, superoxide and peroxynitrite. In later stages of inflammation, tissue remodeling, angiogenesis, and scar formation (fibrosis) may occur as part of the wound healing process. Under normal circumstances, the inflammatory response is regulated and temporary and is resolved in an orchestrated fashion once the infection or injury has been dealt with adequately. However, acute inflammation can become excessive and life-threatening if regulatory mechanisms fail. Alternatively, inflammation can become chronic and cause cumulative tissue damage or systemic complications. Based at least on the evidence presented above, the compounds of this invention may be used in the treatment or prevention of inflammation or diseases associated with inflammation.

Many serious and intractable human diseases involve dysregulation of inflammatory processes, including diseases such as cancer, melanoma, atherosclerosis, and diabetes, which were not traditionally viewed as inflammatory conditions. In the case of cancer, the inflammatory processes are associated with tumor formation, progression, metastasis, and resistance to therapy. Atherosclerosis, long viewed as a disorder of lipid metabolism, is now understood to be primarily an inflammatory condition, with activated macrophages playing an important role in the formation and eventual rupture of atherosclerotic plaques. Activation of inflammatory signaling pathways has also been shown to play a role in the development of insulin resistance, as well as in the peripheral tissue damage associated with diabetic hyperglycemia. Excessive production of reactive oxygen species and reactive nitrogen species such as superoxide, hydrogen peroxide, nitric oxide, and peroxynitrite is a hallmark of inflammatory conditions. Evidence of dysregulated peroxynitrite production has been reported in a wide variety of diseases (Szabo *et al.,* 2007; Schulz *et al.,* 2008; Forstermann, 2006; Pall, 2007).

Autoimmune diseases such as rheumatoid arthritis, lupus, psoriasis, and multiple sclerosis involve inappropriate and chronic activation of inflammatory processes in affected tissues, arising from dysfunction of self vs. non-self recognition and response mechanisms in the immune system. In neurodegenerative diseases such as Alzheimer's and Parkinson's diseases, neural damage is correlated with activation of microglia and elevated levels of pro-inflammatory proteins such as inducible nitric oxide synthase (iNOS). Chronic organ failure such as renal failure, heart failure, liver failure, and chronic obstructive pulmonary disease is closely associated with the presence of chronic oxidative stress and inflammation, leading to the development of fibrosis and eventual loss of organ function. Oxidative stress in vascular endothelial cells, which line major and minor blood vessels, can lead to endothelial dysfunction and is believed to be an important contributing factor in the development of systemic cardiovascular disease, complications of diabetes, chronic kidney disease and other forms of organ failure, and a number of other aging-related diseases including degenerative diseases of the central nervous system and the retina.

Many other disorders involve oxidative stress and inflammation in affected tissues, including inflammatory bowel disease; inflammatory skin diseases; mucositis related to radiation therapy and chemotherapy; eye diseases such as uveitis, glaucoma, macular degeneration, and various forms of retinopathy; transplant failure and rejection; ischemia-reperfusion injury; chronic pain; degenerative conditions of the bones and joints including osteoarthritis and osteoporosis; asthma and cystic fibrosis; seizure disorders; and neuropsychiatric conditions including schizophrenia, depression, bipolar disorder, post-traumatic stress disorder, attention deficit disorders, autism-spectrum disorders, and eating disorders such as anorexia nervosa. Dysregulation of inflammatory signaling pathways is believed to be a major factor in the pathology of muscle wasting diseases including muscular dystrophy and various forms of cachexia. Dysregulated inflammatory signaling has also been identified as a consequence of obesity, and has been reported to contribute to the development of insulin resistance, cardiovascular disease, metabolic syndrome, and other disorders that are strongly associated with obesity (see, *e.g*., Hotamisligil, 2010; Hotamisligil, 2006; Cai *et al.,* 2005). Preclinical studies have indicated that Nrf2 activation can inhibit weight gain in animals provided with a high-fat diet (Shin et al., Eur. J. Pharmacol, 620(1-3): 138-44.). Clinical trials have shown that, in patients with type 2 diabetes and chronic kidney disease, bardoxolone methyl treatment induced significant weight loss, with the loss being more pronounced in patients having the highest body mass index (i.e., the patients with the highest degree of obesity) (WO 2011/130302). Thus, compounds of the invention may be used in some embodiments in the prevention or treatment of clinically significant obesity and its complications.

A variety of life-threatening acute disorders also involve dysregulated inflammatory signaling, including acute organ failure involving the pancreas, kidneys, liver, or lungs, myocardial infarction or acute coronary syndrome, stroke, septic shock, trauma, severe burns, and anaphylaxis.

Many complications of infectious diseases also involve dysregulation of inflammatory responses. Although an inflammatory response can kill invading pathogens, an excessive inflammatory response can also be quite destructive and in some cases can be a primary source of damage in infected tissues. Furthermore, an excessive inflammatory response can also lead to systemic complications due to overproduction of inflammatory cytokines such as TNF-α and IL-1. This is believed to be a factor in mortality arising from severe influenza, severe acute respiratory syndrome, and sepsis.

The aberrant or excessive expression of either iNOS or cyclooxygenase-2 (COX-2) has been implicated in the pathogenesis of many disease processes. For example, it is clear that NO is a potent mutagen (Tamir and Tannebaum, 1996), and that nitric oxide can also activate COX-2 (Salvemini *et al.,* 1994). Furthermore, there is a marked increase in iNOS in rat colon tumors induced by the carcinogen, azoxymethane (Takahashi *et al.,* 1997). A series of synthetic triterpenoid analogs of oleanolic acid have been shown to be powerful inhibitors of cellular inflammatory processes, such as the induction by IFN-γ of inducible nitric oxide synthase (iNOS) and of COX-2 in mouse macrophages. See Honda *et al*. (2000a); Honda *et al.* (2000b), and Honda *et al*. (2002).

In one aspect, compounds disclosed herein are characterized by their ability to inhibit the production of nitric oxide in macrophage-derived RAW 264.7 cells induced by exposure to γ-interferon. They are further characterized by their ability to induce the expression of antioxidant proteins such as NQO1 and reduce the expression of pro-inflammatory proteins such as COX-2 and inducible nitric oxide synthase (iNOS). These properties are relevant to the treatment of a wide array of diseases and disorders involving oxidative stress and dysregulation of inflammatory processes including cancer, complications from localized or total-body exposure to ionizing radiation, mucositis resulting from radiation therapy or chemotherapy, autoimmune diseases, cardiovascular diseases including atherosclerosis, ischemia-reperfusion injury, acute and chronic organ failure including renal failure and heart failure, respiratory diseases, insulin resistance, diabetes and complications of diabetes, severe allergies, transplant rejection, graft-versus-host disease, neurodegenerative diseases, diseases of the eye and retina, acute and chronic pain, degenerative bone diseases including osteoarthritis and osteoporosis, inflammatory bowel diseases, dermatitis and other skin diseases, sepsis, burns, seizure disorders, and neuropsychiatric disorders.

Without being bound by theory, the activation of the antioxidant/anti-inflammatory Keap1/Nrf2/ARE pathway is believed to be implicated in both the anti-inflammatory and anti-carcinogenic properties of the compounds disclosed herein.

In another aspect, compounds disclosed herein may be used for treating a subject having a condition caused by elevated levels of oxidative stress in one or more tissues. Oxidative stress results from abnormally high or prolonged levels of reactive oxygen species such as superoxide, hydrogen peroxide, nitric oxide, and peroxynitrite (formed by the reaction of nitric oxide and superoxide). The oxidative stress may be accompanied by either acute or chronic inflammation. The oxidative stress may be caused by mitochondrial dysfunction, by activation of immune cells such as macrophages and neutrophils, by acute exposure to an external agent such as ionizing radiation or a cytotoxic chemotherapy agent (e.g., doxorubicin), by trauma or other acute tissue injury, by ischemia/reperfusion, by poor circulation or anemia, by localized or systemic hypoxia or hyperoxia, by elevated levels of inflammatory cytokines and other inflammation-related proteins, and/or by other abnormal physiological states such as hyperglycemia or hypoglycemia.

In animal models of many such conditions, stimulating expression of inducible heme oxygenase (HO-1), a target gene of the Nrf2 pathway, has been shown to have a significant therapeutic effect including models of myocardial infarction, renal failure, transplant failure and rejection, stroke, cardiovascular disease, and autoimmune disease (*e.g.,* Sacerdoti *et al.,* 2005; Abraham & Kappas, 2005; Bach, 2006; Araujo *et al.,* 2003; Liu *et al.,* 2006; Ishikawa *et al.,* 2001; Kruger *et al.,* 2006; Satoh *et al.,* 2006; Zhou *et al.,* 2005; Morse and Choi, 2005; Morse and Choi, 2002). This enzyme breaks free heme down into iron, carbon monoxide (CO), and biliverdin (which is subsequently converted to the potent antioxidant molecule, bilirubin).

The compounds of this invention may be used in preventing or treating tissue damage or organ failure, acute and chronic, resulting from oxidative stress exacerbated by inflammation. Examples of diseases that fall in this category include: heart failure, liver disease (e.g., alcoholic liver disease, fatty liver disease, non-alcoholic steatohepatitis, cirrhosis) and liver failure, transplant failure and rejection, renal failure, pancreatitis, asthma, fibrotic lung diseases (cystic fibrosis, COPD, and idiopathic pulmonary fibrosis, among others), diabetes (including complications), atherosclerosis, ischemia-reperfusion injury, glaucoma, stroke, autoimmune disease, autism, macular degeneration, and muscular dystrophy. For example, in the case of autism, studies suggest that increased oxidative stress in the central nervous system may contribute to the development of the disease (Chauhan and Chauhan, 2006).

Evidence also links oxidative stress and inflammation to the development and pathology of many other disorders of the central nervous system, including psychiatric disorders such as psychosis, major depression, and bipolar disorder; seizure disorders such as epilepsy; pain and sensory syndromes such as migraine, neuropathic pain or tinnitus; and behavioral syndromes such as the attention deficit disorders. See, *e.g.,* Dickerson *et al.,* 2007; Hanson *et al.,* 2005; Kendall-Tackett, 2007; Lencz *et al.,* 2007; Dudhgaonkar *et al.,* 2006; Lee *et al.,* 2007; Morris *et al.,* 2002; Ruster *et al.,* 2005; McIver *et al.,* 2005; Sarchielli *et al.,* 2006; Kawakami *et al.,* 2006; Ross *et al.,* 2003. For example, elevated levels of inflammatory cytokines, including TNF, interferon-γ, and IL-6, are associated with major mental illness (Dickerson *et al.,* 2007). Microglial activation has also been linked to major mental illness. Therefore, downregulating inflammatory cytokines and inhibiting excessive activation of microglia could be beneficial in patients with schizophrenia, major depression, bipolar disorder, autism-spectrum disorders, and other neuropsychiatric disorders.

Accordingly, in pathologies involving oxidative stress alone or oxidative stress exacerbated by inflammation, treatment may comprise administering to a subject a therapeutically effective amount of a compound of this invention, such as those described above or throughout this specification. Treatment may be administered preventively, in advance of a predictable state of oxidative stress (*e*.*g*., organ transplantation or the administration of radiation therapy to a cancer patient), or it may be administered therapeutically in settings involving established oxidative stress and inflammation.

The compounds disclosed herein may be generally applied to the treatment of inflammatory conditions, such as sepsis, dermatitis, autoimmune disease and osteoarthritis. In one aspect, the compounds of this invention may be used to treat inflammatory pain and/or neuropathic pain, for example, by inducing Nrf2 and/or inhibiting NF-κB.

The compounds disclosed herein may be used in the treatment and prevention of diseases such as cancer, inflammation, Alzheimer's disease, Parkinson's disease, multiple sclerosis, autism, amyotrophic lateral sclerosis, Huntington's disease, autoimmune diseases such as rheumatoid arthritis, lupus, Crohn's disease, psoriasis, inflammatory bowel disease, melanoma, and all other diseases whose pathogenesis is believed to involve excessive production of either nitric oxide or prostaglandins, and pathologies involving oxidative stress alone or oxidative stress exacerbated by inflammation.

Another aspect of inflammation is the production of inflammatory prostaglandins such as prostaglandin E. These molecules promote vasodilation, plasma extravasation, localized pain, elevated temperature, and other symptoms of inflammation. The inducible form of the enzyme COX-2 is associated with their production, and high levels of COX-2 are found in inflamed tissues. Consequently, inhibition of COX-2 may relieve many symptoms of inflammation and a number of important anti-inflammatory drugs (e.g., ibuprofen and celecoxib) act by inhibiting COX-2 activity. Recent research, however, has demonstrated that a class of cyclopentenone prostaglandins (cyPGs) (*e*.*g*., 15-deoxy prostaglandin J2, a.k.a. PGJ2) plays a role in stimulating the orchestrated resolution of inflammation (*e*.*g*., Rajakariar *et al,* 2007). COX-2 is also associated with the production of cyclopentenone prostaglandins. Consequently, inhibition of COX-2 may interfere with the full resolution of inflammation, potentially promoting the persistence of activated immune cells in tissues and leading to chronic, "smoldering" inflammation. This effect may be responsible for the increased incidence of cardiovascular disease in patients using selective COX-2 inhibitors for long periods of time.

The compounds disclosed herein may be used to control the production of pro-inflammatory cytokines within the cell by selectively activating regulatory cysteine residues (RCRs) on proteins that regulate the activity of redox-sensitive transcription factors. Activation of RCRs by cyPGs has been shown to initiate a pro-resolution program in which the activity of the antioxidant and cytoprotective transcription factor Nrf2 is potently induced and the activities of the pro-oxidant and pro-inflammatory transcription factors NF-κB and the STATs are suppressed. This increases the production of antioxidant and reductive molecules (NQO1, HO-1, SOD1, γ-GCS) and decreases oxidative stress and the production of pro-oxidant and pro-inflammatory molecules (iNOS, COX-2, TNF-α). The compounds of this invention may cause the cells that host the inflammatory event to revert to a non-inflammatory state by promoting the resolution of inflammation and limiting excessive tissue damage to the host.

In another aspect, the compounds disclosed are useful for the treatment and prevention of immune-mediated diseases such as the resistance by transplantation of organs or tissue such as heart, kidney, liver, medulla ossium, skin, cornea, lung, pancreas, intestinum tenue, limb, muscle, nerves, duodenum, small-bowel, pancreatic-islet-cell, and the like; graft-versus-host diseases brought about by medulla ossium transplantation; autoimmune diseases such as rheumatoid arthritis, systemic lupus erythematosus, Hashimoto's thyroiditis, multiple sclerosis, myasthenia gravis, type I diabetes, uveitis, allergic encephalomyelitis, glomerulonephritis, and the like. Further uses include the treatment and prophylaxis of inflammatory and hyperproliferative skin diseases and cutaneous manifestations of immunologically-mediated illnesses, such as psoriasis, atopic dermatitis, contact dermatitis and further eczematous dermatitises, seborrhoeis dermatitis, lichen planus, pemphigus, bullous pemphigoid, epidermolysis bullosa, urticaria, angioedemas, vasculitides, erythemas, cutaneous eosinophilias, lupus erythematosus, acne and alopecia areata; various eye diseases (autoimmune and otherwise) such as keratoconjunctivitis, vernal conjunctivitis, uveitis associated with Behcet's disease, keratitis, herpetic keratitis, conical cornea, dystrophia epithelialis corneae, corneal leukoma, and ocular pemphigus. In addition reversible obstructive airway disease, which includes conditions such as asthma (for example, bronchial asthma, allergic asthma, intrinsic asthma, extrinsic asthma and dust asthma), particularly chronic or inveterate asthma (for example, late asthma and airway hyper-responsiveness), bronchitis, allergic rhinitis, and the like are targeted by compounds of this invention. The compounds disclosed are also useful in the treatment of inflammation of mucosa and blood vessels such as gastric ulcers, vascular damage caused by ischemic diseases and thrombosis. Moreover, hyperproliferative vascular diseases such as intimal smooth muscle cell hyperplasia, restenosis and vascular occlusion, particularly following biologically- or mechanically-mediated vascular injury, could be treated or prevented by the compounds of the invention.

The compounds or drugs described herein can be incorporated into stents or catheters that are constructed from or have been coated with a polymeric compound forming a drug-eluting stent or catheter, respectively. The drug-eluting stent containing the compound or drug can then be delivered to the coronary vessel by deployment from a balloon catheter. In addition to stents, other devices that can be used to introduce the drugs of this invention to the vasculature include, but are not limited to grafts, catheters, and balloons.

Actual dosage levels of active ingredients in the pharmaceutical compositions of this invention can be varied so as to obtain an amount of the active compound(s) that is effective to achieve the desired therapeutic response for a particular patient, compositions and mode of administration. The selected dosage level can depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

Compounds of the invention can also be administered as a pharmaceutical composition comprising the compounds of interest in combination with one or more pharmaceutically acceptable carriers. The phrase "therapeutically effective amount" of the compound of the invention means a sufficient amount of the compound to treat disorders, at a reasonable benefit/risk ratio applicable to any medical treatment. It can be understood, however, that the total daily usage of the compounds and compositions of the invention can be decided by the attending physician within the scope of sound medical judgment. The specific therapeutically effective dose level for any particular patient can depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well-known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or other animal range from about 0.01 mg/kg body weight to about 100 mg/kg body weight. More preferable doses can be in the range of from about 0.01 mg/kg body weight to about 30 mg/kg body weight. If desired, the effective daily dose can be divided into multiple doses for purposes of administration. Consequently, single dose compositions can contain such amounts or submultiples thereof to make up the daily dose.

The present invention also is directed, in part, to a use of one or more compounds and/or salts of the invention, and, optionally one or more additional therapeutic agents to prepare a medicament. In some embodiments, the medicament is for co-administration with one or more additional therapeutic agents. In some embodiments, the medicament is for treating inflammation.

This invention also is directed, in part, to one or more compounds and/or salts of the invention, and, optionally one or more additional therapeutic agents, for use as a medicament. In some embodiments, the medicament is for treating inflammation.

### C. PHARMACEUTICAL COMPOSITIONS

The present invention further provides pharmaceutical compositions that comprise compounds of the present invention or a pharmaceutically acceptable salt or solvate thereof. The pharmaceutical compositions comprise compounds of the present invention that can be formulated together with one or more non-toxic pharmaceutically acceptable carriers.

In addition to being used as a monotherapy, the compounds of the present invention may also find use in combination therapies. Effective combination therapy may be achieved with a single composition or pharmacological formulation that includes both agents, or with two distinct compositions or formulations, administered at the same time, wherein one composition includes a compound of this invention, and the other includes the second agent(s). Alternatively, the therapy may precede or follow the other agent treatment by intervals ranging from minutes to months.

Non-limiting examples of such combination therapy include combination of one or more compounds of the invention with another anti-inflammatory agent, a chemotherapeutic agent, radiation therapy, an antidepressant, an antipsychotic agent, an anticonvulsant, a mood stabilizer, an anti-infective agent, an antihypertensive agent, a cholesterol-lowering agent or other modulator of blood lipids, an agent for promoting weight loss, an antithrombotic agent, an agent for treating or preventing cardiovascular events such as myocardial infarction or stroke, an antidiabetic agent, an agent for reducing transplant rejection or graft-versus-host disease, an anti-arthritic agent, an analgesic agent, an anti-asthmatic agent or other treatment for respiratory diseases, or an agent for treatment or prevention of skin disorders. Compounds of the invention may be combined with agents designed to improve a patient's immune response to cancer, including (but not limited to) cancer vaccines. See Lu *et al.* (2011).

The pharmaceutical compositions of this invention can be administered to humans and other mammals orally, rectally, parenterally, intracisternally, intravaginally, intraperitoneally, topically (as by powders, ointments or drops), bucally or as an oral or nasal spray. The term "parenterally" as used herein, refers to modes of administration which include intravenous, intramuscular, intraperitoneal, intrasternal, subcutaneous and intraarticular injection and infusion.

The term "pharmaceutically acceptable carrier" as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such a propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions can also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It can also be desirable to include isotonic agents such as sugars, sodium chloride and the like. Prolonged absorption of the injectable pharmaceutical form can be brought about by the inclusion of agents which delay absorption such as aluminum monostearate and gelatin.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, can depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound can be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form can also comprise buffering agents.

Solid compositions of a similar type can also be employed as fillers in soft and hard-filled gelatin capsules using such carriers as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They can optionally contain opacifying agents and can also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned carriers.

Liquid dosage forms for oral administration include pharmaceutically acceptable emulsions, solutions, suspensions, syrups and elixirs. In addition to the active compounds, the liquid dosage forms can contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions can also include adjuvants such as wetting agents, emulsifying and suspending agents, sweetening, flavoring and perfuming agents.

Suspensions, in addition to the active compounds, can contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which can be prepared by mixing the compounds of this invention with suitable non-irritating carriers or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of this invention include powders, sprays, ointments and inhalants. The active compound can be mixed under sterile conditions with a pharmaceutically acceptable carrier and any needed preservatives, buffers or propellants which can be required. Ophthalmic formulations, eye ointments, powders and solutions are also contemplated as being within the scope of this invention.

The compounds of the present invention can be used in the form of pharmaceutically acceptable salts derived from inorganic or organic acids. The phrase "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio.

Pharmaceutically acceptable salts are well known in the art. For example, S. M. Berge et al. describe pharmaceutically acceptable salts in detail in (J. Pharmaceutical Sciences, 1977, 66: 1 et seq). The salts can be prepared in situ during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, malate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, *p*-toluenesulfonate and undecanoate. Also, the basic nitrogen-containing groups can be quaternized with such agents as lower alkyl halides such as, but not limited to, methyl, ethyl, propyl, and butyl chlorides, bromides and iodides; dialkyl sulfates like dimethyl, diethyl, dibutyl and diamyl sulfates; long chain halides such as, but not limited to, decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulfuric acid, and phosphoric acid and such organic acids as acetic acid, fumaric acid, maleic acid, 4-methylbenzenesulfonic acid, succinic acid and citric acid.

Basic addition salts can be prepared in situ during the final isolation and purification of compounds of this invention by reacting a carboxylic acid-containing moiety with a suitable base such as, but not limited to, the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as, but not limited to, lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, ethylammonium and the like. Other representative organic amines useful for the formation of base addition salts include ethylenediamine, ethanolamine, diethanolamine, piperidine, piperazine and the like.

The compounds of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

The present invention also is directed, in part, to a kit comprising one or more compounds and/or salts of the invention. The kit can optionally contain one or more additional therapeutic agents and/or instructions for, for example, using the kit.

### D. GENERAL SYNTHESIS

This invention is intended to encompass compounds of the invention when prepared by synthetic processes or by metabolic processes. Preparation of the compounds by metabolic processes includes those occurring in the human or animal body (in vivo) or processes occurring *in vitro.*

This invention is directed, in part, to the synthetic processes for preparing compounds of Formula (I) as shown in Schemes 1-3 and the Examples below. This invention also is directed, in part, to novel intermediates that can be used to prepare the compounds of Formula (I) (and their salts) as shown in Schemes 1-3 and the Examples below.

The compounds of the invention can be prepared by a variety of processes well known for the preparation of compounds of this class. For example, the compounds of the invention wherein the groups Y and Z, have the meanings as set forth in the Summary of the Invention section unless otherwise noted, can be synthesized as shown in Schemes 1-3.

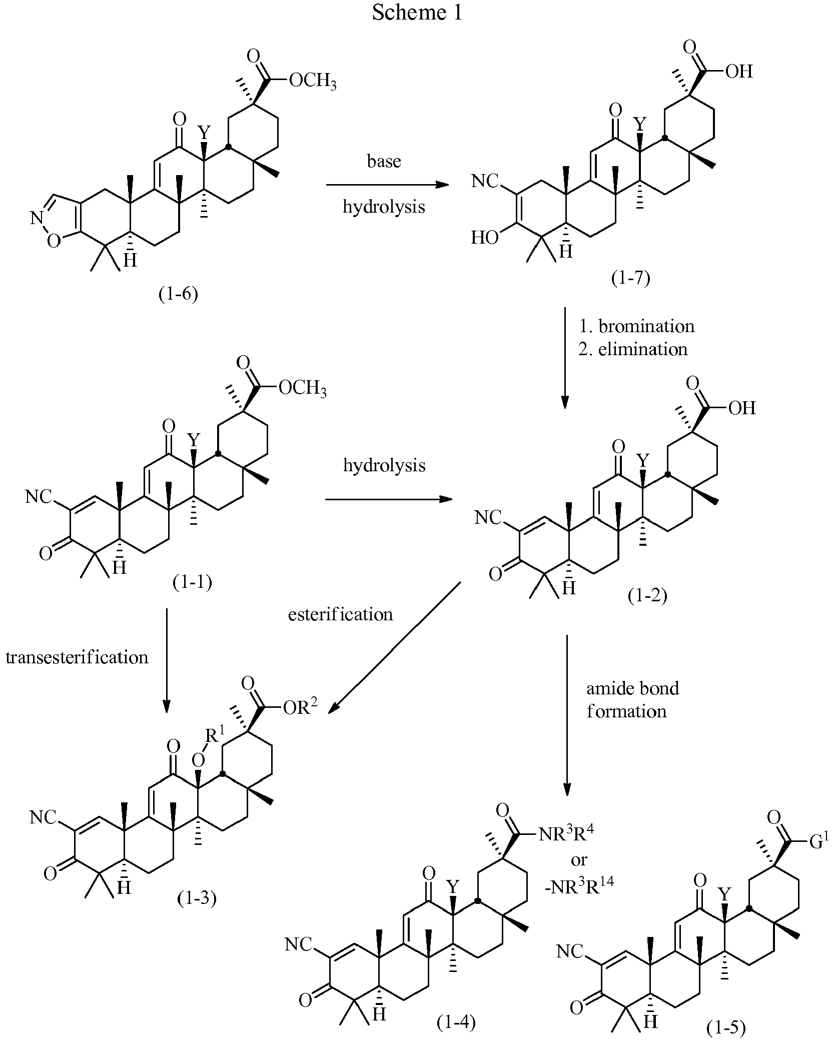

As illustrated in Scheme 1, compounds of Formula (1-1) can be converted to compounds of Formula (1-2) and subsequently to compounds of Formulas (1-3), (1-4), and (1-5), wherein Y is as defined in the Summary of the Invention. Compounds of Formula (1-1) can be hydrolyzed to compounds of Formula (1-2) under conditions known to one of skill in the art. One hydrolysis condition contemplated is to combine compounds of Formula (1-1) with sodium acetate and lithium bromide in optionally heated *N,N-*dimethylacetamide under an inert atmosphere to supply compounds of Formula (1-2). Compounds of Formula (1-2), wherein Y is -OR¹, can be esterified under conditions known to one of skill in the art to give compounds of Formula (1-3). Alternatively, compounds of Formula (1-1), wherein Y is -OR¹, can be transesterified directly to compounds of Formula (1-3) under conditions known to one of skill in the art. Carboxylic acids of Formula (1-2) can be coupled with amines HNR³R⁴, HNR³R¹⁴, or H-G¹, wherein R³, R⁴, R¹⁴, and G¹ are as defined in the Summary of the Invention, to give compounds of Formula (1-4) or Formula (1-5). Examples of conditions known to generate amides from a mixture of a carboxylic acid and an amine include but are not limited to adding a coupling reagent such as but not limited to *N-*(3-dimethylaminopropyl)-*N'*-ethylcarbodiimide (EDC or EDCI), 1,3-dicyclohexyl-carbodiimide (DCC), bis(2-oxo-3-oxazolidinyl)phosphinic chloride (BOPC1), O-(7-azabenzotriazol-1-yl)-*N,N,N',N'*-tetramethyluronium hexafluorophosphate (HATU), O-benzotriazol-1-yl-*N,N,N',N'-*tetramethyluronium tetrafluoroborate (TBTU), and 2-(1*H-*benzo[*d*][1,2,3]triazol-1-yl)-1,1,3,3-tetramethylisouronium hexafluorophosphate(V) (HBTU). The coupling reagents can be added as a solid, a solution or as the reagent bound to a solid support resin. In addition to the coupling reagents, auxiliary-coupling reagents can facilitate the coupling reaction. Auxiliary coupling reagents that are often used in the coupling reactions include but are not limited to (dimethylamino)pyridine (DMAP), 1-hydroxy-7-azabenzotriazole (HOAT) and 1-hydroxybenzotriazole (HOBT). The reaction can be carried out optionally in the presence of a base such as triethylamine or diisopropylethylamine. The coupling reaction can be carried out in solvents such as but not limited to tetrahydrofuran, *N,N-*dimethylformamide, dichloromethane, and ethyl acetate. The reaction can be conducted at ambient or elevated temperatures.

Also as illustrated in Scheme 1, compounds of Formula (1-6), which are prepared as described in the Examples, can be converted to compounds of Formula (1-7) and then compounds of Formula (1-2). Initial treatment of compounds of Formula (1-6) with a base such as sodium methoxide in methanol at 0 °C to 60 °C opens the isoxazole to provide the α-cyanoketone moiety. Subsequent ester hydrolysis with a base such as aqueous sodium hydroxide provides compounds of Formula (1-7). Compounds of Formula (1-7) can be converted to compound of Formula (1-2) in a two-step process. Compounds of Formula (1-7) can be brominated with a reagent such as 1,3-dibromo-5,5-dimethylhydantoin at 0 °C to room temperature, preferably near 0 °C. Subsequent treatment with pyridine at ambient temperature to 60 °C delivers compounds of Formula (1-2). Compounds of Formula (1-7) can also be converted to compounds of Formula (1-2) by treatment with 2,3-dichloro-5,6-dicyano-1.4-benzoquinone in heated benzene or toluene or in tetrahydrofuran at ambient temperature.

Compounds of Formula (1-1), Formula (1-2), Formula (1-3), Formula (1-4), and Formula (1-5) are representative of compounds of Formula (I).

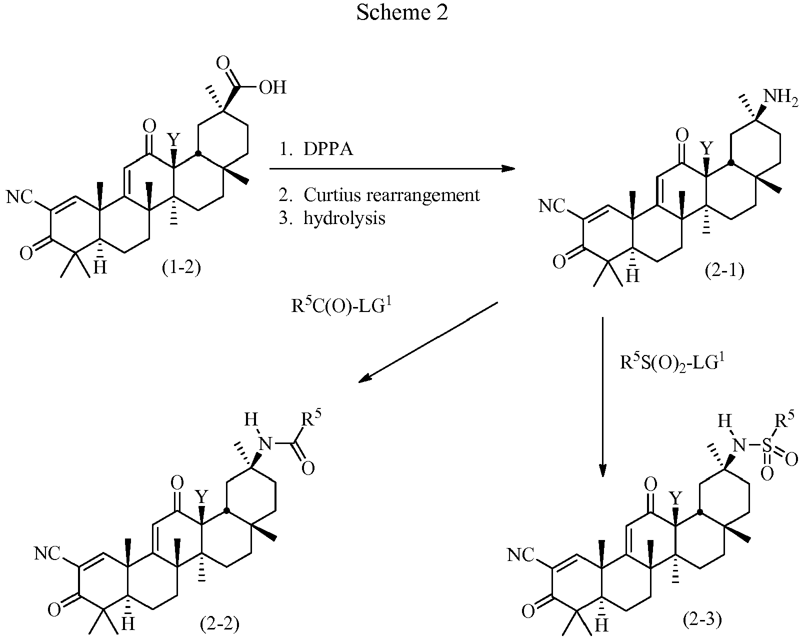

As illustrated in Scheme 2, compounds of Formula (1-2) can be transformed to compounds of Formula (2-2) and Formula (2-3), both of which are representative of compounds of Formula (I). Compounds of Formula (1-2) can be reacted with diphenylphosphoryl azide (DPPA) in the presence of a base to give an intermediate acyl azide. The acyl azide can be treated under Curtius rearrangement conditions and subsequently hydrolyzed to give compound of Formula (2-1). Compounds of Formula (2-1) can be reacted with compounds of formula R⁵C(O)-LG¹ in the presence of a base to give compounds of Formula (2-2). LG¹ is a leaving group known to one of skill in the art such as halogen, *p*-nitrophenoxide, pentafluorophenoxide, and the like. Compounds of formula R⁵C(O)-LG¹ can be acid chlorides, chloroformates or carbamic chlorides. The ureas formed from reaction with carbamic chlorides can alternatively be formed by reaction with the corresponding isocyanates. Compounds of Formula (2-1) can be reacted with compounds of formula R⁵SO₂Cl in the presence of a base to give compounds of Formula (2-3).

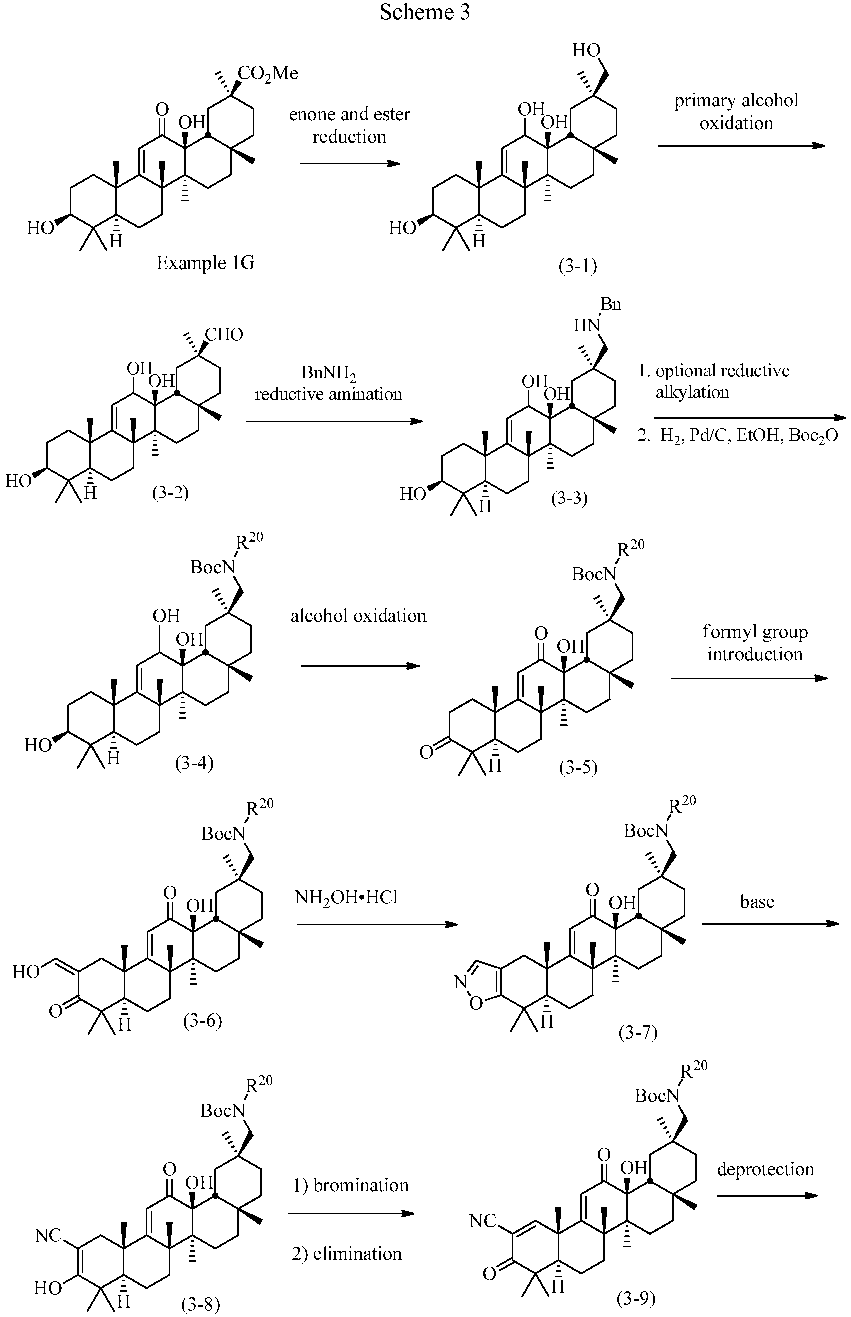

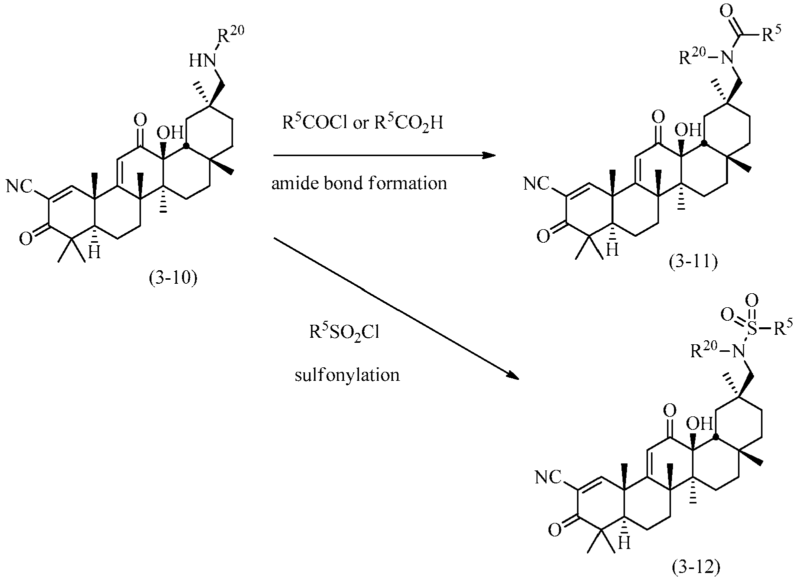

As illustrated in Scheme 3, Example 1G can be transformed to compounds of Formula (3-11) and Formula (3-12), both of which are representative of compounds of Formula (I). The enone and ester moieties in Example 1G can be reduced using a reductant such as lithium aluminum hydride to give compound (3-1). Selective oxidation of the primary alcohol in compound (3-1) using a reagents such as a combination of (2,2,6,6-tetramethylpiperidin-1-yl)oxidanyl (TEMPO) and (diacetoxyiodo)benzene in solvents such as a mixture of dichloromethane and water gives compound (3-2). The aldehyde of compound (3-2) can be reductively aminated with benzylamine in the presence of sodium triacetoxyborohydride and acetic acid to give compound (3-3). An optional second reductive alkylation using formaldehyde can introduce a methyl group. The benzyl group can then be cleaved reductively with hydrogen in the presence of palladium. Protection can occur in the same reaction vessel in the presence of di-*tert*-butyl dicarbonate delivering compounds of Formula (3-4), wherein R²⁰ is either hydrogen or methyl. The allylic and secondary alcohols of compounds of Formula (3-4) can be oxidized with reagents such as tetrapropylammonium perruthenate (TPAP) and 4-methylmorpholine *N-*oxide to give compounds of Formula (3-5). Compounds of Formula (3-5) can be treated with ethyl formate in the presence of a base such as sodium methoxide to give compounds of Formula (3-6). Compounds of Formula (3-6) can be converted to isoxazoles of Formula (3-7) upon treatment with hydroxylamine hydrochloride. Treatment of the isoxazoles of Formula (3-7) with a base such as sodium methoxide in methanol opens the isoxazole to give compounds of Formula (3-8). A bromination/elimination sequence can be carried out by first treating compounds of Formula (3-8) with 1,3-dibromo-5,5-dimethylhydantoin in *N,N*-dimethylformamide, and subsequently treating the intermediate bromide with pyridine and warming to supply compounds of Formula (3-9). The *tert*-butoxy carbonyl (Boc) protecting group of compounds of Formula (3-9) can be removed by treatment with hydrochloric acid in dioxane or trifluoroacetic acid in dichloromethane to give compounds of Formula (3-10). Compounds of Formula (3-10) can be treated with acid chlorides, R⁵C(O)Cl, wherein R⁵ is as described in the Summary, optionally in the presence of a tertiary amine base, to give compounds of Formula (3-11). Alternatively, compounds of Formula (3-10) can be converted to amides of Formula (3-11) by treatment with carboxylic acids, R⁵CO₂H, using the amide bond forming conditions described in Scheme 1. Compounds of Formula (3-10) can also be sulfonylated with sulfonyl chlorides, R⁵SO₂Cl, optionally in the presence of a tertiary amine base, to give compounds of Formula (3-12).

Optimum reaction conditions and reaction times for each individual step can vary depending on the particular reactants employed and substituents present in the reactants used. Unless otherwise specified, solvents, temperatures and other reaction conditions can be readily selected by one of ordinary skill in the art. Specific procedures are provided in the Examples section. Reactions can be worked up in the conventional manner, *e.g*. by eliminating the solvent from the residue and further purified according to methodologies generally known in the art such as, but not limited to, crystallization, distillation, extraction, trituration and chromatography. Unless otherwise described, the starting materials and reagents are either commercially available or can be prepared by one skilled in the art from commercially available materials using methods described in the chemical literature.

Routine experimentations, including appropriate manipulation of the reaction conditions, reagents and sequence of the synthetic route, protection of any chemical functionality that cannot be compatible with the reaction conditions, and deprotection at a suitable point in the reaction sequence of the method are included in the scope of the invention. Suitable protecting groups and the methods for protecting and deprotecting different substituents using such suitable protecting groups are well known to those skilled in the art; examples of which can be found in T. Greene and P. Wuts, Protective Groups in Organic Synthesis (3rd ed.), John Wiley & Sons, NY (1999).

Synthesis of the compounds of the invention can be accomplished by methods analogous to those described in the synthetic schemes described hereinabove and in specific examples.

Starting materials, if not commercially available, can be prepared by procedures selected from standard organic chemical techniques, techniques that are analogous to the synthesis of known, structurally similar compounds, or techniques that are analogous to the above described schemes or the procedures described in the synthetic examples section.

When an optically active form of a compound of the invention is required, it can be obtained by carrying out one of the procedures described herein using an optically active starting material (prepared, for example, by asymmetric induction of a suitable reaction step), or by resolution of a mixture of the stereoisomers of the compound or intermediates using a standard procedure (such as chromatographic separation, recrystallization or enzymatic resolution).

Similarly, when a pure geometric isomer of a compound of the invention is required, it can be obtained by carrying out one of the above procedures using a pure geometric isomer as a starting material, or by resolution of a mixture of the geometric isomers of the compound or intermediates using a standard procedure such as chromatographic separation.

It can be appreciated that the synthetic schemes and specific examples as illustrated in the Examples section are illustrative and are not to be read as limiting the scope of the invention as it is defined in the appended claims. All alternatives, modifications, and equivalents of the synthetic methods and specific examples are included within the scope of the claims.

### E. EXAMPLES

Abbreviations: APCI for atmospheric pressure chemical ionization; ESI for electrospray ionization; HPLC for high performance liquid chromatography; LC-MS for liquid chromatography-mass spectrometry; and psig for pounds per square inch gas.

### Analytical LC-MS on Thermo-Finnigan Navigator instruments (Open-Access):

Analytical LC-MS was performed on a Finnigan Navigator mass spectrometer and Agilent 1100 HPLC system running Xcalibur 1.2, Open-Access 1.3, and custom login software. The mass spectrometer was operated under positive APCI ionization conditions. The HPLC system comprised an Agilent Quaternary pump, degasser, column compartment, autosampler and diode-array detector, with a Polymer Labs ELS-2100 evaporative light-scattering detector. The column used was a Phenomenex® Luna® Combi-HTS C8(2) 5µm 100Å (2.1 mm × 50 mm), at a temperature of 55 °C. A gradient of 20-100% acetonitrile (A) and 0.1% trifluoroacetic acid in water (B) was used, at a flow rate of 2.0 mL/minute (0-0.1 minutes 20% A, 0.1-2.6 minutes 20-100% A, 2.6-2.9 minutes 100% A, 2.9-3.0 minutes 100-20% A. 0.5 minutes post-run delay).

### Preparative LC Method 1:

Samples were purified by preparative HPLC on a Phenomenex® Luna® C8(2) 5 µm 100Å AXIA™ column (50 mm × 21.2 mm). A gradient of acetonitrile (A) and 0.1% trifluoroacetic acid in water (B) was used, at a flow rate of 30 mL/minute (0-0.5 minute 5% A, 0.5-6.5 minute linear gradient 5-100% A, 6.5-8.5 minute 100% A, 8.5-9.0 minute linear gradient 100-5% A, 9.0-10 minute 5% A). A sample volume of 1.0 mL was injected directly from the flow reactor stream to the HPLC system. A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Preparative LC Method 2:

Samples were purified by preparative HPLC on a Phenomenex® Luna® C8(2) 5 µm 100Å AXIA™ column (50 mm × 21.2 mm). A gradient of acetonitrile (A) and 0.1% ammonium acetate in water (B) was used, at a flow rate of 30 mL/minute (0-0.5 minute 5% A, 0.5-6.5 minute linear gradient 5-100% A, 6.5-8.5 minute 100% A, 8.5-9.0 minute linear gradient 100-5% A, 9.0-10 minute 5% A). A sample volume of 1.0 mL was injected directly from the flow reactor stream to the HPLC system. A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Preparative LC Method 3:

Samples were purified by preparative HPLC on a Phenomenex® Luna® C8(2) 5 µm 100Å AXIA™ column (50 mm × 21.2 mm). A gradient of acetonitrile (A) and 0.1% ammonium acetate in water (B) was used, at a flow rate of 30 mL/minute (0-0.1 minute 5% A, 0.1-0.5 minute linear gradient 5-30% A, 0.5-6.5 minute linear gradient 30-100% A, 6.5-8.5 minute 100% A, 8.5-9.0 minute linear gradient 100-5% A, 9.0-10 minute 5% A). A sample volume of 1.0 mL was injected directly from the flow reactor stream to the HPLC system. A custom purification system was used, consisting of the following modules: Gilson 305 and 306 pumps; Gilson 806 Manometric module; Gilson UV/Vis 155 detector; Gilson 506C interface box; Gilson FC204 fraction collector; Agilent G1968D Active Splitter; Thermo MSQ Plus mass spectrometer. The system was controlled through a combination of Thermo Xcalibur 2.0.7 software and a custom application written in-house using Microsoft Visual Basic 6.0.

### Example 1

(2*S*,4a*S*,6a*S*,6b*R*,8a*R*,12a*S*,14a*S*,14b*R*)-methyl 11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate

### Example 1A

### (2S,4aS,6aS,6bR,8aR,10S,12aS,12bR,14bR)-methyl 10-hydroxy-2,4a,6a,6b,9,9,12a-heptamethyl-13-oxo-1,2,3,4,4a,5,6,6a,6b,7,8,8a,9,10,11,12,12a,12b,13,14b-icosahydropicene-2-carboxylate

Glycyrrhetinic acid (19.92 g, 42.3 mmol) was suspended in mixture of diethyl ether (116 mL) and methanol (116 mL). (Trimethylsilyl)diazomethane solution (31.7 mL, 63.5 mmol, 2 M in diethyl ether) was added via an addition funnel over 20 minutes. After 10 mL of (trimethylsilyl)diazomethane solution was added, the reaction solution became clear. After addition of another 10 mL of (trimethylsilyl)diazomethane solution, the reaction mixture started to turn cloudy again and remained a suspension for the rest of the reaction. LC-MS analysis 1 hour after all the (trimethylsilyl)diazomethane solution was added showed approximately 5% glycyrrhetinic acid remaining. Another 10 mL of (trimethylsilyl)diazomethane solution was added, and the mixture was stirred overnight. LC-MS at this point showed complete consumption of the glycyrrhetinic acid. Acetic acid (5.33 mL, 93 mmol) was added portionwise. The first portions of acetic acid produced some gas evolution. The latter portions showed no further bubbling. The mixture was concentrated and the titled compound was taken forward without additional purification. ¹H NMR (400 MHz, CDCl₃) δ ppm 5.67 (s, 1H), 3.69 (d, *J=* 3.1 Hz, 3H), 3.22 (dt, *J=* 11.0, 5.6 Hz, 1H), 2.84 - 2.76 (m, 1H), 2.11 - 1.97 (m, 3H), 1.96 - 1.88 (m, 1H), 1.82 (tt, *J* = 14.6, 4.7 Hz, 1H), 1.69 - 1.57 (m, 5H), 1.50 - 1.24 (m, 9H), 1.21 (dd, *J* = 5.5, 3.3 Hz, 1H), 1.15 (dd, *J* = 11.3, 7.5 Hz, 9H), 1.05 - 0.94 (m, 5H), 0.81 (s, 6H), 0.70 (dd, *J=* 8.7, 3.1 Hz, 1H); MS (ESI) *m*/*z* 485.3 (M+H)⁺.

### Example 1B

### (2S,4aS,6aS,6bR,8aR,10S,12aR,12bR,14bR)-methyl 10-hydroxy-2,4a,6a,6b,9,9,12a-heptamethyl-1,2,3,4,4a,5,6,6a,6b,7,8,8a,9,10,11,12,12a,12b,13,14b-icosahydropicene-2-carboxylate

Platinum(IV) oxide (79.8 mg, 0.351 mmol, 32 weight%) in glacial acetic acid (2.5 mL) was stirred under 60 psig of hydrogen for 10 minutes at room temperature in a 20 mL glass pressure bottle. The product of Example 1A (250.2 mg, 0.516 mmol) and acetic acid (10.0 mL) were added. The mixture was stirred under 60 psig of hydrogen at room temperature for 15 hours. HPLC analysis indicated < 1% of the product of Example 1A. The product mixture was a white slurry. Dichloromethane (7.5 mL) was added, the resultant mixture was filtered through a 0.45 µm nylon membrane. The filtrate was concentrated to give the titled compound as a foamy solid that was used in the next step without additional purification. MS (APCI) *m*/*z* 471 (M+H)⁺, 453 [M-H₂O+H]⁺.

HPLC Analysis Method: Supelco® Ascentis® Express C8 column (4.6 × 100 mm, 2.7 µm fused silica), 20% to 90% CH₃CN in 0.1% aqueous HiPO₄ over 3 minutes, then hold for 9 minutes at 90% CH₃CN, flow rate 1.5 mL/minute, 30 °C, 210 nm. Samples containing 1 mg of substance were homogenized by dilution with 1 mL of CH₃CN and mild warming. Observed retention times: starting material 4.57 minutes, titled compound 6.12 minutes.

### Example 1C

### (2S,4aS,6aS,6bR,8aR,10S,12aR,12bR,14bR)-methyl 10-acetoxy-2,4a,6a,6b,9,9,12a-heptamethyl-1,2,3,4,4a,5,6,6a,6b,7,8,8a,9,10,11,12,12a,12b,13,14b-icosahydropicene-2-carboxylate

To a turbid solution of the product of Example 1B (10.13 g, 21.52 mmol) in pyridine (200 mL) was added acetyl chloride (4.59 mL, 64.6 mmol) dropwise at 0 °C. The resultant suspension was stirred at 0 °C for 40 minutes and then warmed to room temperature and stirred for 1 hour. LC-MS indicated incomplete reaction. The reaction mixture was cooled to 0 °C, and more acetyl chloride (1.6 mL, 1 equivalent) was added. The suspension was stirred at room temperature for 1 hour. LC-MS indicated consumption of starting material. The reaction mixture was cooled to 0 °C, and dichloromethane (300 mL) was added followed by the slow addition of aqueous 3 N HCl (150 mL). The layers were separated, and the organic layer was washed with aqueous HCl (3 N, 150 mL × 4 then 6 N, 150 mL × 2) until the aqueous layer showed pH = 1. The combined aqueous layers (pH = 5) were back extracted with ethyl acetate (200 mL × 2). The combined organic layers were dried (MgSO₄) and concentrated to give the titled compound (12.59 g, 24.55 mmol, 114 % yield) as a light yellow solid. ¹H NMR (400 MHz, CDCl₃) δ ppm 5.27 (t, *J* = 3.5 Hz, 1H), 4.53 - 4.48 (m, 1H), 3.68 (s, 3H), 2.05 (s, 3H), 2.00 - 1.84 (m, 6H), 1.76 (tt, *J* = 12.5, 4.4 Hz, 1H), 1.67 - 1.59 (m, 5H), 1.50 (ddd, *J=* 9.1, 3.0, 2.3 Hz, 4H), 1.46 - 1.23 (m, 6H), 1.17 - 1.10 (m, 6H), 1.08 - 0.98 (m, 2H), 0.96 (s, 6H), 0.87 (d, *J=* 4.1 Hz, 6H), 0.81 - 0.76 (m, 3H).

### Example 1D

### (2S,4aS,6aR,6bR,8aR,10S,12aR,12bR,14aR,14bS)-methyl 10-acetoxy-2,4a,6a,6b,9,9,12a-heptamethyl-14-oxodocosahydropicene-2-carboxylate

To a turbid solution of the product of Example 1C (8.8 g, 17.16 mmol) in dichloroethane (150 mL) was added dichloroacetic acid (7.08 mL, 86 mmol) and an aqueous solution of 30% hydrogen peroxide (8.77 mL, 86 mmol). The solution was stirred at 40 °C overnight. LC-MS showed consumption of starting material. The reaction mixture was cooled to 0 °C, diluted with dichloromethane (50 mL), and quenched with a solution of 10% Na₂SO₃ (15 mL). The organic layer was checked for peroxide with a Baker peroxide test strip and washed with water (15 mL). The combined aqueous layers were back extracted with dichloromethane (10 mL × 2). The combined organic layers were dried (MgSO₄) and concentrated to give the titled compound (9.21 g, 17.42 mmol, 101 % yield). ¹H NMR (400 MHz, CDCl₃) δ ppm 4.47 (dt, *J=* 9.1, 4.6 Hz, 1H), 3.72 (s, 3H), 2.76 (t, *J=* 4.7 Hz, 1H), 2.60 - 2.53 (m, 1H), 2.31 - 2.25 (m, 1H), 2.20 - 2.11 (m, 1H), 2.05 (s, 3H), 1.99 - 1.74 (m, 4H), 1.70 - 1.58 (m, 3H), 1.55 - 1.34 (m, 5H), 1.31 - 1.20 (m, 3H), 1.13 (d, *J* = 7.1 Hz, 6H), 1.00 (ddd, *J=* 11.6, 11.0, 7.0 Hz, 2H), 0.94 - 0.83 (m, 17H); MS (ESI) *m*/*z* 529.4 (M+H)⁺.

### Example 1E

### (2S,4aS,6aR,6bS,8aR,10S,12aS,14aR,14bS)-methyl 10-acetoxy-2,4a,6a,6b,9,9,12a-heptamethyl-14-oxo-1,2,3,4,4a,5,6,6a,6b,7,8,8a,9,10,11,12,12a,14,14a,14b-icosahydropicene-2-carboxylate

To a suspension of the product of Example 1D (9.21 g, 17.42 mmol) in acetic acid (85 mL) was added HBr (0.095 mL, 1.742 mmol) and a solution of Br2 (1.167 mL, 22.64 mmol) in acetic acid (10 mL). The suspension gradually turned clear and was stirred at room temperature for 3 hours. LC-MS showed complete consumption of starting material. After stirring overnight, LC-MS showed no change. The reaction solution was slowly poured into a beaker of ice. The solid was collected by filtration and rinsed with water. The solid was dissolved in dichloromethane (200 mL) and washed with water (50 mL) and a solution of 10% sodium sulfite (50 mL) (The aqueous layer pH was ~ 7). The combined aqueous layers were back extracted with dichloromethane (20 mL × 2). The combine organic layers were dried (MgSO₄) and concentrated to give the titled compound (9.38 g, 17.81 mmol, 102 % yield) as a yellow solid. ¹H NMR (400 MHz, CDCl₃) δ ppm 5.79 (s, 1H), 4.49 (dd, *J* = 11.7, 4.7 Hz, 1H), 3.76 (s, 3H), 2.99 (d, *J* = 4.7 Hz, 1H), 2.22 (dt, *J* = 13.1, 2.8 Hz, 1H), 2.22 (dt, *J* = 13.1, 2.8 Hz, 1H), 2.07 (s, 3H), 1.97 (dd, *J* = 9.7, 3.6 Hz, 2H), 1.90 - 1.81 (m, 2H), 1.80 - 1.60 (m, 5H), 1.54 - 1.43 (m, 3H), 1.43 - 1.36 (m, 3H), 1.35 - 1.20 (m, 7H), 1.13 (s, 3H), 1.09 - 0.99 (m, 2H), 0.97 (s, 3H), 0.92 (d, *J=* 4.8 Hz, 9H); MS (ESI) *m*/*z* 527.2 (M+H)⁺.

### Example IF

### (2S,4aS,6aR,6bS,8aR,10S,12aS,14aR,14bS)-methyl 10-hydroxy-2,4a,6a,6b,9,9,12a-heptamethyl-14-oxo-1,2,3,4,4a,5,6,6a,6b,7,8,8a,9,10,11,12,12a,14,14a,14b-icosahydropicene-2-carboxylate

To the product of Example 1E (9.38 g, 17.81 mmol) was added dichloromethane (20 mL), tetrahydrofuran (53 mL) and methanol (26 mL). To the resultant solution was added water (26 mL) (solid appeared), and the mixture was cooled to 0 °C. Lithium hydroxide hydrate (1.499 g, 35.7 mmol) was added, and the mixture was stirred at 40 °C (the mixture turned clear) overnight. LC-MS showed complete consumption of starting material. The mixture was cooled to 0 °C and quenched with a solution of aqueous HCl (0.5 N, 50 mL). The mixture was extracted with dichloromethane (200 mL). The aqueous layer was back extracted with dichloromethane (20 mL × 2). The combined organic layers were dried (MgSO₄), filtered, and concentrated. The residue was purified chromatographically using a Biotage® SNAP 340 g silica cartridge eluted with a step gradient of ethyl acetate/dichloromethane (1 column volume (CV) 0%, 8 CV 0-30%, 2 CV 30% then 3 CV 50%) giving the titled compound (6.29 g) as a yellow foamy solid and impure fractions (450 mg). The impure material was purified chromatographically using a Biotage® SNAP 50 g silica cartridge eluted with a step gradient of ethyl acetate/dichloromethane (gradient described above) and gave additional titled compound (267.2 mg). The batches were combined to give the titled compound (6.5572 g, 13.53 mmol, 76 % yield). ¹H NMR (400 MHz, benzene-*d₆*) δ ppm 5.54 (s, 1H), 3.27 (s, 3H), 2.56 (d, *J=* 4.7 Hz, 1H), 2.50 - 2.37 (m, 2H), 2.00 (d, *J* = 13.4 Hz, 1H), 1.92 - 1.85 (m, 1H), 1.58 (td, *J* = 13.9, 4.3 Hz, 1H), 1.43 (dt, *J=* 13.2, 6.5 Hz, 1H), 1.28 - 1.13 (m, 3H), 1.07 (d, *J* = 15.6 Hz, 2H), 0.93 (dddd, *J* = 32.0, 17.9, 9.5, 4.3 Hz, 10H), 0.81 (s, 3H), 0.79 - 0.72 (m, 2H), 0.71 - 0.64 (m, 5H), 0.60 - 0.56 (m, 6H), 0.55 - 0.40 (m, 11H), 0.37 (d, *J* = 4.3 Hz, 3H), 0.30 (s, 1H); MS (ESI) *m*/*z* 485.3 (M+H)⁺.

### Example 1 G

### (2S,4aS,6aS,6bR,8aR,10S,12aS,14aS,14bR)-methyl 10,14a-dihydroxy-2,4a,6a,6b,9,9,12a-heptamethyl-14-oxo-1,2,3,4,4a,5,6,6a,6b,7,8,8a,9,10,11,12,12a,14,14a,14b-icosahydropicene-2-carboxylate

The product of Example IF (1.499 g, 3.09 mmol) and tetrahydrofuran (30 mL) were combined, and the solution was sparged with nitrogen for 20 minutes. Potassium hydride (30% in mineral oil, 2 portions (each ∼0.85 mg); ∼3.4 equivalents) was added at 0 °C. The mixture was stirred at 0 °C for 5 minutes and then at room temperature for 1.25 hours while sparging throughout. The reaction mixture was cooled to -30 °C, and 3-phenyl-2-(phenylsulfonyl)-1,2oxaziridine (2.9963 g, 10.89 mmol) was added slowly from a solid addition funnel. The reaction mixture was allowed to gradually warm to room temperature with continued stirring overnight. LC-MS indicated product formation. The reaction mixture was quenched with nitrogen sparged aqueous HCl (1 N, 10.5 mL) and stirred for 5 minutes at 0 °C. The mixture was extracted with dichloromethane (150 mL). The organic layer was washed with water (50 mL × 2). The combined aqueous layers were back extracted with dichloromethane (20 mL × 2). The combined organic layers were dried (MgSO₄), filtered, and concentrated. The residue was purified chromatographically on a Biotage® SNAP 100 g silica cartridge eluted with a step gradient of CH₃CN/CHCl₃ (2 CV 0%, 8 CV 0-15%, 3 CV 15%, 5 CV 15-30% then 5 CV 30%) giving impure batches of the titled compound. The impure material was subjected to a second chromatography using a Biotage® SNAP 10 g silica cartridge eluted with a step gradient of CH₃CN/CHCl₃ (gradient described above). Pure compound from the chromatographic purifications were combined to give the titled compound (629.32 mg, 1.257 mmol, 40.6 % yield). ¹H NMR (400 MHz, CDCl₃) δ ppm 5.81 (s, 1H), 3.77 (s, 3H), 3.20 (dd, *J=* 11.5, 4.6 Hz, 1H), 2.22 - 2.05 (m, 3H), 1.93 (dt, *J=* 12.9, 3.5 Hz, 1H), 1.85 - 1.63 (m, 7H), 1.47 - 1.16 (m, 15H), 1.18 - 1.07 (m, 4H), 1.03 (s, 3H), 0.97 (s, 3H), 0.93 - 0.80 (m, 5H).

### Example 1H

### (2S,4aS,6aS,6bR,8aR,12aS,14aS,14bR)-methyl 14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate

Batch 1: To the product of Example 1G (126.92 mg, 0.253 mmol) was added toluene (1.5 mL) and dimethyl sulfoxide (0.108 mL, 1.521 mmol) followed by 1-hydroxy-1,2-benziodoxol-3(1*H*)-one 1-oxide (355 mg, 1.267 mmol). The reaction mixture was stirred at 90 °C overnight. The reaction mixture was cooled and combined with Batch 2.

Batch 2: To the product of Example 1G (502.4 mg, 1.003 mmol) was added toluene (6 mL) and dimethyl sulfoxide (0.427 mL, 6.02 mmol) followed by 1-hydroxy-1,2-benziodoxol-3(1*H*)-one 1-oxide (1405 mg, 5.02 mmol). The reaction mixture was stirred at 85 °C overnight. LC-MS showed incomplete reaction. The reaction mixture was then stirred at 92 °C for 7 hours, and LC-MS indicated predominantly product formation. The reaction mixture was cooled and combined with Batch 1.

The combined Batch 1 and Batch 2 were diluted with dichloromethane (150 mL) and washed with a 5% aqueous solution of NaHCO₃ (50 mL × 2). The combined aqueous layers were extracted with dichloromethane (50 mL × 2). The combined organic layers were dried (MgSO₄), filtered, and concentrated. The residue was purified chromatographically on a Biotage® SNAP 100 g silica cartridge eluted with a gradient of 0-25% ethyl acetate/dichloromethane to give the titled compound (416 mg, 0.838 mmol, 67 % yield). ¹H NMR (400 MHz, CDCl₃) δ ppm 7.35 (d, *J=* 10.5 Hz, 1H), 6.03 (s, 1H), 5.91 (d, *J=* 10.5 Hz, 1H), 3.78 (s, 4H), 2.18 (t, *J=* 13.5 Hz, 3H), 1.88 (t, *J=* 13.8 Hz, 1H), 1.73 (dd, *J=* 17.4, 6.7 Hz, 5H), 1.56 (s, 3H), 1.46 (s, 3H), 1.41 (d, *J* = 10.1 Hz, 1H), 1.36 - 1.34 (m, 1H), 1.29 (s, 3H), 1.19 (d, *J=* 7.8 Hz, 4H), 1.12 (d, *J=* 7.5 Hz, 10H), 0.98 (d, *J=* 4.9 Hz, 3H); MS (ESI) *m*/*z* 497.2 (M+H)⁺.

### Example 1I

### (2S,4aS,6aS,6bR,8aR,12aR,14aS,14bR)-methyl 14a-hydroxy-11-iodo-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate

To the product of Example 1H (416 mg, 0.838 mmol) was added pyridine (0.677 mL, 8.38 mmol) and I₂ (850 mg, 3.35 mmol). The reaction mixture was refluxed at 80 °C overnight. The reaction mixture was cooled to room temperature and poured onto ice. The resultant mixture was partitioned with dichloromethane (100 mL), and the layers were separated. The organic layer was washed with an aqueous solution of sodium thiosulfate (0.1 N, 20 mL × 3), HCl (1 N, 20 mL) and aqueous Na₂S₂O₃ (10%, 20 mL). The combined aqueous layers were back extracted with dichloromethane (20 mL × 2). The combined organic layers were dried and concentrated. The residue was purified chromatographically using a Biotage® SNAP 50 g silica cartridge eluted with a gradient of 0-5% ethyl acetate/dichloromethane to give the titled compound (398.88 mg, 0.641 mmol, 76 % yield). ¹H NMR (400 MHz, CDCl₃) δ ppm 8.17 (s, 1H), 6.03 (s, 1H), 3.78 (s, 3H), 2.24 - 2.07 (m, 3H), 1.99 - 1.64 (m, 7H), 1.51 - 1.34 (m, 5H), 1.33 - 1.23 (m, 11H), 1.21 - 1.06 (m, 8H), 1.00 (s, 3H); MS (ESI) *m*/*z* 623.1 (M+H)⁺.

### Example 1J

### (2S,4aS,6aS,6bR,8aR,12aS,14aS,14bR)-methyl 11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate

Batch 1: To the product of Example 1I (60 mg, 0.096 mmol) was added 1-methyl-2-pyrrolidinone (1 mL), and the resultant solution was sparged for 10 minute with nitrogen. Copper(I) cyanide (12.21 mg, 0.135 mmol) was added, and the solution was sparged with nitrogen and stirred at 120 °C for 2 hours. LC/MS showed incomplete reaction. Additional copper(I) cyanide (7.38 mg, 0.082 mmol) was added, and the solution was sparged with nitrogen and stirred at 120 °C for 1 hour. The reaction mixture was cooled to room temperature, concentrated, and combined with Batch 2.

Batch 2. To the product of Example 1I (398.88 mg, 0.641 mmol) was added 1-methyl-2-pyrrolidinone (6 mL), and the resultant solution was sparged for 10 minute with nitrogen. Copper(I) cyanide (81 mg, 0.897 mmol) was added, and the solution was sparged with nitrogen and stirred at 120 °C for 2 hours. The reaction mixture was cooled to 0 °C, water was added, and a solid formed. The solid was collected by filtration, rinsed with water, and combined with the solid from Batch 1. The solid was rinsed with acetone and then with dichloromethane. The filtrate was concentrated to give a gray solid. The solid was purified chromatographically on a Biotage® SNAP 25 g silica cartridge eluted with a gradient of 0-33% ethyl acetate/hexane that gave pure fractions (160 mg) and impure fractions (85 mg). The impure material was subjected to a second chromatography on a Biotage® SNAP 10 g silica cartridge eluted with a gradient of 0-20% ethyl acetate/dichloromethane that gave pure fractions and impure fractions. The impure material was purified by preparative TLC (20% ethyl acetate/dichloromethane). All the pure fractions were combined to give the titled compound (202.8 mg, 53% yield, 98% purity by HPLC). ¹H NMR (400 MHz, benzene-*d*₆) δ ppm 7.40 (s, 1H), 5.74 (s, 1H), 3.73 (s, 3H), 2.45 (dd, *J=* 13.5, 6.1 Hz, 2H), 2.04 - 1.93 (m, 1H), 1.84 (d, *J=* 12.7 Hz, 1H), 1.60 - 1.40 (m, 2H), 1.30 - 0.98 (m, 18H), 0.93 - 0.85 (m, 3H), 0.78 - 0.71 (m, 4H), 0.66 (d, *J=* 2.2 Hz, 5H), 0.45 (s, 2H); MS (ESI) *m*/*z* 522.1 (M+H)⁺.

### Example 2

### (comparative example)

(2*S*,4a*S*,6a*R*,6b*S*,8a*R*,12a*S*,14a*R*,14b*S*)-methyl 11-cyano-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate

### Example 2A

(2*S*,4a*S*,6a*R*,6b*S*,8a*R*,12a*S*,14a*R*,14b*S*)-methyl 2,4a,6a,6b,9,9,12a-heptamethyl-10,14-dioxo-1,2,3,4,4a,5,6,6a,6b,7,8,8a,9,10,11,12,12a,14,14a,14b-icosahydropicene-2-carboxylate To the product of Example IF (0.65 g, 1.341 mmol) was added dichloromethane (27 mL) followed by sodium bicarbonate (0.597 g, 7.11 mmol) and Dess-Martin periodinane (0.844 g, 1.990 mmol). The mixture was stirred at room temperature for 1 hour and LC-MS showed complete reaction. The mixture was filtered and rinsed with dichloromethane, and the filtrate was concentrated. The residue was purified chromatographically on a Biotage® SNAP 100 g silica cartridge eluted with a gradient of 0-33% ethyl acetate/hexane yielding the titled compound (0.55 g, 1.139 mmol, 85 % yield). ¹H NMR (400 MHz, CDCl₃) δ ppm 5.86 - 5.81 (m, 1H), 3.76 (s, 3H), 3.05 - 3.01 (m, 1H), 2.71 - 2.61 (m, 1H), 2.53 - 2.46 (m, 1H), 2.25 - 2.19 (m, 2H), 2.04 - 1.95 (m, 2H), 1.91 - 1.65 (m, 6H), 1.53 - 1.48 (m, 2H), 1.43 (s, 3H), 1.35 - 1.20 (m, 9H), 1.12 (dd, *J* = 9.6, 3.7 Hz, 9H), 0.99 (s, 3H), 0.94 (s, 3H); MS (ESI) *m*/*z* 483.3 (M+1)+.

### Example 2B

### (2S,4aS,6aR,6bS,8aR,12aS,14aR,14bS)-methyl 11-cyano-10-hydroxy-2,4a,6a,6b,9,9,12a-heptamethyl-14-oxo-1,2,3,4,4a,5,6,6a,6b,7,8,8a,9,12,12a,14,14a,14b-octadecahydropicene-2-carboxylate

To a solution the product of Example 2A (17.6 mg, 0.036 mmol) in tetrahydrofuran (0.5 mL) was added lithium diisopropylamide (1.8 M in tetrahydrofuran/heptane/ethylbenzene, 65 µL, 0.117 mmol) at -78 °C. The solution was stirred at -78 °C for 1 hour and *p*-toluene sulfonyl cyanide (9.91 mg, 0.055 mmol) was added. The reaction solution was gradually warmed to room temperature and stirred overnight. The reaction was quenched with a saturated aqueous solution of NHₐCl (5 mL) and extracted with dichloromethane (15 mL). The organic layer was washed with water (5 mL), dried (MgSO₄), filtered, and concentrated. The residue was purified chromatographically on a Biotage® SNAP 10 g silica cartridge eluted with a gradient of CH₃CN/CHCl₃ (3 CV 0%, 3 CV 0-5%, 4 CV 5%, 3 CV 5-15% and 6 CV 15%) giving the titled compound (14.82 mg, 0.029 mmol, 80 % yield). ¹H NMR (400 MHz, CDCl₃) δ ppm 5.83 - 5.73 (m, 1H), 3.76 (s, 3H), 3.03 (dd, *J=* 11.1, 4.8 Hz, 1H), 2.37 - 2.16 (m, 1H), 2.10 - 1.94 (m, 2H), 1.93 - 1.61 (m, 5H), 1.49 - 1.11 (m, 21H), 1.03 (s, 1H), 1.00 - 0.91 (m, 6H), 0.92 - 0.82 (m, 3H); MS (ESI) *m*/*z* 508.2 (M+H)⁺.

### Example 2C

### (2S,4aS,6aR,6bS,8aR,12aS,14aR,14bS)-methyl 11-cyano-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylate

The product of Example 2B (14.1 mg, 0.028 mmol) and toluene (600 µL) were combined and 2,3-dichloro-5,6-dicyano-*p*-benzoquinone (DDQ) (6.93 mg, 0.031 mmol) was added at room temperature. The solution was stirred overnight and LC-MS showed complete reaction. The reaction mixture was concentrated, and the residue was purified chromatographically on a Biotage® SNAP 10 g silica cartridge eluted with a gradient of CH₃CN/CHCl₃ (3 CV 0%, 7 CV 0-10%, 5 CV 10%, 5 CV 10-15%, 4 CV 15%) furnishing the titled compound (4.62 mg, 9.14 µmol, 32.9 % yield). ¹H NMR (400 MHz, CDCl₃) δ ppm 8.04 (s, 1H), 6.00 (s, 1H), 3.76 (s, 3H), 3.06 (d, *J=* 4.7 Hz, 1H), 2.21 (dt, *J=* 13.2, 3.0 Hz, 1H), 2.07 - 1.78 (m, 7H), 1.77 - 1.60 (m, 1H), 1.55 (s, 4H), 1.52 (d, *J=* 4.0 Hz, 3H), 1.38 - 1.27 (m, 2H), 1.29 - 1.22 (m, 5H), 1.24 - 1.07 (m, 8H), 1.00 (s, 3H), 0.94 (s, 3H); MS (ESI) *m*/*z* 506.3 (M+H)⁺.

### Example 3

(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N-*(1,3-oxazol-5-ylmethyl)-10,14-dioxo-1,2,3,4,4a,5,6,6a,6b,7,8,8a,9,10,12a,14,14a,14b-octadecahydropicene-2-carboxamide

### Example 3A

### (2S,4aS,6aS,6bR,8aR,12aS,14aS,14bR)-methyl 14a-hydroxy-2,4a,6a,6b,9,9,12a-heptamethyl-10,14-dioxo-1,2,3,4,4a,5,6,6a,6b,7,8,8a,9,10,11,12,12a,14,14a,14b-icosahydropicene-2-carboxylate

To a stirred solution of (2*S*,4a*S*,6a*S*,6b*R*,8a*R*,10*S*,12a*S*,14a*S*,14b*R*)-methyl 10,14a-dihydroxy-2,4a,6a,6b,9,9,12a-heptamethyl-14-oxo-1,2,3,4,4a,5,6,6a,6b,7,8,8a,9,10,11,12,12a,14,14a,14b-icosahydropicene-2-carboxylate (18.25 g, 31.3 mmol, Example 1 G) in dichloromethane (292 mL) was added sodium hydrogencabonate (18.43 g, 219 mmol). The suspension was cooled to 5 °C. Dess-Martin periodinane (17.82 g, 40.7 mmol) was added in six portions before the mixture was stirred between 5-10 °C for 1 hour. Water (300 mL) was added, and the mixture was allowed to reach ambient temperature. The layers were separated, and the organic layer was washed with water (300 mL), 10% sodium bisulfite (2× 300 mL), water (300 mL), 7% sodium hydrogencabonate (200 mL), and 10% sodium chloride (200 mL). The organic fraction was dried (anhydrous sodium sulfate), filtered, and then concentrated under reduced pressure to provide a residue that was purified by flash chromatography: 30-60% ethyl acetate/heptanes. The fractions containing the titled compound were combined and concentrated under reduced pressure. Heptanes (100 mL) were added to the solids which were stirred for 1 hour. The solids were collected by filtration to give the titled compound (12.9 g, 83% yield). ¹H NMR (400 MHz, CDCl₃) δ ppm 1.01 (s, 3H), 1.06 - 1.21 (m, 10H), 1.19 - 1.36 (m, 10H), 1.34 - 1.51 (m, 6H), 1.63 - 1.96 (m, 7H), 2.10 - 2.32 (m, 4H), 2.49 (ddd, J = 15.7, 6.3, 3.4 Hz, 1H), 2.74 (ddd, J = 15.7, 12.0, 6.8 Hz, 1H), 3.80 (d, J = 0.8 Hz, 3H), 5.86 (s, 1H); MS (ESI) m/z 499 (M+H)⁺.

### Example 3B

### methyl (2S,4aS,6aS,6bR,8aR,13aS,15aS,15bR)-15a-hydroxy-2,4a,6a,6b,9,9,13a-heptamethyl-15-oxo-1,2,3,4,4a,5,6,6a,6b,7,8,8a,9,13,13a,15,15a,15b-octadecahydropiceno[2,3-d][1,2]oxazole-2-carboxylate

Ethyl formate (103 g, 1390 mmol) and the compound of Example 3A (15.4 g, 30.4 mmol) were stirred together to give a white suspension which was cooled to 5 °C. Sodium methoxide 25 weight% (14.0 g, 64.8 mmol) was added, and the mixture was stirred at 5 °C for 3 hours and then at 0 °C for an additional 3 hours. The mixture was cooled to -8 °C. In a separate container, phosphoric acid (10.68 g, 93 mmol) and ethanol (100 mL) were mixed and then cooled to 0 °C. The cold acidic solution was then added to the mixture in ethyl formate set at below -4.5 °C. Water (15 mL) was added to the white slurry that formed before the temperature was brought up to 25 °C. Hydroxylamine hydrochloride (4.40 g, 63.3 mmol) was added, and the reaction mixture was stirred at 50 °C for 9 hours. The reaction mixture was cooled to 0 °C before water (70 mL) was added. The slurry was filtered, and the wet-cake was rinsed with water (60 mL). The filtrate and rinse were combined and concentrated in vacuo with a bath temperature set at 30 °C. The resulting solids were collected by filtration and rinsed with water. The combined solids were dissolved in ethyl acetate (200 mL). The organic solution was washed with water (2×70 mL) and 10% sodium chloride (80 mL) and then dried over anhydrous sodium sulfate. The solution was filtered and then concentrated in vacuo. The solid residue was dissolved in methanol (24 mL) and stirred at ambient temperature for 1 hour. The solids formed were collected by filtration and then rinsed with a minimal amount of methanol and heptanes. The solids were dried in vacuo at 40 °C for 16 hours to give the titled compound (12.9 g, 83% yield). ¹H NMR (400 MHz, CDCl₃) δ ppm 1.04 (s, 3H), 1.17 (s, 3H), 1.12-1.21 (m, 1H), 1.22 (s, 3H), 1.24-1.35 (m, 10 H), 1.36 - 1.48 (m, 6H), 1.53 (dd, J = 12.4, 2.8 Hz, 1H), 1.59 (s, 3H), 1.61 - 1.95 (m, 6H), 2.10 - 2.30 (m, 3H), 2.48 (d, J = 14.7 Hz, 1H), 2.81 (d, J = 14.8 Hz, 1H), 3.52 (s, 2H), 3.82 (s, 3H), 5.97 (s, 1H), 8.09 (s, 1H); MS (ESI) *m*/*z* 524 (M+H)⁺.

### Example 3C

### (2S,4aS,6aS,6bR,8aR,12aS,14aS,14bR)-11-cyano-10,14a-dihydroxy-2,4a,6a,6b,9,9,12a-heptamethyl-14-oxo-1,2,3,4,4a,5,6,6a,6b,7,8,8a,9,12,12a,14,14a,14b-octadecahydropicene-2-carboxylic acid

To a stirred suspension of the product of Example 3B (12.7 g, 22.8 mmol) in methanol (50 mL), at ambient temperature, was added sodium methoxide 25 weight% (39.4 g, 182 mmol). The resulting solution was stirred at 50 °C for 1 hour, and then cooled to 10 °C. Water (10 mL) and sodium hydroxide 20 weight% (45.6 g, 228 mmol) were added, and the mixture was stirred at ambient temperature for 19 hours. The mixture was cooled to 5 °C, and the pH was adjusted to 2 by the addition of phosphoric acid 20 weight% (268 g, 547 mmol). The formed precipitate was collected by filtration and washed with water (50 mL). The solids were then dissolved in ethyl acetate (100 mL), and the solution was washed with 10% sodium chloride (2× 30 mL) and dried over anhydrous sodium sulfate. The solution was filtered and then concentrated in vacuo to give a solid. The solid was slurried in heptanes (25 mL) and then collected by filtration. The wet-cake was washed with heptanes (15 mL). The solids were further dried in vacuo at 45 °C to give the titled compound (11.5 g, 92% yield). ¹H NMR (400 MHz, DMSO-*d*₆) δ ppm 0.89 (s, 3H), 0.96 - 1.09 (m, 8H), 1.10 - 1.30 (m, 16H), 1.31 - 1.91 (m, 10H), 2.00 - 2.26 (m, 2H), 4.77 (s, 1H), 5.63 (s, 1H), 9.88 (s, 1H), 11.87 (bs, 1H); MS (ESI) *m*/*z* 510 (M+H)⁺.

### Example 3D

### (2S,4aS,6aS,6bR,8aR,12aS,14aS,4bR)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylic acid

A solution of the product of Example 3C (11.5 g, 20.98 mmol) in dimethylformamide (40 mL) was sparged with nitrogen for 5 minutes and then cooled to -3 °C. 1,3-Dibromo-5,5-dimethylhydantoin (3.12 g, 10.91 mmol) was added, and the mixture was stirred at ambient temperature for 40 minutes. The mixture was cooled to 15 °C, and then pyridine (17 mL, 210 mmol) was added. The solution was stirred at 50 °C for 3 hours. The solution was cooled to 5 °C before ethyl acetate (250 mL) and cold (5 °C) phosphoric acid 25 weight% (132 g, 336 mmol) were added. The organic layer was separated and washed with water (4 × 60 mL). The organic layer turned into slurry with white solids. The solids were collected by filtration and rinsed with isopropyl acetate (30 mL), water (20 mL × 4), and heptanes (30 mL), sequentially. The solid was dried in vacuo at 45 °C to give the titled compound (5.4 g). The filtrate from the slurry was washed with water (60 mL), dried over sodium sulfate, and concentrated in vacuo to give a second slurry. The solids were collected from the second slurry by filtration, and the wet-cake was rinsed with isopropyl acetate and heptanes before being dried in vacuo at 45 °C for 16 hours to give more titled compound (5.6 g; total titled compound 11 g, 100% yield). ¹H NMR (400 MHz, CDCl₃) δ ppm 1.03 - 1.25 (m, 8H), 1.32 - 1.55 (m, 10H), 1.58 (s, 3H), 1.73 - 2.13 (m, 14H), 2.22 - 2.35 (m, 2H), 5.08 (s, 1H), 6.10 (s, 1H), 8.10 (s, 1H), 10.78 (br, 1H); MS (ESI) *m*/*z* 508 (M+H)⁺.

### Example 3E

### (2S,4aS,6aS,6bR,8aR,12aS,14aS,14bR)-11-cyano-14a-hydroxy-2,4a,6a,6b,9,9,12a-heptamethyl-N-(1,3-oxazol-5-ylmethyl)-10,14-dioxo-1,2,3,4,4a,5,6,6a,6b,7,8,8a,9,10,12a,14,14a,14b-octadecahydropicene-2-carboxamide

A stock solution of the product of Exampel 3D and *N,N*-diisopropylethylamine (0.10 M and 0.30 M in *N,N*-dimethylacetamide, respectively, 287.5 µL, 0.030 mmol the product of Example 3D, 1.0 equivalent and 0.085 mmol *N,N*-diisopropylethylamine, 2.8 equivalents), *O-*(7-azabenzotriazol-1-yl)-*N,N,N',N'*-tetramethyluronium hexfluorophosphate (0.10 M in *N,N-*dimethylacetamide, 278.5 µL, 0.030 mmol, 1.0 equivalent) and and oxazol-5-yl-methylamine (0.40 M in *N,N*-dimethylacetamide, 221 µL, 0.088 mmol, 3 equivalents) were aspirated from their respective source vials, mixed through a perfluoroalkoxy (PFA) mixing tube (0.2 mm inner diameter), and loaded into an injection loop. The reaction segment was injected into the flow reactor (Hastelloy® coil, 0.75 mm inner diameter, 1.8 mL internal volume) set at 100 °C, and passed through the reactor at 180 µL/minute (10 minute residence time). Upon exiting the reactor, the reaction was loaded directly into an injection loop and purified using preparative LC Method 1 to yield the title compound (0.00636 g, 37% yield). ¹H NMR (400 MHz, DMSO-*d*₆/D₂O) δ ppm 0.88 - 0.95 (m, 3H), 1.03 - 1.10 (m, 7H), 1.15 - 1.37 (m, 12H), 1.49 (s, 3H), 1.51 - 1.59 (m, 4H), 1.64 - 1.97 (m, 8H), 2.14 (dd, J = 13.6, 9.8 Hz, 1H), 4.30 (d, J = 16.7 Hz, 1H), 4.59 (d, J = 15.9, 1H), 6.11 (s, 1H), 7.01 (s, 1H), 8.13 (s, 1H), 8.53 (s, 1H); MS (APCI) *m*/*z* 588.2 (M+H)⁺.

### Example 4

### (2S,4aS,6aS,6bR,8aR,12aS,14aS,14bR)-11-cyano-14a-hydroxy-N-(2-hydroxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with aminoethanol (0.00565 g, 35% yield). ¹H NMR (400 MHz, DMSO-*d*₆/D₂O) δ ppm 0.92 (s, 3H), 0.99 - 1.11 (m, 7H), 1.15 - 1.35 (m, 11H), 1.49 (s, 3H), 1.55 (d, J = 13.6 Hz, 4H), 1.64 - 1.95 (m, 8H), 2.14 (dd, J = 13.8, 10.1 Hz, 1H), 3.27 - 3.31 (m, 2H), 3.56 (t, J = 6.1 Hz, 2H), 6.12 (s, 1H), 8.53 (s, 1H); MS (APCI) *m*/*z* 551.1 (M+H)⁺.

### Example 5

### (2S,4aS,6aS,6bR,8aR,12aS,14aS,14bR)-11-cyano-14a-hydroxy-2,4a,6a,6b,9,9,12a-heptamethyl-10,14-dioxo-N-(pyridazin-4-ylmethyl)-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 4-pyridazinemethanamine (0.00784 g, 44% yield). ¹H NMR (400 MHz, DMSO-*d*₆/D₂O) δ ppm 0.89 - 0.97 (m, 3H), 1.04 - 1.16 (m, 7H), 1.17-1.39 (m, 11H), 1.49 (s, 3H), 1.55 (d, J = 12.8 Hz, 4H), 1.63 - 2.04 (m, 8H), 2.15 (t, J = 11.6 Hz, 1H), 4.34 (d, J = 16.4 Hz, 1H), 4.52 (d, J = 16.2 Hz, 1H), 6.11 (s, 1H), 7.60 (dd, J = 5.4, 2.4 Hz, 1H), 8.52 (s, 1H), 9.12 (dd, J = 5.3, 1.2 Hz, 1H), 9.18 (s, 1H); MS (APCI) *m*/*z* 599.9 (M+H)⁺.

### Example 6

### (2S,4aS,6aS,6bR,8aR,12aS,14aS,14bR)-11-cyano-14a-hydroxy-N,2,4a,6a,6b,9,9,12a-octamethyl-10,14-dioxo-N-propyl-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with *N*-methylpropylamine (0.00792 g, 48% yield). ¹H NMR (400 MHz, DMSO-*d*₆/D₂O) δ ppm 0.86 (t, J = 7.4 Hz, 3H), 0.93 (s, 3H), 1.01 - 1.34 (m, 18H), 1.43 - 1.64 (m, 9H), 1.69 - 1.93 (m, 6H), 2.04 - 2.25 (m, 2H), 2.39 (d, J = 13.4 Hz, 1H), 3.07 (s, 3H), 3.30 (t, J = 7.5 Hz, 2H), 6.04 (s, 1H), 8.50 (s, 1H); MS (APCI) *m*/*z* 563.3 (M+H)⁺.

### Example 7

### (2S,4aS,6aS,6bR,8aR,12aS,14aS,14bR)-11-cyano-N-(2,2-dimethylpropyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 2,2-dimethyl-1-propanamine (0.00814 g, 48% yield). ¹H NMR (400 MHz, DMSO-*d*₆/D₂O) δ ppm 0.90 - 0.98 (m, 12H), 1.09 (t, J = 17.4 Hz, 8H), 1.14 - 1.43 (m, 11H), 1.45 - 1.61 (m, 7H), 1.66 - 1.99 (m, 8H), 2.14 (t, J = 11.3 Hz, 1H), 2.78 (dd, J = 13.0, 4.7 Hz, 1H), 3.31 (dd, J = 12.5, 7.1 Hz, 1H), 6.11 (s, 1H), 7.33 (s, 1H), 8.54 (s, 1H); MS (APCI) *m*/*z* 577.3 (M+H)⁺.

### Example 8

### (2S,4aS,6aS,6bR,8aR,12aS,14aS,14bR)-11-cyano-14a-hydroxy-2,4a,6a,6b,9,9,12a-heptamethyl-10,14-dioxo-N-(pyridazin-3-ylmethyl)-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 3-aminomethylpyridazine (0.00207 g, 12% yield). ¹H NMR (400 MHz, DMSO-*d*₆/D₂O) δ ppm 0.93 (s, 3H), 1.08 (s, 7H), 1.15 - 1.40 (m, 11H), 1.48 (s, 3H), 1.50 - 1.59 (m, 4H), 1.63 - 2.07 (m, 8H), 2.07 - 2.23 (m, 1H), 4.58 (d, J = 15.8 Hz, 1H), 4.78 (d, J = 16.0 Hz, 1H), 6.10 (s, 1H), 7.59 - 7.68 (m, 2H), 8.51 (s, 1H), 9.04-9.14 (m, 1H); MS (APCI) *m*/*z* 599.9 (M+H)⁺.

### Example 9

### (2S,4aS,6aS,6bR,8aR,12aS,14aS,14bR)-11-cyano-14a-hydroxy-2,4a,6a,6b,9,9,12a-heptamethyl-N-[(1-methyl-1H-pyrazol-4-yl)methyl]-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 4-aminomethyl-1-methylpyrazole (0.0079 g, 45% yield). ¹H NMR (400 MHz, DMSO-*d*₆/D₂O) δ ppm 0.92 (s, 3H), 1.00 - 1.11 (m, 7H), 1.13-1.37 (m, 11H), 1.49 (s, 3H), 1.51 - 1.59 (m, 4H), 1.66 - 1.98 (m, 8H), 2.08 - 2.19 (m, 1H), 3.79 (s, 3H), 4.00 - 4.13 (m, 1H), 4.31 - 4.43 (m, 1H), 6.10 (s, 1H), 7.37 (s, 1H), 7.53 (s, 1H), 8.52 (s, 1H); MS (APCI) *m*/*z* 601.2 (M+H)⁺.

### Example 10

### (2S,4aS,6aS,6bR,8aR,12aS,14aS,14bR)-11-cyano-14a-hydroxy-2,4a,6a,6b,9,9,12a-heptamethyl-N-[2-(morpholin-4-yl)ethyl]-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 4-(2-aminoethyl)morpholine (0.0074 g, 40% yield). ¹H NMR (400 MHz, DMSO-*d₆*/D₂O) δ ppm 0.92 (s, 3H), 1.03 - 1.12 (m, 7H), 1.19 (s, 3H), 1.20 - 1.36 (m, 9H), 1.50 (s, 3H), 1.51 - 1.60 (m, 4H), 1.79 (ddd, J = 40.8, 28.6, 16.4 Hz, 8H), 2.14 (t, J = 11.4 Hz, 1H), 3.28 - 3.36 (m, 7H), 3.56 (t, J = 6.2 Hz, 2H), 3.88 (s, 4H), 6.09 (s, 1H), 8.49 (s, 1H); MS (APCI) *m*/*z* 620.3 (M+H)⁺.

### Example 11

### (2S,4aS,6aS,6bR,8aR,12aS,14aS,14bR)-11-cyano-14a-hydroxy-2,4a,6a,6b,9,9,12a-heptamethyl-N-(2-methylbenzyl)-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 2-methylbenzylamine (0.00817 g, 45% yield). ¹H NMR (400 MHz, DMSO-*d*₆/D₂O) δ ppm 0.88 - 0.97 (m, 3H), 1.02 - 1.13 (m, 7H), 1.13-1.39 (m, 11H), 1.48 (s, 3H), 1.49 - 1.58 (m, 4H), 1.66 - 2.01 (m, 8H), 2.08 - 2.21 (m, 1H), 2.32 (s, 3H), 4.16 (dd, J = 15.2, 4.5 Hz, 1H), 4.61 (dd, J = 15.3, 6.9 Hz, 1H), 6.08 (s, 1H), 7.10 - 7.22 (m, 3H), 7.23 - 7.32 (m, 1H), 7.91 (s, 1H), 8.51 (s, 1H); MS (APCI) *m*/*z* 611.2 (M+H)⁺.

### Example 12

### (2S,4aS,6aS,6bR,8aR,12aS,14aS,14bR)-11-cyano-14a-hydroxy-2,4a,6a,6b,9,9,12a-heptamethyl-N-[(1-methyl-1H-pyrazol-3-yl)methyl]-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with (1-methyl-1*H*-pyrazol-3-yl)methylamine (0.0087 g, 49% yield). ¹H NMR (400 MHz, DMSO-*d₆*/D₂O) δ ppm 0.93 (s, 3H), 1.02 - 1.11 (m, 7H), 1.12 - 1.36 (m, 11H), 1.48 (s, 3H), 1.54 (d, J = 16.0 Hz, 4H), 1.84 (ddd, J = 51.7, 29.6, 12.3 Hz, 9H), 2.14 (s, 1H), 3.78 (s, 3H), 4.16 (d, J = 15.1 Hz, 1H), 4.49 (d, J = 15.1 Hz, 1H), 6.10 (s, 1H), 6.18 (d, J = 2.2 Hz, 1H), 7.51 (d, J = 2.2 Hz, 1H), 8.52 (s, 1H); MS (APCI) *m*/*z* 601.2 (M+H)⁺.

### Example 13

### (2S,4aS,6aS,6bR,8aR,12aS,14aS,14bR)-11-cyano-14a-hydroxy-N-(2-methoxybenzyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 2-methoxybenzylamine (0.0055 g, 30% yield). ¹H NMR (400 MHz, DMSO-*d₆*/D₂O) δ ppm 0.93 (s, 3H), 1.03 - 1.11 (m, 8H), 1.11 - 1.39 (m, 12H), 1.48 (s, 3H), 1.50 - 1.59 (m, 4H), 1.67 - 2.05 (m, 8H), 2.07 - 2.21 (m, 1H), 3.83 (s, 3H), 4.20 (d, J = 15.7 Hz, 1H), 4.57 (d, J = 15.6 Hz, 1H), 6.09 (s, 1H), 6.92 (t, J = 7.4 Hz, 1H), 6.98 (d, J = 7.9 Hz, 1H), 7.23 (t, J = 7.2 Hz, 2H), 8.50 (s, 1H); MS (APCI) *m*/*z* 627.2 (M+H)⁺.

### Example 14

### (2S,4aS,6aS,6bR,8aR,12aS,14aS,14bR)-11-cyano-14a-hydroxy-N-(2-hydroxybutyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 1-amino-2-butanol (0.00824 g, 48% yield). ¹H NMR (400 MHz, DMSO-*d*₆/D₂O) δ ppm 0.85 - 0.96 (m, 6H), 1.01 - 1.10 (m, 7H), 1.12-1.45 (m, 12H), 1.45 - 1.51 (m, 4H), 1.51 - 1.61 (m, 4H), 1.82 (ddd, J = 43.3, 29.6, 15.8 Hz, 8H), 2.14 (t, J = 11.7 Hz, 1H), 3.14 - 3.19 (m, 1H), 3.57 (t, J = 5.9 Hz, 1H), 6.11 (s, 1H), 8.51 (s, 1H); MS (APCI) *m*/*z* 579.2 (M+H)⁺.

### Example 15

### (4aR,6aR,6bS,8aS,11S,12aR,12bS,14bS)-12b-hydroxy-11-(1H-imidazol-1-ylcarbonyl)-4,4,6a,6b,8a, 11,14b-heptamethyl-3,13-dioxo-3,4,4a,5,6,6a,6b,7,8,8a,9,10,11,12,12a,12b,13,14b-octadecahydropicene-2-carbonitrile

The titled compound was prepared using conditions described in Example 3E substituting oxazol-5-yl-methylamine with imidazole (0.0015 g, 9% yield). ¹H NMR (400 MHz, DMSO-*d*₆/D₂O) δ ppm 0.94 (s, 3H), 1.07 (d, J = 11.8 Hz, 8H), 1.22 (dd, J = 19.7, 12.6 Hz, 10H), 1.47 (t, J = 30.4 Hz, 8H), 1.66 - 1.99 (m, 7H), 2.03 - 2.36 (m, 2H), 6.01 (s, 1H), 7.39 (s, 2H), 8.44 - 8.54 (m, 2H); MS (APCI) *m*/*z* 558.2 (M+H)⁺.

### Example 16

### (2S,4aS,6aS,6bR,8aR,12aS,14aS,14bR)-11-cyano-14a-hydroxy-N-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 2-methoxyethylamine (0.00822 g, 49% yield). ¹H NMR (400 MHz, DMSO-*d*₆/D₂O) δ ppm 0.93 (d, J = 6.8 Hz, 3H), 1.00 - 1.11 (m, 7H), 1.11-1.36 (m, 11H), 1.48 (d, J = 6.7 Hz, 3H), 1.56 (d, J = 5.4 Hz, 4H), 1.64 - 1.96 (m, 8H), 2.13 (s, 1H), 3.23 (s, 1H), 3.30 (s, 3H), 3.37 - 3.59 (m, 3H), 6.12 (s, 1H), 8.53 (s, 1H); MS (APCI) *m*/*z* 565.2 (M+H)⁺.

### Example 17

### (2S,4aS,6aS,6bR,8aR,12aS,14aS,14bR)-11-cyano-14a-hydroxy-2,4a,6a,6b,9,9,12a-heptamethyl-N-(3-methylbutyl)-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with isopentylamine (0.00742 g, 44% yield). ¹H NMR (400 MHz, DMSO-*d*₆/D₂O) δ ppm 0.92 (t, J = 6.2 Hz, 8H), 0.99 - 1.10 (m, 7H), 1.11 - 1.34 (m, 11H), 1.42 - 1.59 (m, 8H), 1.59 - 1.95 (m, 8H), 2.14 (t, J = 11.5 Hz, 1H), 2.98 - 3.14 (m, 1H), 3.27 - 3.34 (m, 1H), 6.13 (s, 1H), 8.46 - 8.59 (m, 1H); MS (APCI) *m*/*z* 577.3 (M+H)⁺.

### Example 18

### (2S,4aS,6aS,6bR,8aR,12aS,14aS,14bR)-11-cyano-14a-hydroxy-2,4a,6a,6b,9,9,12a-heptamethyl-10,14-dioxo-N-(tetrahydro-2H-pyran-4-yl)-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 4-aminotetrahydropyran (0.01158 g, 66% yield). ¹H NMR (400 MHz, DMSO-*d*₆/D₂O) δ ppm 0.90 - 0.96 (m, 3H), 1.01 - 1.11 (m, 8H), 1.16-1.37 (m, 12H), 1.45 - 1.51 (m, 3H), 1.51 - 1.59 (m, 4H), 1.59 - 1.98 (m, 12H), 2.14 (t, J = 13.2 Hz, 1H), 3.40 (tt, J = 11.5, 2.7 Hz, 2H), 3.74 - 3.94 (m, 3H), 6.15 (s, 1H), 8.56 (s, 1H); MS (APCI) *m*/*z* 591.2 (M+H)⁺.

### Example 19

### (2S,4aS,6aS,6bR,8aR,12aS,14aS,14bR)-11-cyano-14a-hydroxy-N-(2-hydroxypropyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with amino-2-propanol (0.00582 g, 35% yield). ¹H NMR (400 MHz, DMSO-*d*₆/D₂O) δ ppm 0.94 (d, J = 10.6 Hz, 3H), 1.04 (s, 3H), 1.09 (dt, J = 17.6, 7.9 Hz, 8H), 1.13 - 1.37 (m, 12H), 1.49 (s, 3H), 1.55 (d, J = 13.9 Hz, 4H), 1.65 - 1.98 (m, 9H), 2.14 (t, J = 11.4 Hz, 1H), 3.05 - 3.24 (m, 2H), 3.81 (dd, J = 13.1, 6.4 Hz, 1H), 6.12 (s, 1H), 8.53 (s, 1H); MS (APCI) *m*/*z* 565.2 (M+H)⁺.

### Example 20

### (2S,4aS,6aS,6bR,8aR,12aS,14aS,14bR)-11-cyano-14a-hydroxy-2,4a,6a,6b,9,9,12a-heptamethyl-10,14-dioxo-N-[(1S)-1-phenylethyl]-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with (*S*)-(-)-alpha-methylbenzylamine (0.00638 g, 35% yield). ¹H NMR (400 MHz, DMSO-*d*₆/D₂O) δ ppm 0.92 - 1.01 (m, 3H), 1.01 - 1.11 (m, 7H), 1.12 - 1.40 (m, 11H), 1.45 - 1.61 (m, 9H), 1.61 - 2.05 (m, 8H), 2.15 (t, J = 11.5 Hz, 1H), 4.83 - 4.94 (m, 1H), 6.16 (s, 1H), 7.19 (t, J = 7.3 Hz, 1H), 7.29 (t, J = 7.6 Hz, 2H), 7.38 (d, J = 7.3 Hz, 2H), 7.77 (d, J = 7.2 Hz, 1H), 8.55 (s, 1H); MS (APCI) *m*/*z* 611.2 (M+H)⁺.

### Example 21

### (2S,4aS,6aS,6bR,8aR,12aS,14aS,14bR)-II-cyano-N-cyclohexyl-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with cyclohexylamine (0.00714 g, 41% yield). ¹H NMR (400 MHz, DMSO-*d*₆/D₂O) δ ppm 0.88 - 0.95 (m, 3H), 0.99 - 1.11 (m, 8H), 1.11-1.46 (m, 17H), 1.46 - 1.66 (m, 8H), 1.67 - 2.01 (m, 12H), 2.14 (t, J = 11.5 Hz, 1 H), 3.51-3.65 (m, 1H), 6.13 (s, 1H), 8.56 (s, 1H); MS (APCI) *m*/*z* 589.2 (M+H)⁺.

### Example 22

### (2S,4aS,6aS,6bR,8aR,12aS,14aS,14bR)-N-benzyl-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with benzylamine (0.01013 g, 58% yield). ¹H NMR (400 MHz, DMSO-*d*₆/D₂O) δ ppm 0.89 - 1.01 (m, 3H), 1.01 - 1.12 (m, 7H), 1.15 - 1.39 (m, 11H), 1.46 - 1.59 (m, 7H), 1.66 - 2.00 (m, 8H), 2.14 (t, J = 13.1 Hz, 1H), 4.19 (dd, J = 15.1, 4.6 Hz, 1H), 4.61 (dd, J = 15.2, 6.8 Hz, 1H), 6.10 (s, 1H), 7.24 (dd, J = 8.8, 4.5 Hz, 1H), 7.32 (d, J = 4.4 Hz, 3H), 8.08 (s, 1H), 8.52 (s, 1H); MS (APCI) *m*/*z* 597.2 (M+H)⁺.

### Example 23

### (2S,4aS,6aS,6bR,8aR,12aS,14aS,14bR)-11-cyano-N-(1,3-dimethoxypropan-2-yl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 2-amino-1,3-dimethoxypropane. The titled compound was impure following the initial chromatography and was subsequently purified by dissolving the residue in methanol (1 mL) and injecting onto a preparative HPLC using preparative LC Method 2 (0.00597 g, 42% yield). ¹H NMR (400 MHz, DMSO-*d*₆/D₂O) δ ppm 0.91 - 0.95 (m, 3H), 0.99 - 1.11 (m, 8H), 1.12 - 1.37 (m, 12H), 1.44 - 1.51 (m, 3H), 1.51 - 1.59 (m, 4H), 1.66 - 1.96 (m, 8H), 2.14 (t, J = 12.7 Hz, 1H), 3.30 (d, J = 2.8 Hz, 6H), 3.39 - 3.57 (m, 4H), 4.05 - 4.18 (m, 1H), 6.11 (s, 1H), 8.54 (s, 1H); MS (APCI) *m*/*z* 609.3 (M+H)⁺.

### Example 24

### methyl 1-{[(2S,4aS,6aS,6bR,8aR,12aS,14aS,14bR)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl]carbonyl}prolinate

The titled compound was prepared using conditions described in Example 3E substituting oxazol-5-yl-methylamine with methylpyrrolidine-2-carboxylate. The titled compound was impure following the initial chromatography and was subsequently purified by dissolving the residue in methanol (1 mL) and injecting onto a preparative HPLC using preparative LC Method 3 (0.00142 g, 10% yield). ¹H NMR (400 MHz, DMSO-*d*₆/D₂O) δ ppm 0.93 (s, 3H), 1.07 (t, J = 15.4 Hz, 10H), 1.18 (s, 8H), 1.51 (d, J = 29.7 Hz, 8H), 1.79 (d, J = 15.7 Hz, 7H), 2.13 (s, 5H), 2.34 (s, 2H), 3.62 (s, 3H), 4.04 - 4.15 (m, 1H), 4.34 - 4.46 (m, 1H), 6.04 (s, 1H), 8.50 (s, 1H); MS (APCI) *m*/*z* 619.3 (M+H)⁺.

### Example 25

### (2S,4aS,6aS,6bR,8aR,12aS,14aS,14bR)-11-cyano-14a-hydroxy-2,4a,6a,6b,9,9,12a-heptamethyl-10,14-dioxo-N-propyl-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with propylamine. The titled compound was impure following the initial chromatography and was subsequently purified by dissolving the residue in methanol (1 mL) and injecting onto a preparative HPLC using preparative LC Method 3 (0.00306 g, 24% yield). ¹H NMR (400 MHz, DMSO-*d*₆/D₂O) δ ppm 0.85 - 0.96 (m, 6H), 1.04 (t, J = 17.3 Hz, 8H), 1.14 - 1.38 (m, 11H), 1.48 (s, 3H), 1.52 - 1.64 (m, 6H), 1.64 - 2.02 (m, 8H), 2.03 - 2.24 (m, 1H), 3.05 (dd, J = 13.6, 6.8 Hz, 1H), 3.15 - 3.24 (m, 1H), 6.13 (s, 1H), 8.46 - 8.58 (m, 1H); MS (APCI) *m*/*z* 549.4 (M+H)⁺.

### Example 26

### (2S,4aS,6aS,6bR,8aR,12aS,14aS,14bR)-11-cyano-N-(2-furylmethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with furfurylamine. The titled compound was impure following the initial chromatography and was subsequently purified by dissolving the residue in methanol (1 mL) and injecting onto a preparative HPLC using preparative LC Method 3 (0.00415 g, 30% yield). ¹H NMR (400 MHz, DMSO-*d*₆/D₂O) δ ppm 0.92 (s, 3H), 0.96-1.12 (m, 8H), 1.13 - 1.37 (m, 12H), 1.44 - 1.59 (m, 7H), 1.66 - 1.98 (m, 8H), 2.07 - 2.21 (m, 1H), 4.18 (d, J = 15.6 Hz, 1H), 4.57 (d, J = 15.6 Hz, 1H), 6.11 (s, 1H), 6.27 (d, J = 2.5 Hz, 1H), 6.33 - 6.42 (m, 1H), 7.44 - 7.52 (m, 1H), 8.53 (s, 1H); MS (APCI) *m*/*z* 587.3 (M+H)⁺.

### Example 27

### (2S,4aS,6aS,6bR,8aR,12aS,14aS,14bR)-11-cyano-14a-hydroxy-N-(4-methoxyphenyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 4-methoxyaniline. The titled compound was impure following the initial chromatography and was subsequently purified by dissolving the residue in methanol (1 mL) and injecting onto a preparative HPLC using preparative LC Method 3 (0.00294 g, 20% yield). ¹H NMR (400 MHz, DMSO-*d*₆/D₂O) δ ppm 0.95 (d, J = 6.6 Hz, 3H), 1.07 - 1.35 (m, 19H), 1.49 - 1.62 (m, 7H), 1.66 - 1.98 (m, 8H), 2.09 - 2.23 (m, 1H), 3.75 (s, 3H), 6.19 (s, 1H), 6.83 - 6.94 (m, 2H), 7.61 - 7.72 (m, 2H), 8.56 (s, 1H), 9.34 (s, 1H); MS (APCI) *m*/*z* 613.3 (M+H)⁺.

### Example 28

### (2S,4aS,6aS,6bR,8aR,12aS,14aS,14bR)-11-cyano-14a-hydroxy-N-(2-methoxyethyl)-N,2,4a,6a,6b,9,9,12a-octamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with (2-methoxyethyl)methylamine. The titled compound was impure following the initial chromatography and was subsequently purified by dissolving the residue in methanol (1 mL) and injecting onto a preparative HPLC using preparative LC Method 3 (0.00398 g, 29% yield). ¹H NMR (400 MHz, DMSO-*d*₆/D₂O) δ ppm 0.93 (s, 3H), 1.02 - 1.21 (m, 16H), 1.28 (t, J = 13.3 Hz, 1H), 1.43 - 1.59 (m, 8H), 1.77 (dt, J = 27.3, 12.0 Hz, 6H), 1.99 - 2.25 (m, 2H), 2.38 (d, J = 13.6 Hz, 1H), 3.13 (s, 3H), 3.28 (s, 3H), 3.43 - 3.59 (m, 4H), 6.04 (s, 1H), 8.50 (s, 1H); MS (APCI) *m*/*z* 579.4 (M+H)⁺.

### Example 29

### (2S,4aS,6aS,6bR,8aR,12aS,14aS,14bR)-11-cyano-N-(cyanomethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with aminoacetonitrile. The titled compound was impure following the initial chromatography and was subsequently purified by dissolving the residue in methanol (1 mL) and injecting onto a preparative HPLC using preparative LC Method 3 (0.0026 g, 20% yield). ¹H NMR (400 MHz, DMSO-*d*₆/D₂O) δ ppm 0.93 (d, J = 10.7 Hz, 3H), 1.08 (d, J = 10.4 Hz, 7H), 1.14 - 1.37 (m, 11H), 1.43 - 1.60 (m, 7H), 1.63-1.99 (m, 8H), 2.13 (t, J = 11.1 Hz, 1H), 4.05 (d, J = 17.4 Hz, 1H), 4.23 (d, J = 17.3 Hz, 1H), 6.13 (s, 1H), 8.53 (s, 1H); MS (APCI) *m*/*z* 546.3 (M+H)⁺.

### Example 30

### (2S,4aS,6aS,6bR,8aR,12aS,14aS,14bR)-11-cyano-14a-hydroxy-2,4a,6a,6b,9,9,12a-heptamethyl-N-(1,2-oxazol-5-ylmethyl)-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with isoxazol-5-yl-methylamine. The titled compound was impure following the initial chromatography and was subsequently purified by dissolving the residue in methanol (1 mL) and injecting onto a preparative HPLC using preparative LC Method 3 (0.00455 g, 33% yield). ¹H NMR (400 MHz, DMSO-*d*₆/D₂O) δ ppm 0.94 (s, 3H), 1.04 - 1.13 (m, 7H), 1.13 - 1.38 (m, 11H), 1.46 - 1.61 (m, 7H), 1.65 - 2.02 (m, 8H), 2.07 - 2.22 (m, 1H), 4.38 (d, J = 16.3 Hz, 1H), 4.66 (d, J = 16.5 Hz, 1H), 6.12 (s, 1H), 6.33 (s, 1H), 8.38 (d, J = 1.8 Hz, 1H), 8.51 (d, J = 9.8 Hz, 1H); MS (APCI) *m*/*z* 588.3 (M+H)⁺.

### Example 31

### (4aR,6aR,6bS,8aS,11S,12aR,12bS,14bS)-12b-hydroxy-4,4,6a,6b,8a,11,14b-heptamethyl-3,13-dioxo-11-(pyrrolidin-1-ylcarbonyl)-3,4,4a,5,6,6a,6b,7,8,8a,9,10,11,12,12a,12b,13,14b-octadecahydropicene-2-carbonitrile

The titled compound was prepared using conditions described in Example 3E substituting oxazol-5-yl-methylamine with pyrrolidine. The titled compound was impure following the initial chromatography and was subsequently purified by dissolving the residue in methanol (1 mL) and injecting onto a preparative HPLC using preparative LC Method 3 (0.00334 g, 25% yield). ¹H NMR (400 MHz, DMSO-*d*₆/D₂O) δ ppm 0.92 (d, J = 8.5 Hz, 3H), 0.96 - 1.13 (m, 10H), 1.13 - 1.32 (m, 8H), 1.43 - 1.59 (m, 8H), 1.62 - 1.93 (m, 10H), 2.07 - 2.25 (m, 2H), 2.35 (d, J = 13.4 Hz, 1H), 3.38 - 3.49 (m, 2H), 3.67 (s, 2H), 6.04 (s, 1H), 8.50 (s, 1H); MS (APCI) *m*/*z* 561.4 (M+H)⁺.

### Example 32

### (2S,4aS,6aS,6bR,8aR,12aS,14aS,14bR)-11-cyano-14a-hydroxy-N-isobutyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with isobutylamine. The titled compound was impure following the initial chromatography and was subsequently purified by dissolving the residue in methanol (1 mL) and injecting onto a preparative HPLC using preparative LC Method 3 (0.00299 g, 23% yield). ¹H NMR (400 MHz, DMSO-*d*₆/D₂O) δ ppm 0.88 - 0.95 (m, 9H), 1.01 - 1.13 (m, 7H), 1.14 - 1.40 (m, 11H), 1.45 - 1.61 (m, 7H), 1.63 - 1.96 (m, 9H), 2.04-2.21 (m, 1H), 2.86 (dd, J = 12.9, 6.5 Hz, 1H), 3.18 (dd, J = 13.0, 7.0 Hz, 1H), 6.12 (s, 1H), 8.54 (s, 1H); MS (APCI) *m*/*z* 563.4 (M+H)⁺.

### Example 33

### (2S,4aS,6aS,6bR,8aR,12aS,14aS,14bR)-11-cyano-14a-hydroxy-2,4a,6a,6b,9,9,12a-heptamethyl-N (2-methylbutyl)-10,14-dioxo-1,2,3,4,4a,5,6,6a,6b,7,8,8a,9,10,12a,14,14a,14b-octadecahydropicene-2-carboxamide

The titled compound was prepared using conditions described in Example 3E substituting oxazol-5-yl-methylamine with 2-methylbutylamine. The titled compound was impure following the initial chromatography and was subsequently purified by dissolving the residue in methanol (1 mL) and injecting onto a preparative HPLC using preparative LC Method 3 (0.00151 g, 11% yield). ¹H NMR (400 MHz, DMSO-*d*₆/D₂O) δ ppm 0.83 - 0.96 (m, 10H), 1.01 - 1.13 (m, 7H), 1.13 - 1.47 (m, 14H), 1.47 - 1.61 (m, 7H), 1.62 - 1.96 (m, 9H), 2.76 - 3.02 (m, 1H), 6.12 (s, 1H), 8.55 (s, 1H); MS (APCI) *m*/*z* 577.4 (M+H)⁺.

### Example 34

(2*S*,4a*S*,6a*R*,6b*S*,8a*R*,12a*S*,14a*R*,14b*S*-11-cyano-*N*-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

### Example 34A

### (2S,4aS,6aR,6bS,8aR,12aS,14aR,14bS)-methyl 2,4a,6a,6b,9,9,12a-heptamethyl-10,14-dioxo-1,2,3,4,4a,5,6,6a,6b,7,8,8a,9,10,11,12,12a,14,14a,14b-icosahydropicene-2-carboxylate

The titled compound was prepared using conditions described in Example 3A, substituting (2*S*,4a*S*,6a*S*,6b*R*,8a*R*,10*S*,12aS,14aS,14b*R*)-methyl 10,14a-dihydroxy-2,4a,6a,6b,9,9,12a-heptamethyl-14-oxo-1,2,3,4,4a,5,6,6a,6b,7,8,8a,9,10,11,12,12a,14,14a,14b-icosahydropicene-2-carboxylate (Example 1G) with (2*S*,4a*S*,6a*R*,6b*S*,8a*R*,10*S*,12a*S*,14a*R*,14b*S*)-methyl 10-hydroxy-2,4a,6a,6b,9,9,12a-heptamethyl-14-oxo-1,2,3,4,4a,5,6,6a,6b,7,8,8a,9,10,11,12,12a,14,14a,14b-icosahydropicene-2-carboxylate (Example IF, 3.67073 g, 7.57 mmol) to give the titled compound (3.44 g, 7.13 mmol, 94 % yield). ¹H NMR (400 MHz, CDCl₃) δ ppm 0.94 (s, 3H), 0.99 (s, 3H), 1.07 - 1.11 (m, 4H), 1.13 (s, 3H), 1.14 (s, 3H), 1.21 - 1.35 (m, 7H), 1.43 (s, 3H), 1.48 - 1.55 (m, 3H), 1.65 - 2.05 (m, 9H), 2.18 - 2.26 (m, 2H), 2.49 (ddd, J = 15.8, 7.0, 3.8 Hz, 1H), 2.66 (ddd, J = 15.9, 10.9, 7.3 Hz, 1H), 3.03 (d, J = 4.7 Hz, 1H), 3.76 (s, 3H), 5.84 (s, 1H); MS (ESI) *m*/*z* 483.2 (M+H)⁻.

### Example 34B

### methyl (2S,4aS,6aR,6bS,8aR,13aS,15aR,15bS)-2,4a,6a,6b,9,9,13a-heptamethyl-15-oxo-1,2,3,4,4a,5,6,6a,6b,7,8,8a,9,13,13a,15,15a,15b-octadecahydropiceno[2,3-d][1,2]oxazole-2-carboxylate

The titled compound was prepared using conditions described in Example 3B, substituting the product of Example 3A with the product Example 34A. ¹H NMR (400 MHz, CDCl₃) δ ppm 0.95 (s, 3H), 1.01 (s, 3H), 1.13 (s, 3H), 1.18 (s, 3H), 1.28 (s, 3H), 1.30 - 1.35 (m, 2H), 1.36 (s, 3H), 1.44 (s, 3H), 1.47 - 1.55 (m, 3H), 1.67 - 2.07 (m, 10H), 2.21 - 2.27 (m, 1H), 2.41 (d, J = 15.1 Hz, 1H), 2.79 (d, 1H), 3.07 (d, J = 4.7 Hz, 1H), 3.77 (s, 3H), 5.93 (s, 1H), 8.08 (s, 1H); MS (ESI) *m*/*z* 508.2 (M+H)⁻.

### Example 34C

### (2S,4aS,6aR,6bS,8aR,12aS,14aR,14bS)-11-cyano-10-hydroxy-2,4a,6a,6b,9,9,12a-heptamethyl-14-oxo-1,2,3,4,4a,5,6,6a,6b,7,8,8a,9,12,12a,14,14a,14b-octadecahydropicene-2-carboxylic acid

The titled compound was prepared using conditions described in Example 3C, substituting the product of Example 3B with the product Example 34B. The reaction was incomplete, and the impure titled compound (1.1837 g) was obtained chromatographically using a Biotage® SNAP 340 g silica cartridge eluted with a step gradient of acetone/heptane (3 column volumes (CV) 0%, 5 CV 0-30%, 4 CV 30%, 2.5 CV 30-55% then 3 CV 55%). MS (ESI) *m*/*z* 494.1 (M+H)⁺.

### Example 34D

### (2S,4aS,6aR,6bS,8aR,12aS,14aR,14bS)-11-cyano-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylic acid

The product of Example 34C (1.1837 g, 2.398 mmol) was dissolved in dimethylformamide (8 mL). The solution was cooled to 0 °C and 1,3-dibromo-5,5-dimethylhydantoin (0.356 g, 1.247 mmol) was added. After stirring at 0 °C for 1 hour, the solution was sparged with nitrogen for 10 minutes, and pyridine (3 mL, 37.1 mmol) was added. The solution was sparged with nitrogen again for 10 minutes and was then warmed to room temperature and stirred at 50 °C overnight under a constant flow of nitrogen. The solution was concentrated under reduced pressure. The residue was purified chromatographically using a Biotage® SNAP 100 g silica cartridge eluted with a step gradient of acetone/heptane (3 column volumes (CV) 0%, 5 CV 0-30%, 4 CV 30%, 2.5 CV 30-55% then 3 CV 55%) to give the titled compound (0.54 g, 1.098 mmol, 45.8% yield). ¹H NMR (400 MHz, DMSO-*d*₆) δ ppm 0.89 (s, 3H), 0.93 (s, 3H), 1.04 (s, 3H), 1.07 (s, 3H), 1.18 (s, 3H), 1.22 - 1.28 (m, 4H), 1.39 - 1.52 (m, 8H), 1.61 - 2.10 (m, 10H), 2.96 (d, J = 4.6 Hz, 1H), 6.23 (s, 1H), 8.68 (s, 1H), 11.96 (s, 1H); MS (ESI) *m*/*z* 492.0 (M+H)⁺.

### Example 34E

### (2S,4aS,6aR,6bS,8aR,12aS,14aR,14bS)-11-cyano-N-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

Example 34D (0.020 g, 0.041 mmol) was dissolved in *N,N*-dimethylformamide (814 µL) and treated with *N,N*-diisopropylethylamine (14.21 µL, 0.081 mmol) and 2-methoxyethanamine (6.37 µL, 0.061 mmol). (O-(7-Azabenzotriazol-1-yl)-*N,N,N',N'-*tetramethyluronium hexafluorophosphate) (0.017 g, 0.045 mmol) was added last, and the reaction mixture was stirred at room temperature for one hour. The reaction mixture was then diluted with acetonitrile and 0.1% trifluoroacetic acid in water and purified by reversed-phase Waters HPLC using a Nova-Pak® C18 Radial-Pak 6 µm, 60 Å, 40 × 100 mm, cartridge eluted with a gradient of 10-90% acetonitrile in aqueous 0.1% trifluoroacetic acid (60 mL/minute) to give the titled compound (0.012 mg, 56% yield). ¹H NMR (400 MHz, DMSO-*d₆*) δ ppm 0.85 (s, 3H), 0.91 (s, 3H), 0.96 (s, 3H), 1.06 (s, 3H), 1.16 (s, 3H), 1.20 (d, J = 12.1 Hz, 2H), 1.23 - 1.36 (m, 2H), 1.44 (d, J = 5.0 Hz, 6H), 1.60 - 1.73 (m, 2H), 1.75-1.98 (m, 7H), 2.98 (d, J = 4.4 Hz, 1H), 3.24 (s, 3H), 6.27 (s, 1H), 7.05 (t, J = 5.4 Hz, 1H), 8.65 (s, 1H); MS (ESI) *m*/*z* 581 (M+CH₃OH+H)⁺.

### Example 35

### (2S,4aS,6aR,6bS,8aR,12aS,14aR,14bS)-11-cyano-N-(2-furylmethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The title compound was prepared using the conditions described in Example 34E substituting 2-methoxyethanamine with furan-2-ylmethanamine to give the title compound (0.013 g, 56% yield). ¹H NMR (400 MHz, DMSO-*d*₆) δ ppm 0.85 (s, 3H), 0.92 (s, 3H), 1.00 (s, 3H), 1.07 (s, 3H), 1.17 (s, 3H), 1.18 - 1.37 (m, 4H), 1.44 (d, J = 5.3 Hz, 6H), 1.49 (d, J = 11.2 Hz, 1H), 1.62 - 1.75 (m, 2H), 1.75 - 2.00 (m, 7H), 2.98 (d, J = 4.5 Hz, 1H), 4.34 (d, J = 6.1 Hz, 1H), 6.26 (s, 1H), 6.30 (d, J = 2.7 Hz, 1H), 6.38 (dd, J = 3.1, 1.9 Hz, 1H), 7.53 (d, J = 9.4 Hz, 2H), 8.65 (s, 1H); MS (ESI) *m*/*z* 603 (M+CH₃OH+H)⁺.

### Example 36

### (2S,4aS,6aR,6bS,8aR,12aS,14aR,14bS)-11-cyano-N-cyclohexyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The title compound was prepared using the conditions described in Example 34E substituting 2-methoxyethanamine with cyclohexylamine to give the title compound (0.010 g, 22% yield). ¹H NMR (400 MHz, DMSO-*d*₆) δ ppm 0.85 (s, 3H), 0.91 (s, 3H), 0.96 (s, 3H), 1.06 (s, 3H), 1.16 (s, 5H), 1.19 - 1.31 (m, 7H), 1.43 (d, J = 13.4 Hz, 6H), 1.48 - 1.99 (m, 16H), 3.00 (d, J = 4.4 Hz, 2H), 6.31 (s, 1H), 6.74 (d, J = 7.7 Hz, 1H), 8.63 (s, 1H); MS (ESI) *m*/*z* 605 (M+CH₃OH+H)⁺.

### Example 37

### (2S,4aS,6aR,6bS,8aR,12aS,14aR,14bS)-11-cyano-N-(1,3-dimethoxypropan-2-yl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide

The title compound was prepared using the conditions described in Example 34E substituting 2-methoxyethanamine with 2-amino-1,3-dimethoxypropane to give the title compound (0.005 g, 10 % yield). ¹H NMR (400 MHz, DMSO-*d*₆) δ ppm 0.87 (s, 4H), 0.92 (d, J = 9.6 Hz, 3H), 0.98 (s, 3H), 1.09 (d, J = 12.7 Hz, 4H), 1.18 (s, 3H), 1.24 (t, J = 15.4 Hz, 3H), 1.38 (t, J = 13.9 Hz, 1H), 1.46 (d, J = 2.9 Hz, 7H), 1.61 - 1.99 (m, 9H), 1.99 - 2.17 (m, 2H), 2.99 (d, J = 4.7 Hz, 1H), 3.25 (d, J = 22.2 Hz, 8H), 3.37 - 3.45 (m, 4H), 4.12 (d, J = 8.3 Hz, 1H), 6.32 (s, 1H), 6.78 (d, J = 8.4 Hz, 1H), 8.68 (s, 1H); MS (ESI) *m*/*z* 615 (M+Na)⁺.

### Example 38

### (2S,4aS,6aR,6bS,8aR,12aS,14aR,14bS)-11-cyano-2,4a,6a,6b,9,9,12a-heptamethyl-N-[(1-methyl-1H-pyrazol-4-yl)methyl]-10,14-dioxo-1,2,3,4,4a,5,6,6a,6b,7,8,8a,9,10,12a,14,14a,14b-octadecahydropicene-2-carboxamide

The title compound was prepared using the conditions described in Example 34E substituting 2-methoxyethanamine with 4-aminomethyl-1-methylpyrazole to give the title compound (0.018 g, 76% yield). ¹H NMR (400 MHz, DMSO-*d*₆) δ ppm 0.83 (d, J = 9.9 Hz, 2H), 0.93 (s, 3H), 1.01 (d, J = 13.0 Hz, 3H), 1.03 - 1.14 (m, 3H), 1.17 (d, J = 7.7 Hz, 3H), 1.30 (dd, J = 35.3, 21.2 Hz, 3H), 1.41 - 1.52 (m, 6H), 1.68 (t, J = 17.7 Hz, 1H), 1.80 - 1.98 (m, 4H), 2.99 (d, J = 4.5 Hz, 2H), 3.76 (d, J = 14.0 Hz, 6H), 4.17 (d, J = 5.4 Hz, 4H), 6.29 (s, 1H), 7.28 - 7.41 (m, 2H), 7.59 (s, 1H), 8.67 (s, 1H); MS (ESI) *m*/*z* 617 (M+CH₃OH+H)⁺.

### Example 39

### (2S,4aS,6aR,6bS,8aR,12aS,14aR,14bS)-11-cyano-2,4a,6a,6b,9,9,12a-heptamethyl-N-(1,3-oxazol-5-ylmethyl)-10,14-dioxo-1,2,3,4,4a,5,6,6a,6b,7,8,8a,9,10,12a,14,14a,14b-octadecahydropicene-2-carboxamide

The title compound was prepared using the conditions described in Example 34E substituting 2-methoxyethanamine with oxazol-5-ylmethanamine to give the title compound (0.017 g, 73% yield). ¹H NMR (400 MHz, DMSO-*d*₆) δ ppm 0.85 (s, 3H), 0.93 (s, 3H), 1.02 (s, 3H), 1.08 (s, 3H), 1.18 (s, 3H), 1.22 - 1.42 (m, 4H), 1.46 (t, J = 7.5 Hz, 7H), 1.52 - 2.02 (m, 10H), 2.98 (d, J = 4.3 Hz, 1H), 4.35 (dd, J = 16.1, 4.9 Hz, 1H), 4.47 (dd, J = 15.7, 5.9 Hz, 1H), 6.28 (s, 1H), 7.09 (s, 1H), 7.69 (t, J = 5.6 Hz, 1H), 8.27 (s, 1H), 8.67 (s, 1H); MS (ESI) *m*/*z* 604 (M+CH₃OH+H)⁺.

The following compounds can be prepared using the methods described in the Schemes or in the above examples:
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(prop-2-yn-1-yl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(methylsulfonyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(ethylsulfonyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-(3-(1*H*-imidazol-1-yl)propyl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
methyl 4-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamido)piperidine-1-carboxylate;
(2*S*,4a*S*,6a*S*,6b*R*,8a*R*,12a*S*,14a*S*,14b*R*)-*N*-(1-benzylpiperidin-4-yl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
methyl 2-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamido)acetate;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-N (2-(methylamino)-2-oxoethyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(isoxazol-3-ylmethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(oxazol-2-ylmethyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-((1*H*-pyrazol-3-yl)methyl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-((1*H*-pyrazol-4-yl)methyl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-((1*H*-pyrazol-5-yl)methyl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-((1-methyl-1*H*-pyrazol-5-yl)methyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyrimidin-4-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyrimidin-2-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyrimidin-5-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*,2,4a,6a,6b,9,9,12a-octamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-ethyl-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-isopropyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(2-methylpentyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(2,2-dimethylpentyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-hydroxypentyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(2,4-dihydroxybutyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(4-hydroxy-2-methylbutyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(4a*R*,6a*R*,6b*S*,8a*S*,11*S*,12a*R*,12b*S*,14b*S*)-12b-hydroxy-11-(2-(hydroxymethyl)pyrrolidine-1-carbonyl)-4,4,6a,6b,8a,11,14b-heptamethyl-3,13-dioxo-3,4,4a,5,6,6a,6b,7,8,8a,9,10,11,12,12a, 12b, 13,14b-octadecahydropicene-2-carbonitrile;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-phenyl-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-phenethyl-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-(4-bromophenyl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(4-cyanophenyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(2-phenoxybenzyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-(2-(4-chlorophenoxy)benzyl)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(2-(3,4-dichlorophenoxy)benzyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(2-(3,4,5-trichlorophenoxy)benzyl)-1,2,3,4,4a,5,6,6a,6b,7,8,8a,9,10,12a,14,14a,14b-octadecahydropicene-2-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)propionamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)but-3-ynamide;
2-cyano-*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)acetamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-fluoroacetamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-3,3,3-trifluoropropanamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-3-hydroxypropanamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-hydroxyacetamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-morpholinoacetamide;
methyl 4-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)piperazine-1-carboxylate;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-(4-hydroxypiperidin-1-yl)acetamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-(methylsulfonamido)acetamide;
methyl (2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)carbamate;
methyl (1-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-1-oxopropan-2-yl)carbamate;
methyl (2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxo-1-phenylethyl)carbamate;
*N*-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)-1*H*-pyrazole-3-carboxamide;
*N*-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)-1-methyl-1*H*-pyrazole-3-carboxamide;
*N*-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)-1*H*-pyrazole-5-carboxamide;
N-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)-1-methyl-1*H*-pyrazole-5-carboxamide;
*N*-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)-1*H*-pyrazole-4-carboxamide;
*N*-(2-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)amino)-2-oxoethyl)-1-methyl-1*H*-pyrazole-4-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1*H*-pyrazole-3-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-methyl-1H-pyrazole-3-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1*H*-pyrazole-4-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-methyl-1*H*-pyrazole-4-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1*H*-pyrazole-5-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-methyl-1*H*-pyrazole-5-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyrimidine-5-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyrimidine-4-carboxamide;
methyl ((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)carbamate;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)isoxazole-3-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)isoxazole-4-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)isoxazole-5-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyridazine-4-carboxamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyridazine-3-carboxamide;
phenyl ((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)carbamate;
3-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-(2-methoxyethyl)-1-methylurea;
1-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-3-(2-methoxyethyl)urea;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)morpholine-4-carboxamide;
methyl 4-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)carbamoyl)piperazine-1-carboxylate;
methyl 1-(((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)carbamoyl)pyrrolidine-2-carboxylate;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)ethanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)propane-1-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)propane-2-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)sulfuric diamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-*N*'-methylsulfuric diamide;
(2*S*,4a*S*,6a*S*,6b*R*,8a*R*,12a*S*,14a*S*,14b*R*)-11-cyano-2-((cyanosulfonyl)amino)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene;
methyl ((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)sulfamate;
1-cyano-*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-fluoromethanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)prop-2-yne-1-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-hydroxyethanesulfonamide;
2-cyano-*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)ethanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-methoxyethanesulfonamide;
*N*-(*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)sulfamoyl)acetamide;
methyl 2-(*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)sulfamoyl)acetate;
methyl *N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)sulfamoylcarbamate;
methyl 2-((*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)sulfamoyl)amino)acetate;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-*N*'-(2-methoxyethyl)sulfuric diamide;
*N'*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-*N*-(cyanomethyl)-*N*-methylsulfuric diamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)tetrahydro-2*H*-pyran-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-(tetrahydrofuran-3-yl)methanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)morpholine-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-4-methylpiperazine-1-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-2-oxooxazolidine-3-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-(pyridin-3-yl)methanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-(pyridin-4-yl)methanesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1*H*-pyrazole-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)isoxazole-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1*H*-imidazole-5-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1*H*-imidazole-2-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)thiazole-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)thiazole-2-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-3-hydroxybenzenesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-4-hydroxybenzenesulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyridine-2-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyridine-3-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyridine-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyrimidine-2-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyrimidine-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)pyridazine-4-sulfonamide;
*N*-((2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)-1-(methylsulfonyl)methanesulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)methanesulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)ethanesulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)propane-1-sulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)propane-2-sulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)sulfuric diamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*'-methylsulfuric diamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)propionamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)but-3-ynamide;
2-cyano-*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)acetamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-2-fluoroacetamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3,3,3-trifluoropropanamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3-hydroxypropanamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-2-hydroxyacetamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-2-morpholinoacetamide;
methyl 4-(2-((((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)amino)-2-oxoethyl)piperazine-1-carboxylate;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-2-(4-methylpiperidin-1-yl)acetamide;
*N*-(((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)-2-(methylsulfonamido)acetamide;
methyl (2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)carbamate;
methyl (1-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-1-oxopropan-2-yl)carbamate;
methyl (2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxo-1-phenylethyl)carbamate;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1-methyl-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1*H*-pyrazole-5-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1-methyl-1*H*-pyrazole-5-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1*H*-pyrazole-4-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1-methyl-1*H*-pyrazole-4-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1-methyl-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1*H*-pyrazole-4-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)amino)-2-oxoethyl)-1-methyl-1*H*-pyrazole-4-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-1*H*-pyrazole-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-1-methyl-1*H*-pyrazole-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)pyrimidine-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)pyrimidine-4-carboxamide;
methyl (((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)carbamate;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)isoxazole-3-carboxamide;
*N*-(((2S,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)isoxazole-4-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)isoxazole-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)pyridazine-4-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)pyridazine-3-carboxamide;
phenyl (((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)carbamate;
3-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-1-(2-methoxyethyl)-1-methylurea;
1-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3-(2-methoxyethyl)urea;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)morpholine-4-carboxamide;
methyl 4-((((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)carbamoyl)piperazine-1-carboxylate;
methyl 1-((((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)carbamoyl)pyrrolidine-2-carboxylate;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylmethanesulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylethanesulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpropane-1-sulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpropane-2-sulfonamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylsulfuric diamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*,*N*'-dimethylsulfuric diamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpropionamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylbut-3-ynamide;
2-cyano-*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylacetamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-2-fluoro-*N*-methylacetamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3,3,3-trifluoro-*N*-methylpropanamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3-hydroxy-*N*-methylpropanamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-2-hydroxy-*N*-methylacetamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methyl-2-morpholinoacetamide;
methyl 4-(2-((((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)piperazine-1-carboxylate;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methyl-2-(4-methylpiperidin-1-yl)acetamide;
*N*-(((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)-*N*-methyl-2-(methylsulfonamido)acetamide;
methyl (2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)carbamate;
methyl (1-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-1-oxopropan-2-yl)carbamate;
methyl (2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxo-1-phenylethyl)carbamate;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1-methyl-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1*H*-pyrazole-5-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1-methyl-1*H*-pyrazole-5-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1*H*-pyrazole-4-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1-methyl-1*H*-pyrazole-4-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1-methyl-1*H*-pyrazole-3-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1*H*-pyrazole-4-carboxamide;
*N*-(2-((((2*S*,4a*R*,6a*S*,6b*R*,8a*S*,12a*R*,14a*S*,14b*R*)-11-cyano-14a-hydroxy-2,4a,6a,6b,12a-pentamethyl-10,14-dioxo-1,2,3,4,4a,5,6,6a,6b,7,8,8a,9,10,12a,14,14a,14b-octadecahydropicen-2-yl)methyl)(methyl)amino)-2-oxoethyl)-1-methyl-1*H*-pyrazole-4-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methyl-1*H*-pyrazole-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*,1-dimethyl-1*H*-pyrazole-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpyrimidine-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpyrimidine-4-carboxamide;
methyl (((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)(methyl)carbamate;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylisoxazole-3-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylisoxazole-4-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylisoxazole-5-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpyridazine-4-carboxamide;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylpyridazine-3-carboxamide;
phenyl (((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)(methyl)carbamate;
1-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3-(2-methoxyethyl)-1,3-dimethylurea;
1-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-3-(2-methoxyethyl)-1-methylurea;
*N*-(((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)-*N*-methylmorpholine-4-carboxamide;
methyl 4-((((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)(methyl)carbamoyl)piperazine-1-carboxylate; and
methyl 1-((((2*S*,4a*R*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl)methyl)(methyl)carbamoyl)pyrrolidine-2-carboxylate.

### REFERENCES

The following references are cited in connection with the present case.
WO 2011/130302
Abraham and Kappas, Free Radical Biol. Med., 39:1-25, 2005.
Ahmad et al., Cancer Res., 68:2920-2926, 2008.
Ahmad et a/., J. Biol. Chem., 281:35764-9, 2006.
Araujo et al., J. Immunol., 171(3):1572-1580, 2003.
Bach, Hum. Immunol., 67(6):430-432, 2006.
Cai et al., Nat. Med., 11(2):183-90, 2005.
Chauhan and Chauhan, Pathophysiology, 13(3):171-181 2006.
Dickerson et al., Prog Neuropsychopharmacol Biol. Psychiatry, March 6, 2007.
Dinkova-Kostova et al., Proc. Natl. Acad. Sci. USA, 102(12):4584-4589, 2005.
Dudhgaonkar et al., Eur. J. Pain, 10(7):573-9, 2006.
Forstermann, Biol. Chem., 387:1521, 2006.
Hanson et al., BMC Medical Genetics, 6(7), 2005.
Honda et al. Bioorg. Med. Chem. Lett., 12:1027-1030, 2002.
Honda et al., J. Med. Chem., 43:4233-4246, 2000a.
Honda, et al., J. Med. Chem., 43:1866-1877, 2000b.
Honda et al., Bioorg. Med. Chem. Lett., 7:1623-1628, 1997.
Honda et al., Bioorg. Med. Chem. Lett., 9(24):3429-3434, 1999.
Honda et al., Bioorg. Med. Chem. Lett., 8(19):2711-2714, 1998.
Honda et al., Bioorg. Med. Chem. Lett., 16(24):6306-6309, 2006.
Hotamisligil, Nature 444:860-867, 2006.
Hotamisligil, Cell 140:900-917, 2010.
Ishikawa et al., Circulation, 104(15):1831-1836, 2001.
Kawakami et al., Brain Dev., 28(4):243-246, 2006.
Kendall-Tackett, Trauma Violence Abuse, 8(2):117-126, 2007.
Kruger et al., J. Pharmacol. Exp. Ther., 319(3):1144-1152, 2006.
Lee et al., Glia., 55(7):712-22, 2007.
Lencz et al., Mol. Psychiatry, 12(6):572-80, 2007.
Liby et al., Cancer Res., 65(11):4789-4798, 2005.
Liby et al., Nat. Rev. Cancel, 7(5):357-356, 2007a.
Liby et al., Mol. Cancer Ther., 6(7):2113-9, 2007b.
Liu et al., FASEB J., 20(2):207-216, 2006.
Lu et al., J. Clin. Invest., 121(10):4015-29, 2011.
McIver et al., Pain, 120(1-2):161-9, 2005.
Morris et al., J. Mol. Med., 80(2):96-104, 2002.
Morse and Choi, Am. J. Respir. Crit. Care Med., 172(6):660-670, 2005.
Morse and Choi, Am. J. Respir. Crit. Care Med., 27(1):8-16, 2002.
Pall, Med. Hypoth., 69:821-825, 2007.
Place et al., Clin. Cancer Res., 9(7):2798-806, 2003.
Rajakariar et al., Proc. Natl. Acad. Sci. USA, 104(52):20979-84, 2007.
Ross et al., Am. J. Clin. Pathol., 120(Suppl):S53-71, 2003.
Ross et al., Expert Rev. Mol. Diagn., 3(5):573-585, 2003.
Ruster et a/., Scand. J. Rheumatol., 34(6):460-3, 2005.
Sacerdoti et al., Curr Neurovasc Res. 2(2):103-111, 2005.
Salvemini et al., J. Clin. Invest., 93(5):1940-1947, 1994.
Sarchielli et al., Cephalalgia, 26(9):1071-1079 , 2006.
Satoh et al., Proc. Natl. Acad. Sci. USA, 103(3):768-773, 2006.
Schulz et al., Antioxid. Redox. Sig., 10:115, 2008.
Shin et al., Eur. J. Pharmacol., 620(1-3):138-44, 2009.
Suh et al., Cancer Res., 58:717-723, 1998.
Suh et al., Cancer Res., 59(2):336-341, 1999.
Szabo et al., Nature Rev. Drug Disc., 6:662-680, 2007.
Takahashi et al., Cancer Res., 57:1233-1237, 1997.
Tamir and Tannebaum, Biochim. Biophys. Acta, 1288:F31-F36, 1996.
Zhou et al., Am. J. Pathol., 166(1):27-37, 2005.

## Claims

1. A compound of Formula (I), 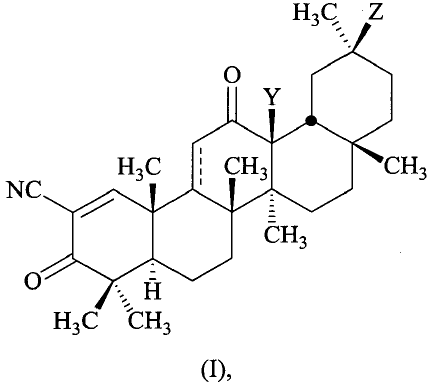 or a pharmaceutically acceptable salt thereof, wherein
----- is a single or double bond;
Y is -OR¹; and
Z is -CO₂H, -CO₂R², -C(O)NR³R⁴, -C(O)G¹, -NR³C(O)R⁵, -NR³S(O)₂R⁵, -CH₂NR³C(O)R⁵, -CH₂NR³S(O)₂R⁵, -NR³R⁶, or -CH₂NR³R⁶;
or
Y is hydrogen; and
Z is -C(O)NR³R¹⁴, -C(O)G¹, -NR³C(O)R⁵, -NR³S(O)₂R⁵, -CH₂NR³C(O)R⁵, -CH₂NR³S(O)₂R⁵, -NR³R⁶, or -CH₂NR³R⁶;
R¹ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, -G^{2b}, or C(O)R^{1a};
R² is C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R³ is hydrogen, C₁-C₆-alkyl, C₁-C₆-haloalkyl, -CH₂CR^{2a}=CR^{2b}R^{2c}, -CH₂G^{2a}, -G^{2a}, -CH₂G^{2b}, or -G^{2b};
R⁴ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘC(O)OR², -(CR^{4a}R^{4b})ₘ-CO₂H, -(CR^{4a}R^{4b})ₘC(O)N(R³)₂, -(CR^{4a}R^{4b})ₘ-C(O)N(OH)R³, or -SO₂R^{1a};
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a},-NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R⁶ is hydrogen, C₁-C₈-alkyl, C₁-C₈-alkenyl, C₁-C₈-alkynyl, C₁-C₈-haloalkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², or -(CR^{4a}R^{4b})ₘ-C(O)N(R³)₂;
R¹⁴ is C₁-C₈-alkenyl, C₁-C₈-alkynyl, C₁-C₈-haloalkyl, C₁-C₆-alkoxy-C₁-C₆-alkyl, C1-C₆-alkoxy-C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -G^{4b},-(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -SO₂R^{1a}, or -(CR^{4a}R^{4b})ₘ-SO₂R^{1a};
R^{1a} is, at each occurrence, independently C₁-C₆-alkyl, C₁-C₆-haloalkyl, C₃-C₈-cycloalkyl, or C₃-C₈-cycloalkyl-C₁-C₃-alkyl;
R^{1b} is, at each occurrence, independently hydrogen, C₁-C₆-alkyl or C₁-C₆-haloalkyl;
R^{2a}, R^{2b}, and R^{2c} are independently hydrogen, C₁-C₆-alkyl or C₁-C₆-haloalkyl;
R^{4a} and R^{4b} are, at each occurrence, independently hydrogen, C₁-C₆-alkyl, or C₁-C₆-haloalkyl;
R^{4c} is, at each occurrence, independently hydrogen, C₁-C₄-alkyl, C₂-C₄-alkenyl, C₂-C₄-alkynyl or C₁-C₄-haloalkyl;
R^{4d} is, at each occurrence, independently C₁-C₄-alkyl, C₂-C₄-alkenyl, C₂-C₄-alkynyl or C₁-C₄-haloalkyl;
R^{5a} and R^{5b} are, at each occurrence, independently hydrogen, C₁-C₆-alkyl, or C₁-C₆-haloalkyl;
R^{5c} is, at each occurrence, independently hydrogen, C₁-C₄-alkyl, C₂-C₄-alkenyl, C₂-C₄-alkynyl or C₁-C₄-haloalkyl;
R^{5d} is, at each occurrence, independently C₁-C₄-alkyl, C₂-C₄-alkenyl, C₂-C₄-alkynyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, cyano, hydroxy, oxo, C₁-C₆-alkoxy, C₁-C₈-alkyl, C₁-C₈-alkenyl, C₁-C₈-alkynyl, C₁-C₈-haloalkyl, C₁-C₆-alkoxy-C₁-C₆-alkyl, C₁-C₆-cyanoalkyl, C₁-C₆-hydroxyalkyl, -CO₂R^{1a}, or -C(O)N(R³)₂;
G^{2a} is C₃-C₈-cycloalkyl;
G^{2b} is aryl or heteroaryl, wherein the aryl is unsubstituted or substituted with 1, 2, 3, 4 or 5 C₁-C₆-alkyl, C₁-C₆-alkoxy, cyano or halogen;
G^{4a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, or -(CR^{4a}R^{4b})ₘ-G^{a};
G^{4b} is aryl or heteroaryl, wherein the aryl and heteroaryl are unsubstituted or substituted with C₁-C₄-alkyl, C₂-C₄-alkenyl, C₂-C₄-alkynyl, halogen, cyano, -NO₂, -OR^{4c}, -alkyl-OR^{4c}, -OC(O)R^{4d}, -OC(O)N(R^{4c})₂, -SR^{4c}_{,} -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, -G^{a}, -(CR^{4a}R^{ab})ₘ-G^{a}, or -O-G^{a};
G^{5a} is cycloalkyl, cycloalkenyl or heterocycle, wherein the cycloalkyl, cycloalkenyl, and heterocycle are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, or -(CR^{5a}R^{5b})ₙ-G^{a};
G^{5b} is aryl or heteroaryl, wherein the aryl and heteroaryl are unsubstituted or substituted with C₁-C₄-alkyl, C₂-C₄-alkenyl, C₂-C₄-alkynyl, halogen, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-cyanoalkyl, C₁-C₄-haloalkyl, -G^{a}, -(CR^{5a}R^{5b})ₙ-G^{a}, or -O-G^{a};
G^{5c} is aryl or heteroaryl, wherein the aryl or heteroaryl is unsubstituted or substituted with 1, 2, 3, 4 or 5 C₁-C₆-alkyl, C₁-C₆-alkoxy, cyano or halogen;
G^{a} is aryl or heteroaryl, wherein the aryl or heteroaryl is unsubstituted or substituted with 1, 2, 3, 4 or 5 C₁-C₆-alkyl, cyano or halogen;
m, at each occurrence, independently is 1, 2, 3, 4, or 5; and
n, at each occurrence, independently is 1, 2, 3, 4, or 5.

2. The compound of claim 1, or a salt thereof, wherein Y is -OR¹.

3. The compound of claim 1, or a salt thereof, wherein Y is OH.

4. The compound of claim 3, or a salt thereof, wherein Z is -CO₂H or -CO₂R²,

5. The compound of claim 4, wherein R² is C₁-C₆-alkyl.

6. The compound of claim 3, or a salt thereof, wherein Z is -C(O)NR³R⁴ or -C(O)G¹.

7. The compound of claim 6 wherein,
R² is C₁-C₆-alkyl;
R³ is hydrogen or C₁-C₆-alkyl;
R⁴ is hydrogen, C₁-C₈-alkyl, C₁-C₈-alkynyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, or -SO₂R^{1a};
R^{1a} is, at each occurrence, independently C₁-C₆-alkyl;
R^{4a} and R^{4b} are, at each occurrence, independently hydrogen or C₁-C₆-alkyl; R^{4c} is, at each occurrence, independently hydrogen or C₁-C₄-alkyl;
R^{4d} is, at each occurrence, independently C₁-C₄-alkyl or C₁-C₄-haloalkyl;
G¹ is a nitrogen containing heterocycle or heteroaryl, wherein the heterocycle or heteroaryl is attached to the carbonyl of Z at a nitrogen atom and is unsubstituted or substituted with one or more halogen, hydroxy, C₁-C₆-alkoxy, C₁-C₈-alkyl, C₁-C₈-haloalkyl, C₁-C₆-hydroxyalkyl, -CO₂R^{1a}, or -C(O)N(R³)₂;
G^{4a} is cycloalkyl or heterocycle, wherein the cycloalkyl and heterocycle are unsubstituted or substituted with C₁-C₄-alkyl, halogen, cyano, oxo, -OR^{4c}, -C(O)R^{4d}, -C(O)OR^{4c}, -C(O)N(R^{4c})₂, -N(R^{4c})C(O)R^{4d}, -N(R^{4c})C(O)O(R^{4d}), -N(R^{4c})S(O)₂(R^{4d}), C₁-C₄-haloalkyl, C₁-C₄-hydroxyalkyl or -(CR^{4a}R^{4b})ₘ-G^{a};
G^{4b} is aryl or heteroaryl, wherein the aryl and heteroaryl are unsubstituted or substituted with C₁-C₄-alkyl, halogen, cyano, -OR^{4c}, C₁-C₄-haloalkyl or -O-G^{a};
G^{a} is aryl, wherein the aryl is unsubstituted or substituted with 1, 2, 3, 4 or 5 C₁-C₆-alkyl, cyano or halogen; and
m, at each occurrence, independently is 1, 2, 3 or 4.

8. The compound of claim 6 wherein,
R³ is hydrogen or C₁-C₆-alkyl;
R⁴ is C₁-C₈-alkyl, C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, -G^{4a}, -(CR^{4a}R^{4b})ₘ-G^{4a}, -G^{4b}, or -(CR^{4a}R^{4b})ₘ-G^{4b};
R^{1a} is, at each occurrence, independently C₁-C₆-alkyl;
R^{4a} and R^{4b} are, at each occurrence, independently hydrogen or C₁-C₆-alkyl; R^{4c} is, at each occurrence, independently C₁-C₄-alkyl;
G¹ is a nitrogen containing heterocycle or heteroaryl, wherein the heterocycle or heteroaryl is attached to the carbonyl of Z at a nitrogen atom and is unsubstituted or substituted with one or more -CO₂R^{1a};
G^{4a} is cycloalkyl or heterocycle;
G^{4b} is aryl or heteroaryl, wherein the aryl and heteroaryl are unsubstituted or substituted with C₁-C₄-alkyl, or -OR^{4c}; and
m, at each occurrence, independently is 1 or 2.

9. The compound of claim 3, or a salt thereof, wherein Z is -NR³C(O)R⁵, -NR³S(O)₂R⁵, -CH₂NR³C(O)R⁵, or -CH₂NR³S(O)₂R⁵.

10. The compound of claim 9, wherein
R² is C₁-C₆-alkyl;
R³ is hydrogen or C₁-C₆-alkyl;
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³,-(CR^{5a}R**^{5b}**)ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a},-NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R^{1a} is, at each occurrence, independently C₁-C₆-alkyl;
R^{1b} is, at each occurrence, independently hydrogen or C₁-C₆-alkyl;
R^{5a} and R^{5b} are, at each occurrence, independently hydrogen or C₁-C₆-alkyl;
R^{5c} is, at each occurrence, independently hydrogen or C₁-C₄-alkyl;
G^{5a} is cycloalkyl or heterocycle, wherein the cycloalkyl and heterocycle are unsubstituted or substituted with C₁-C₄-alkyl, oxo, -OR^{5c} or -C(O)OR^{5c};
G^{5b} is aryl or heteroaryl, wherein the aryl and heteroaryl are unsubstituted or substituted with C₁-C₄-alkyl, halogen, -OR^{5c}, -C(O)N(R^{5c})₂ or C₁-C₄-haloalkyl;
G^{5c} is aryl, wherein the aryl is unsubstituted or substituted with 1, 2, 3, 4 or 5 C₁-C₆-alkyl, C₁-C₆-alkoxy, cyano or halogen; and
n, at each occurrence, independently is 1, 2, 3 or 4.

11. The compound of claim 3, or a salt thereof, wherein Z is -NR³R⁶ or -CH₂NR³R⁶.

12. The compound of claim 1, or a salt thereof, wherein Y is H.

13. The compound of claim 12, or a salt thereof, wherein Z is -C(O)NR³R¹⁴ or -C(O)G¹.

14. The compound of claim 13, wherein,
R² is C₁-C₆-alkyl;
R³ is hydrogen or C₁-C₆-alkyl;
R¹⁴ is C₁-C₈-alkenyl, C₁-C₈-alkynyl, C₁-C₈-haloalkyl, C₁-C₆-alkoxy-C₁-C₆-alkyl, C₁-C₆-alkoxy-C₁-C₆-alkoxy-C₁-C₆-alkyl, di(C₁-C₆-alkoxy)-C₁-C₆-alkyl, C₁-C₆-cyanoalkyl, C₁-C₆-hydroxyalkyl, C₁-C₆-di(hydroxy)alkyl, -G^{4a}, -(CR^{4a}R^{4b})ₘ-G^{4a}, -G^{4b},-(CR^{4a}R^{4b})ₘ-G^{4b}, -(CR⁴aR^{4b})ₘ-C(O)OR², -(CR^{4a}R^{4b})ₘ-CO₂H, -(CR^{4a}R^{4b})ₘ-C(O)N(R³)₂, -SO₂R^{1a}, or -(CR^{4a}R^{4b})ₘ-SO₂R^{1a};
R^{1a} is, at each occurrence, independently C₁-C₆-alkyl, C₁-C₆-haloalkyl, C₃-C₈-cycloalkyl, or C₃-C₈-cycloalkyl-C₁-C₃-alkyl;
R^{4a} and R^{4b} are, at each occurrence, independently hydrogen or C₁-C₆-alkyl; R^{4c} is, at each occurrence, independently hydrogen or C₁-C₄-alkyl;
R^{4d} is, at each occurrence, independently C₁-C₄-alkyl or C₁-C₄-haloalkyl;
G^{4a} is cycloalkyl or heterocycle, wherein the cycloalkyl and heterocycle are unsubstituted or substituted with C₁-C₄-alkyl, halogen, cyano, oxo, -OR^{4c}, -C(O)R^{4d}, -C(O)OR^{4C}, -C(O)N(R^{4c})₂, -N(R^{4c})C(O)R^{4d}, -N(R^{4c})C(O)O(R^{4d}), -N(R^{4c})S(O)₂(R^{4d}), C₁-C₄-haloalkyl, C₁-C₄-hydroxyalkyl or -(CR^{4a}R^{4b})ₘ-G^{a};
G^{ab} is aryl or heteroaryl, wherein the aryl and heteroaryl are unsubstituted or substituted with C₁-C₄-alkyl, halogen, cyano, -O-G^{a} or -OR^{4c};
G^{a} is aryl, wherein the aryl is unsubstituted or substituted with 1, 2, 3, 4 or 5 C₁-C₆-alkyl, cyano or halogen; and
m, at each occurrence, independently is 1, 2, 3 or 4.

15. The compound of claim 12, or a salt thereof, wherein Z is -NR³C(O)R⁵, -NR³S(O)₂R⁵, -CH₂NR³C(O)R⁵, or -CH₂NR³S(O)₂R⁵.

16. The compound of claim 15, wherein,
R² is C₁-C₆-alkyl;
R³ is hydrogen or C₁-C₆-alkyl;
R⁵ is C₁-C₈-alkyl, C₁-C₈-alkenyl, C₁-C₈-alkynyl, C₁-C₈-haloalkyl, C₁-C₆-alkoxy, C₁-C₆-alkoxy-C₁-C₆-alkyl, C₁-C₆-alkoxy-C₁-C₆-alkoxy-C₁-C₆-alkyl, cyano, C₁-C₆-cyanoalkyl, C₁-C₆-hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a},-NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, or -(CR^{5a}R^{5b})ₙ-NHC(O)G^{5b};
R^{1a} is, at each occurrence, independently C₁-C₆-alkyl;
R^{1b} is, at each occurrence, independently hydrogen or C₁-C₆-alkyl;
R^{5a} and R^{5b} are, at each occurrence, independently hydrogen or C₁-C₆-alkyl; R^{5c} is, at each occurrence, independently hydrogen, C₁-C₄-alkyl or C₁-C₄-haloalkyl;
G^{5a} is cycloalkyl or heterocycle, wherein the cycloalkyl and heterocycle are unsubstituted or substituted with C₁-C₄-alkyl, oxo, -OR^{5c} or -C(O)OR^{5c};
G^{5b} is aryl or heteroaryl, wherein the aryl and heteroaryl are unsubstituted or substituted with C₁-C₄-alkyl, halogen, -OR^{5c}, -C(O)N(R^{5c})₂ or C₁-C₄-haloalkyl;
G^{5c} is aryl, wherein the aryl is unsubstituted or substituted with 1, 2, 3, 4 or 5 C₁-C₆-alkyl, C₁-C₆-alkoxy, cyano or halogen; and
n, at each occurrence, independently is 1, 2, 3 or 4.

17. The compound of claim 12, or a salt thereof, wherein Z is -NR³R⁶ or -CH₂NR³R⁶.

18. The compound of claim 1, or a salt thereof, wherein the compound is selected from the group consisting of:
(2S,4aS,6aS,6bR,8aR,12a*S*,14a*S*,14b*R*)-methyl 11-cyano-14a-hydroxy-2,4a,6a,6b,9,9,12a-heptamethyl-10,14-dioxo-1,2,3,4,4a,5,6,6a,6b,7,8,8a,9,10,12a, 14,14a, 14b-octadecahydropicene-2-carboxylate;
(*2*S,4a*S*,6a*S*,6b*R*,8a*R*,12a*S*,14a*S*,14b*R*)-11-cyano-14a-hydroxy-2,4a,6a,6b,9,9,12a-heptamethyl-*N*-(1,3-oxazol-5-ylmethyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxylic acid;
(2*S*,4a*S*,6a*S*,6b*R*,8a*R*,12a*S*,14a*S*,14b*R*)-11-cyano-14a-hydroxy-*N-*(2-hydroxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyridazin-4-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*,2,4a,6a,6b,9,9,12a-octamethyl-10,14-dioxo-*N*-propyl-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N-*(2,2-dimethylpropyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(pyridazin-3-ylmethyl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-[(1-methyl-1*H*-pyrazol-4-yl)methyl]-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-[2-(morpholin-4-yl)ethyl]-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(2-methylbenzyl)-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-[(1-methyl-1*H*-pyrazol-3-yl)methyl]-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-methoxybenzyl)-2,4a,6a,6b,9,9,12a-heptamethyl-10,14-dioxo-1,2,3,4,4a,5,6,6a,6b,7,8,8a,9,10,12a, 14,14a, 14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-hydroxybutyl)-2,4a,6a,6b,9,9,12a-heptamethyl-10,14-dioxo-1,2,3,4,4a,5,6,6a,6b,7,8,8a,9,10,12a, 14,14a, 14b-octadecahydropicene-2-carboxamide;
(4a*R*,6a*R*,6bS*,*8a*S*,11*S*,12a*R*,12b*S*,14b*S*)-12b-hydroxy-11-(1*H*-imidazol-1-ylcarbonyl)-4,4,6a,6b,8a,11,14b-heptamethyl-3,13-dioxo-3,4,4a,5,6,6a,6b,7,8,8a,9,10,11,12,12a, 12b, 13,14b-octadecahydropicene-2-carbonitrile;
(2*S*,4a*S*,6a*S*,6b*R*,8a*R*,12a*S*,14a*S*,14b*R*)-11-cyano-14a-hydroxy-*N*-(2-methoxyethyl)-2,4a,6a,6b,9,9,12a-heptamethyl-10,14-dioxo-1,2,3,4,4a,5,6,6a,6b,7,8,8a,9,10,12a, 14,14a, 14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(3-methylbutyl)-10,14-dioxo-1,2,3,4,4a,5,6,6a,6b,7,8,8a,9,10,12a, 14,14a, 14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-(tetrahydro-2*H*-pyran-4-yl)-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-hydroxypropyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-[(1*S*)-1-phenylethyl]-1,2,3,4,4a,5,6,6a,6b,7,8,8a,9,10,12a, 14,14a,14b-octadecahydropicene-2-carboxamide;
(2*S*,4a*S*,6a*S*,6b*R*,8a*R*,12a*S*,14a*S*,14b*R*)-11-cyano-*N*-cyclohexyl-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-*N*-benzyl-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N-*(1,3-dimethoxypropan-2-yl)-14a-hydroxy-2,4a,6a,6b,9,9,12a-heptamethyl-10,14-dioxo-1,2,3,4,4a,5,6,6a,6b,7,8,8a,9,10,12a, 14,14a, 14b-octadecahydropicene-2-carboxamide;
methyl 1-{[(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl]carbonyl}prolinate;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-10,14-dioxo-*N*-propyl-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-*N*-(2-furylmethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(4-methoxyphenyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-*N*-(2-methoxyethyl)-*N*,2,4a,6a,6b,9,9,12a-octamethyl-10,14-dioxo-1,2,3,4,4a,5,6,6a,6b,7,8,8a,9,10,12a, 14,14a, 14b-octadecahydropicene-2-carboxamide;
(2*S*,4a*S*,6a*S*,6b*R*,8a*R*,12a*S*,14aS*,*14b*R*)-11-cyano-*N*-(cyanomethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(1,2-oxazol-5-ylmethyl)-10,14-dioxo-1,2,3,4,4a,5,6,6a,6b,7,8,8a,9,10,12a,14,14a,14b-octadecahydropicene-2-carboxamide;
(4a*R*,6a*R*,6b*S*,8a*S*1,11*S*,12a*R*,12b*S*,14b*S*)-12b-hydroxy-4,4,6a,6b,8a,11,14b-heptamethyl-3,13-dioxo-11-(pyrrolidin-1-ylcarbonyl)-3,4,4a,5,6,6a,6b,7,8,8a,9,10,11,12,12a,12b,13,14b-octadecahydropicene-2-carbonitrile;
(2*S*,4a*S*,6a*S*,6b*R*,8a*R*,12a*S*,14a*S*,14b*R*)-11-cyano-14a-hydroxy-*N*-isobutyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*S*,6b*R*,8a*R*,12a*S*,14a*S*,14b*R*)-11-cyano-14a-hydroxy-2,4a,6a,6b,9,9,12a-heptamethyl-*N*-(2-methylbutyl)-10,14-dioxo-1,2,3,4,4a,5,6,6a,6b,7,8,8a,9,10,12a, 14,14a, 14b-octadecahydropicene-2-carboxamide;
(2*S*,4a*S*,6a*R*,6b*S*,8a*R*,12a*S*,14a*R*,14b*S*)-11-cyano-*N*-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N*-(2-furylmethyl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N*-cyclohexyl-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-*N*-(1,3-dimethoxypropan-2-yl)-2,4a,6a,6b,9,9,12a-heptamethyl-10,14-dioxo-1,2,3,4,4a,5,6,6a,6b,7,8,8a,9,10,12a,14,14a,14b-octadecahydropicene-2-carboxamide;
(2*S*,4a*S*,6a*R*,6b*S*,8a*R*,12a*S*,14a*R*,14b*S*)-11-cyano-2,4a,6a,6b,9,9,12a-heptamethyl-*N*-[(1-methyl-1*H*-pyrazol-4-yl)methyl]-10,14-dioxo-1,2,3,4,4a,5,6,6a,6b,7,8,8a,9,10,12a,14,14a,14b-octadecahydropicene-2-carboxamide; and
(2*S*,4a*S*,6a*R*,6b*S*,8a*R*,12a*S*,14a*R*,14b*S*)-11-cyano-2,4a,6a,6b,9,9,12a-heptamethyl-*N*-(1,3-oxazol-5-ylmethyl)-10,14-dioxo-1,2,3,4,4a,5,6,6a,6b,7,8,8a,9,10,12a,14,14a,14b-octadecahydropicene-2-carboxamide.

19. The compound, or a salt thereof, of any one of claims 1 to 18, wherein the salt is a pharmaceutically acceptable salt.

20. A pharmaceutical composition comprising a therapeutically effective amount of a compound of any one of claims 1-18, or a pharmaceutically acceptable salt thereof, in combination with a pharmaceutically acceptable carrier.

21. A compound of any one of claims 1-18, or a pharmaceutically acceptable salt thereof, for use in a method of treating a disease with an inflammatory component in a subject in need thereof.

22. A compound of any one of claims 1-18, or a pharmaceutically acceptable salt thereof, for use in a method of treating a condition in a subject in need thereof, wherein the condition is selected from the group consisting of diseases with an inflammatory component or an autoimmune component; and combinations thereof.

## Patentansprüche

1. Eine Verbindung mit der Formel (I) 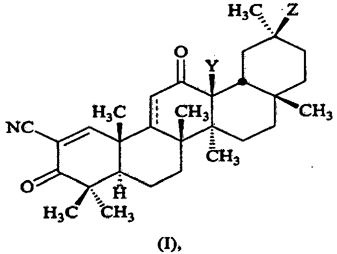 oder ein pharmazeutisch verträgliches Salz davon, worin
----- eine Einzel- oder Doppelbindung ist;
Y ist -OR¹; und
Z ist -CO₂H, -CO₂R², -C(O)NR³R⁴, -C(O)G¹, -NR³C(O)R⁵, -NR³S(O)₂R⁵, -CH₂NR³C(O)R⁵, -CH₂NR³S(O)₂R⁵, -NR³R⁶, oder -CH₂NR³R⁶;
oder
Y ist Wasserstoff; und
Z ist -C(O)NR³R¹⁴, -C(O)G¹, -NR³C(O)R⁵, -NR³S(O)₂R⁵, -CH₂NR³C(O)R⁵, -CH₂NR³S(O)₂R⁵, -NR³R⁶, oder -CH₂NR³R⁶;
R¹ ist Wasserstoff, C₁-C₆-Alkyl, C₁-C₆-Haloalkyl, -CH₂CR^{2a}=CR^{2b}R^{2c}, -CH₂G^{2a}, -G^{2a}, -CH₂G^{2b}, -G^{2b}, oder C(O)R^{1a};
R² ist C₁-C₆-Alkyl, C₁-C₆-Haloalkyl, -CH₂CR^{2a}=CR^{2b}R^{2c}, -CH₂G^{2a}, -G^{2a}, -CH₂G^{2b}, oder -G^{2b};
R³ ist Wasserstoff, C₁-C₆-Alkyl, C₁-C₆-Haloalkyl, -CH₂CR^{2a}=CR^{2b}R^{2c}, -CH₂G^{2a}, -G^{2a}, -CH₂G^{2b}, oder -G^{2b};
R⁴ ist Wasserstoff, C₁-C₈-Alkyl, C₁-C₈-Alkenyl, C₁-C₈-Alkinyl, C₁-C₈-Haloalkyl, C₁-C₆-Alkoxy-C₁-C₆-alkyl, di(C₁-C₆-Alkoxy)-C₁-C₆-alkyl, C₁-C₆-Cyanoalkyl, C₁-C₆-Hydroxyalkyl, C₁-C₆-di(Hydroxy)alkyl, -(CR^{4a}R^{4b})ₘ-N(R³)₂, -(CR^{4a}R^{4b})ₘ-N(R³)C(O)R³, -G^{4a},-(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, oder -SO₂R^{1a};
R⁵ ist C₁-C₈-Alkyl, C₁-C₈-Alkenyl, C₁-C₈-Alkinyl, C₁-C₈-Haloalkyl, C₁-C₆-Alkoxy, C₁-C₆-Alkoxy-C₁-C₆-alkyl, C₁-C₆-Alkoxy-C₁-C₆-alkoxy-C₁-C₆-alkyl, Cyano, C₁-C₆-Cyanoalkyl, C₁-C₆-Hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, - (CR^{5a}R^{5b})ₙ-N(R³)C(O)R³, -(CR^{5a}R^{5b})ₙ-N(R³)C(O)N(R³)₂, - (CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, oder -(CR^{5a}R^{5b})ₙ-NHC(O)G^{5b};
R⁶ ist Wasserstoff, C₁-C₈-Alkyl, C₁-C₈-Alkenyl, C₁-C₈-Alkinyl, C₁-C₈-Haloalkyl, C₁-C₆-Alkoxy-C₁-C₆-alkyl, di(C₁-C₆-Alkoxy)-C₁-C₆-alkyl, C₁-C₆-Cyanoalkyl, C₁-C₆-Hydroxyalkyl, C₁-C₆-di(Hydroxy)alkyl, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², oder -(CR^{4a}R^{4b})ₘ-C(O)N(R³)₂;
R¹⁴ ist C₁-C₈-Alkenyl, C₁-C₈-Alkinyl, C₁-C₈-Haloalkyl, C₁-C₆-Alkoxy-C₁-C₆-alkyl, C₁-C₆-Alkoxy-C₁-C₆-alkoxy-C₁-C₆-alkyl, di(C₁-C₆-Alkoxy)-C₁-C₆-alkyl, C₁-C₆-Cyanoalkyl, C₁-C₆-Hydroxyalkyl, C₁-C₆-di(Hydroxy)alkyl, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, - (CR^{4a}R^{4b})ₘ-C(O)N(R³)₂, -SO₂R^{1a}, oder -(CR^{4a}R^{4b})ₘ-SO₂R^{1a};
R^{1a} ist, bei jedem Vorkommen, unabhängig C₁-C₆-Alkyl, C₁-C₆-Haloalkyl, C₃-C₈-Cycloalkyl, oder C₃-C₈-Cycloalkyl-C₁-C₃-alkyl;
R^{1b} ist, bei jedem Vorkommen, unabhängig Wasserstoff, C₁-C₆-Alkyl oder C₁-C₆-Haloalkyl;
R^{2a}, R^{2b}, und R^{2c} sind unabhängig Wasserstoff, C₁-C₆-Alkyl oder C₁-C₆-Haloalkyl;
R^{4a} und R^{4b} sind, bei jedem Vorkommen, unabhängig Wasserstoff, C₁-C₆-Alkyl, oder C₁-C₆-Haloalkyl;
R^{4c} ist, bei jedem Vorkommen unabhängig Wasserstoff, C₁-C₄-Alkyl, C₂-C₄-Alkenyl, C₂-C₄-Alkinyl oder C₁-C₄-Haloalkyl;
R^{4d} ist, bei jedem Vorkommen, unabhängig C₁-C₄-Alkyl, C₂-C₄-Alkenyl, C₂-C₄-Alkinyl oder C₁-C₄-Haloalkyl;
R^{5a} und R^{5b} sind, bei jedem Vorkommen, unabhängig Wasserstoff, C₁-C₆-Alkyl, oder C₁-C₆-Haloalkyl;
R^{5c} ist, bei jedem Vorkommen, unabhängig Wasserstoff, C₁-C₄-Alkyl, C₂-C₄-Alkenyl, C₂-C₄-Alkinyl oder C₁-C₄-Haloalkyl;
R^{5d} ist, bei jedem Vorkommen, unabhängig C₁-C₄-Alkyl, C₂-C₄-Alkenyl, C₂-C₄-Alkinyl oder C₁-C₄-Haloalkyl;
G¹ ist ein Stickstoff-enthaltender Heterozyklus oder Heteroaryl, worin der Heterozyklus oder das Heteroaryl angeheftet ist an das Carbonyl von Z bei einem Stickstoffatom und unsubstituiert oder substituiert ist mit ein oder mehreren Halogen, Cyano, Hydroxy, Oxo, C₁-C₆-Alkoxy, C₁-C₈-Alkyl, C₁-C₈-Alkenyl, C₁-C₈-Alkinyl, C₁-C₈-Haloalkyl, C₁-C₆-Alkoxy-C₁-C₆-alkyl, C₁-C₆-Cyanoalkyl, C₁-C₆-Hydroxyalkyl, -CO₂R^{1a}, oder -C(O)N(R³)₂;
G^{2a} ist C₃-C₈-Cycloalkyl;
G^{2b} ist Aryl oder Heteroaryl, worin das Aryl unsubstituiert oder substituiert ist mit 1, 2, 3, 4 oder 5 C₁-C₆-Alkyl, C₁-C₆-Alkoxy, Cyano oder Halogen;
G^{4a} ist Cycloalkyl, Cycloalkenyl oder Heterozyklus, worin das Cycloalkyl, Cycloalkenyl, und der Heterozyklus unsubstituiert oder substituiert sind mit C₁-C₄-Alkyl, C₂-C₄-Alkenyl, C₂-C₄-Alkinyl, Halogen, Cyano, Oxo, -NO₂, -OR^{4c}, -(CR^{4a}R^{4b})ₘ-OR^{4c}, -OC(O)R^{4d}, -OC(O)N (R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-cyanoalkyl, C₁-C₄-haloalkyl, C₁-C₄-hydroxyalkyl, -G^{a}, oder -(CR^{4a}R^{4b})ₘ-G^{a};
G^{4b} ist Aryl oder Heteroaryl, worin das Aryl und das Heteroaryl unsubstituiert oder substituiert sind mit C₁-C₄-Alkyl, C₂-C₄-Alkenyl, C₂-C₄-Alkinyl, Halogen, Cyano, -NO₂, -OR^{4c}, -Alkyl-OR^{4c},-OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), C₁-C₄-Cyanoalkyl, C₁-C₄-Haloalkyl, -G^{a}, -(CR^{4a}R^{4b})ₘ-G^{a}, oder -O-_{G}^{a};
G^{5a} ist Cycloalkyl, Cycloalkenyl oder Heterozyklus, worin das Cycloalkyl, Cycloalkenyl, und der Heterozyklus unsubstituiert oder substituiert sind mit C₁-C₄-Alkyl, C₂-C₄-Alkenyl, C₂-C₄-Alkinyl, Halogen, Cyano, Oxo, -NO₂, -OR^{5c}, -Alkyl-OR^{5c}, -OC(O)R^{5d}, -OC(O)N(R^{5c})₂, -SR^{5c}, -S(O)R^{5d}, -S(O)²R^{5d}, -S(O)_{2N}(R^{5C})_{2'} -C(O)R^{5d}, -C(O)OR^{5c}, -C(O)N(R^{5c})₂, -N(R^{5c})₂, -N(R^{5c})C(O)R^{5d}, -N(R^{5c})C(O)O(R⁵d), -N(R^{5c})S(O)₂(R^{5d}), C₁-C₄-Cyanoalkyl, C₁-C₄-Haloalkyl, -G^{a}, oder -(CR^{5a}R^{5b})ₙ-G^{a};
G^{5b} ist Aryl oder Heteroaryl, worin das Aryl und das Heteroaryl unsubstituiert oder substituiert sind mit C₁-C₄-Alkyl, C₂-C₄-Alkenyl, C₂-C₄-Alkinyl, Halogen, Cyano, Oxo, -NO₂, -OR^{5c}, -Alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), C₁-C₄-Cyanoalkyl, C₁-C₄-Haloalkyl, -G^{a}, -(CR^{5a}R^{5b})ₙ-G^{a}, oder -O-G^{a};
G^{5c} ist Aryl oder Heteroaryl, worin das Aryl oder Heteroaryl unsubstituiert oder substituiert ist mit 1, 2, 3, 4 oder 5 C₁-C₆-Alkyl, C₁-C₆-Alkoxy, Cyano oder Halogen;
G^{a} ist Aryl oder Heteroaryl, worin das Aryl oder Heteroaryl unsubstituiert oder substituiert ist mit 1, 2, 3, 4 oder 5 C₁-C₆-Alkyl, Cyano oder Halogen;
m ist, bei jedem Vorkommen, unabhängig 1, 2, 3, 4, oder 5; und
n ist, bei jedem Vorkommen, unabhängig 1, 2, 3, 4, oder 5.

2. Die Verbindung gemäß Anspruch 1, oder ein Salz davon, worin Y -OR¹ ist.

3. Die Verbindung gemäß Anspruch 1, oder ein Salz davon, worin Y OH ist.

4. Die Verbindung gemäß Anspruch 3, oder ein Salz davon, worin Z -CO₂H oder -CO₂R² ist.

5. Die Verbindung gemäß Anspruch 4, worin R² C₁-C₆-Alkyl ist.

6. Die Verbindung gemäß Anspruch 3, oder ein Salz davon, worin Z -C(O)NR³R⁴ oder -C(O)G¹ ist.

7. Die Verbindung gemäß Anspruch 6, worin
R² C₁-C₆-Alkyl ist;
R³ ist Wasserstoff oder C₁-C₆-Alkyl;
R⁴ ist Wasserstoff, C₁-C₈-Alkyl, C₁-C₈-Alkinyl, C₁-C₆-Alkoxy-C₁-C₆-alkyl, di (C₁-C₆-Alkoxy)-C₁-C₆-alkyl, C₁-C₆-Cyanoalkyl, C₁-C₆-Hydroxyalkyl, C₁-C₆-di (Hydroxy) alkyl, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, oder -SO₂R^{1a};
R^{1a} ist, bei jedem Vorkommen, unabhängig C₁-C₆-Alkyl;
R^{4a} und R^{4b} sind, bei jedem Vorkommen, unabhängig Wasserstoff oder C₁-C₆-Alkyl;
R^{4c} ist, bei jedem Vorkommen, unabhängig Wasserstoff oder C₁-C₄-Alkyl;
R^{4d} ist, bei jedem Vorkommen, unabhängig C₁-C₄-Alkyl oder C₁-C₄-Haloalkyl;
G¹ ist ein Stickstoff-enthaltender/s Heterozyklus oder Heteroaryl, worin der Heterozyklus oder das Heteroaryl angeheftet sind an das Carbonyl von Z an einem Stickstoffatom, und unsubstituiert oder substituiert sind mit einem oder mehreren Halogen, Hydroxy, C₁-C₆-Alkoxy, C₁-C₈-Alkyl, C₁-C₈-Haloalkyl, C₁-C₆-Hydroxyalkyl, -CO₂R^{1a}, oder -C(O)N(R³)₂;
G^{4a} ist Cycloalkyl oder Heterozyklus, worin das Cycloalkyl und der Heterozyklus unsubstituiert oder substituiert sind mit C₁-C₄-Alkyl, Halogen, Cyano, Oxo, -OR^{4c}, -C(O)R^{4d}, -C(O)OR^{4c}, -C(O)N(R^{4c})₂, -N(R^{4c})C(O)R^{4d}, **-**N(R^{4c})C(O)O(R^{4d}), -N(R^{4c})S(O)₂(R^{4d}), C₁-C₄-Haloalkyl, C₁-C₄-Hydroxyalkyl oder -(CR^{4a}R^{4b})ₘ-G^{a};
G^{4b} ist Aryl oder Heteroaryl, worin das Aryl und das Heteroaryl unsubstituiert oder substituiert ist mit C₁-C₄-Alkyl, Halogen, Cyano, -OR^{4c}, C₁-C₄-Haloalkyl oder -O-G^{a};
G^{a} ist Aryl, worin das Aryl unsubstituiert oder substituiert ist mit 1, 2, 3, 4 oder 5 C₁-C₆-Alkyl, Cyano oder Halogen; und
m ist, bei jedem Vorkommen, unabhängig 1, 2, 3 oder 4.

8. Die Verbindung gemäß Anspruch 6, worin
R³ Wasserstoff oder C₁-C₆-Alkyl ist;
R⁴ ist C₁-C₈-Alkyl, C₁-C₆-Alkoxy-C₁-C₆-alkyl, di (C₁-C₆-Alkoxy)-C₁-C₆-alkyl, C₁-C₆-Cyanoalkyl, C₁-C₆-Hydroxyalkyl, -G^{4a},-(CR^{4a}R^{4b})ₘ-G^{4a}, -G^{4b}, oder -(CR^{4a}R^{4b})ₘ-G^{4b};
R^{1a} ist, bei jedem Vorkommen, unabhängig C₁-C₆-Alkyl;
R^{4a} und R^{4b} sind, bei jedem Vorkommen, unabhängig Wasserstoff oder C₁-C₆-Alkyl;
R^{4c} ist, bei jedem Vorkommen, unabhängig C₁-C₄-Alkyl;
G¹ ist ein Stickstoff-enthaltender/s Heterozyklus oder Heteroaryl, worin der Heterozyklus oder das Heteroaryl angeheftet ist an das Carbonyl von Z an einem Stickstoffatom, und unsubstituiert oder substituiert ist mit einem oder mehreren -CO₂R^{1a};
G^{4a} ist Cycloalkyl oder Heterozyklus;
G^{4b} ist Aryl oder Heteroaryl, worin das Aryl und das Heteroaryl unsubstituiert oder substituiert ist mit C₁-C₄-Alkyl, oder -OR^{4c}; und
m ist, bei jedem Vorkommen, unabhängig 1 oder 2.

9. Die Verbindung gemäß Anspruch 3, oder ein Salz davon, worin Z -NR³C(O)R⁵, -NR³S(O)₂R⁵, -CH₂NR³C(O)R⁵, oder -CH₂NR³S(O)₂R⁵ ist.

10. Die Verbindung gemäß Anspruch 9, worin
R² C₁-C₆-Alkyl ist;
R³ ist Wasserstoff oder C₁-C₆-Alkyl;
R⁵ ist C₁-C₈-Alkyl, C₁-C₈-Alkenyl, C₁-C₈-Alkinyl, C₁-C₈-Haloalkyl, C₁-C₆-Alkoxy, C₁-C₆-Alkoxy-C₁-C₆-alkyl, C₁-C₆-Alkoxy-C₁-C₆-alkoxy-C₁-C₆-alkyl, Cyano, C₁-C₆-Cyanoalkyl, C₁-C₆-Hydroxyalkyl, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a},-(CR^{5a}R^{5b})ₙ-SO₂R^{1a}, - (CR^{5a}R^{5b})ₙ-NHSO₂R^{1a}, -NHC(O)R^{1a}, -NHC(O)OR^{1a}, -G^{5a}, -(CR^{5a}R^{5b})ₙ-G^{5a}, -G^{5b}, -O-G^{5b}, -N(R^{1b}) -G^{5b}, - (CR^{5a}R^{5b})ₙ-G^{5b}, oder -(CR^{5a}R^{5b})ₙ-NHC(O)G^{5b};
R^{1a} ist, bei jedem Vorkommen, unabhängig C₁-C₆-Alkyl;
R^{1b} ist, bei jedem Vorkommen, unabhängig Wasserstoff oder C₁-C₆-Alkyl;
R^{5a} und R^{5b} sind, bei jedem Vorkommen, unabhängig Wasserstoff oder C₁-C₆-Alkyl;
R^{5c} ist, bei jedem Vorkommen, unabhängig Wasserstoff oder C₁-C₄-Alkyl;
G^{5a} ist Cycloalkyl oder Heterozyklus, worin das Cycloalkyl und Heterozyklus unsubstituiert oder substituiert sind mit C₁-C₄-Alkyl, Oxo, -OR^{5c} oder -C(O)OR^{5c};
G^{5b} ist Aryl oder Heteroaryl, worin das Aryl und Heteroaryl unsubstituiert oder substituiert sind mit C₁-C₄-Alkyl, Halogen, -OR^{5c}, -C(O)N(R^{5c})₂ oder C₁-C₄-Haloalkyl;
G^{5c} ist Aryl, worin das Aryl unsubstituiert oder substituiert ist mit 1, 2, 3, 4 oder 5 C₁-C₆-Alkyl, C₁-C₆-Alkoxy, Cyano oder Halogen; und
n ist, bei jedem Vorkommen, unabhängig 1, 2, 3 oder 4.

11. Die Verbindung gemäß Anspruch 3, oder ein Salz davon, worin Z -NR³R⁶ oder -CH₂NR³R⁶ ist.

12. Die Verbindung gemäß Anspruch 1, oder ein Salz davon, worin Y H ist.

13. Die Verbindung gemäß Anspruch 12, oder ein Salz davon, worin Z -C(O)NR³R¹⁴ oder -C(O)G¹ ist.

14. Die Verbindung gemäß Anspruch 13, worin
R² C₁-C₆-Alkyl ist;
R³ ist Wasserstoff oder C₁-C₆-Alkyl;
R¹⁴ ist C₁-C₈-Alkenyl, C₁-C₈-Alkinyl, C₁-C₈-Haloalkyl, C₁-C₆-Alkoxy-C₁-C₆-alkyl, C₁-C₆-Alkoxy-C₁-C₆-alkoxy-C₁-C₆-alkyl, di (C₁-C₆-Alkoxy) -C₁-C₆-alkyl, C₁-C₆-Cyanoalkyl, C₁-C₆-Hydroxyalkyl, C₁-C₆-di(Hydroxy)alkyl, -G^{4a}, - (CR^{4a}R^{4b})ₘ-G^{4a}, -G^{4b}, -(CR^{4a}R^{ab})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -SO₂R^{1a}, oder -(CR^{4a}R^{4b})ₘ-SO₂R^{1a};
R^{1a} ist, bei jedem Vorkommen, unabhängig C₁-C₆-alkyl, C₁-C₆-Haloalkyl, C₃-C₈-Cycloalkyl, oder C₃-C₈-Cycloalkyl-C₁-C₃-alkyl;
R^{4a} und R^{4b} sind, bei jedem Vorkommen, unabhängig Wasserstoff oder C₁-C₆-Alkyl;
R^{4c} ist, bei jedem Vorkommen, unabhängig Wasserstoff oder C₁-C₄-Alkyl;
R^{4d} ist, bei jedem Vorkommen, unabhängig C₁-C₄-Alkyl oder C₁-C₄-Haloalkyl;
G^{4a} ist Cycloalkyl oder Heterozyklus, worin das Cycloalkyl und der Heterozyklus unsubstituiert oder substituiert sind mit C₁-C₄-Alkyl, Halogen, Cyano, Oxo, -OR^{4c}, -C(O)R^{4d}, -C(O)OR^{4c}, -C(O)N(R^{4c})₂, -N(R^{4c}) C(O)R^{4d}, -N(R^{4c})C(O)O(R^{4d}), -N(R^{4c})S(O)₂(R^{4d}), C₁-C₄-Haloalkyl, C₁-C₄-Hydroxyalkyl oder -(CR^{9a}R^{4b})ₘ-G^{a};
G^{4b} ist Aryl oder Heteroaryl, worin das Aryl und das Heteroaryl unsubstituiert oder substituiert sind mit C₁-C₄-Alkyl, Halogen, Cyano, -O-G^{a} oder -OR^{4c};
G^{a} ist Aryl, worin das Aryl unsubstituiert oder substituiert ist mit 1, 2, 3, 4 oder 5 C₁-C₆-Alkyl, Cyano oder Halogen; und
m ist, bei jedem Vorkommen, unabhängig 1, 2, 3 oder 4.

15. Die Verbindung gemäß Anspruch 12, oder ein Salz davon, worin Z -NR³C(O)R⁵, -NR³S(O)₂R⁵, -CH₂NR³C (O)R⁵, oder -CH₂NR³S(O)₂R⁵ ist.

16. Die Verbindung gemäß Anspruch 15, worin
R²C₁-C₆-Alkyl ist;
R³ ist Wasserstoff oder C₁-C₆-Alkyl;
R⁵ ist C₁-C₈-Alkyl, C₁-C₈-Alkenyl, C₁-C₈-Alkinyl, C₁-C₈-Haloalkyl, C₁-C₆-Alkoxy, C₁-C₆-alkoxy-C₁-C₆-alkyl, C₁-C₆-Alkoxy-C₁-C₆-alkoxy-C₁-C₆-alkyl, Cyano, C₁-C₆-Cyanoalkyl, C₁-C₆-Hydroxyalkyl, -(CR^{5a}R^{5b})ₙ-C(O)OR², - (CR^{5a}R^{5b})ₙ-N(R³)₂, - (CR^{5a}R^{5b})ₙ-C(O)N(R³)₂, -(CR^{5a}R^{5b})ₙ-N(R³)C(O)R³, - (CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a},-(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, oder -(CR^{5a}R^{5b})ₙ-NHC(O)G^{5b};
R^{1a} ist, bei jedem Vorkommen, unabhängig C₁-C₆-Alkyl;
R^{1b} ist, bei jedem Vorkommen, unabhängig Wasserstoff oder C₁-C₆-Alkyl;
R^{5a} und R^{5b} sind, bei jedem Vorkommen, unabhängig Wasserstoff oder C₁-C₆-Alkyl;
R^{5c} ist, bei jedem Vorkommen unabhängig Wasserstoff, C₁-C₄-Alkyl oder C₁-C₄-Haloalkyl;
G^{5a} ist Cycloalkyl oder Heterozyklus, worin das Cycloalkyl und Heterozyklus unsubstituiert oder substituiert ist mit C₁-C₄-Alkyl, Oxo, -OR^{5c} oder -C(O)OR^{5c};
G^{5b} ist Aryl oder Heteroaryl, worin das Aryl und Heteroaryl unsubstituiert oder substituiert sind mit C₁-C₄-Alkyl, Halogen, -OR^{5c}, -C(O)N(R^{5c}) oder C₁-C₉-Haloalkyl;
G^{5c} ist Aryl, worin das Aryl unsubstituiert oder substituiert ist mit 1, 2, 3, 4 oder 5 C₁-C₆-Alkyl, C₁-C₆-Alkoxy, Cyano oder Halogen; und
n ist, bei jedem Vorkommen, unabhängig 1, 2, 3 oder 4.

17. Die Verbindung gemäß Anspruch 12, oder ein Salz davon, worin Z -NR³R⁶ oder -CH₂NR³R⁶ ist.

18. Die Verbindung gemäß Anspruch 1, oder ein Salz davon, worin die Verbindung gewählt ist aus der Gruppe bestehend aus:
(2*S*,4a*S*,6a*S*,6b*R*,8a*R*,12a*S*,14a*S*,14b*R*)-Methyl 11-Cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxylat;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-(1,3-oxazol-5-ylmethyl)-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carbonsäure;
(2*S*,4a*S*,6a*S*,6b*R*,8a*R*,12a*S*,14a*S*,14b*R*)-11-Cyano-14a-hydroxy-*N-*(2-hydroxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-*N*-(pyridazin-4-ylmethyl)-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-*N*,2,4a,6a,6b,9,9,12a-octamethyl-10,14-dioxo-N-propyl-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-*N*-(2,2-dimethylpropyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-*N*-(pyridazin-3-ylmethyl)-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-[(1-methyl-1H-pyrazol-4-yl)methyl]-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-[2-(morpholin-4-yl)ethyl]-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-(2-methylbenzyl)-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-[(1-methyl-1*H*-pyrazol-3-yl)methyl]-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-*N-*(2-methoxybenzyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-*N-*(2-hydroxybutyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(4a*R*,6a*R*,6b*S*,8a*S*,11*S*,12a*R*,12b*S*,14b*S*)-12b-Hydroxy-11-(1*H-*imidazol-1-ylcarbonyl)-4,4,6a,6b,8a,11,14b-heptamethyl-3,13-dioxo-3,4,4a,5,6,6a,6b,7,8,8a,9,10,11,12,12a,12b,13,14b-octadecahydropicen-2-carbonitril;
(2*S*,4a*S*,6a*S*,6b*R*,8a*R*,12a*S*,14a*S*,14b*R*)-11-Cyano-14a-hydroxy-*N-*(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-(3-methylbutyl)-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-*N*-(tetrahydro-2*H-*pyran-4-yl)-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-*N-*(2-hydroxypropyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-*N*-[(1*S*)-1-phenylethyl]-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-*N*-cyclohexyl-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-*N*-Benzyl-11-cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-*N*-(1,3-dimethoxypropan-2-yl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
Methyl 1-{[(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-yl]carbonyl}prolinat;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-10,14-dioxo-*N*-propyl-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-*N*-(2-furylmethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-*N-*(4-methoxyphenyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(*2*S,4a*S*,6a*S*,6b*R*,8a*R*,12a*S*,14a*S*,14b*R*)-11-Cyano-14a-hydroxy-*N-*(2-methoxyethyl)-*N*,2,4a,6a,6b,9,9,12a-octamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-*N-*(cyanomethyl)-14a-hydroxy-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-(1,2-oxazol-5-ylmethyl)-10,14-dioxo-1,2,3,4,4a,5,6,6a,6b,7,8,8a,9,10,12a,14,14a,14b-octadecahydropicen-2-carboxamid;
(4a*R*,6a*R*,6b*S*,8a*S*,11*S*,12a*R*,12b*S*,14b*S*)-12b-Hydroxy-4,4,6a,6b,8a,11,14b-heptamethyl-3,13-dioxo-11-(pyrrolidin-1-ylcarbonyl)-3,4,4a,5,6,6a,6b,7,8,8a,9,10,11,12,12a,12b,13,14b-octadecahydropicen-2-carbonitril;
(2*S*,4a*S*,6a*S*,6b*R*,8a*R*,12a*S*,14a*S*,14b*R*)-11-Cyano-14a-hydroxy-*N-*isobutyl-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*S*,6b*R*,8a*R*,12a*S*,14a*S*,14b*R*)-11-Cyano-14a-hydroxy-2,4a,6a,6b,9,9,12a-heptamethyl-*N*-(2-methylbutyl)-10,14-dioxo-1,2,3,4,4a,5,6,6a,6b,7,8,8a,9,10,12a,14,14a,14b-octadecahydropicen-2-carboxamid;
(2*S*,4a*S*,6a*R*,6b*S*,8a*R*,12a*S*,14a*R*,14b*S*)-11-Cyano-*N*-(2-methoxyethyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*R*,6b*S*,8a*R*,12a*S*,14a*R*,14b*S*)-11-Cyano-*N*-(2-furylmethyl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*R*,6b*S*,8a*R*,12a*S*,14a*R*,14b*S*)-11-Cyano-*N*-cyclohexyl-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*R*,6b*S*,8a*R*,12a*S*,14a*R*,14b*S*)-11-Cyano-*N*-(1,3-dimethoxypropan-2-yl)-2,4a,6a,6b,9,9,12a-heptamethyl-10,14-dioxo-1,2,3,4,4a,5,6,6a,6b,7,8,8a,9,10,12a,14,14a,14b-octadecahydropicen-2-carboxamid;
(2*S*,4a*S*,6a*R*,6b*S*,8a*R*,12a*S*,14a*R*,14b*S*)-11-Cyano-2,4a,6a,6b,9,9,12a-heptamethyl-*N*-[(1-methyl-1*H*-pyrazol-4-yl)methyl]-10,14-dioxo-1,2,3,4,4a,5,6,6a,6b,7,8,8a,9,10,12a,14,14a,14b-octadecahydropicen-2-carboxamid; und
(2*S*,4a*S*,6a*R*,6b*S*,8a*R*,12a*S*,14a*R*,14b*S*)-11-Cyano-2,4a,6a,6b,9,9,12a-heptamethyl-*N*-(1,3-oxazol-5-ylmethyl)-10,14-dioxo-1,2,3,4,4a,5,6,6a,6b,7,8,8a,9,10,12a,14,14a,14b-octadecahydropicen-2-carboxamid.

19. Die Verbindung, oder ein Salz davon, gemäß irgendeinem der Ansprüche 1 bis 18, worin das Salz ein pharmazeutisch verträgliches Salz ist.

20. Eine pharmazeutische Zusammensetzung umfassend eine therapeutisch wirksame Menge einer Verbindung gemäß irgendeinem der Ansprüche 1-18, oder ein pharmazeutisch verträgliches Salz davon, in Kombination mit einem pharmazeutisch verträglichen Träger.

21. Eine Verbindung gemäß irgendeinem der Ansprüche 1-18, oder ein pharmazeutisch verträgliches Salz davon, für die Verwendung in einem Verfahren zur Behandlung einer Erkrankung mit einer entzündlichen Komponente in einem Patienten, der dies benötigt.

22. Eine Verbindung gemäß irgendeinem der Ansprüche 1-18, oder ein pharmazeutisch verträgliches Salz davon, für die Verwendung in einem Verfahren zur Behandlung eines Zustands in einem Patienten, der dies benötigt, worin der Zustand gewählt ist aus der Gruppe bestehend aus Erkrankungen mit einer entzündlichen Komponente oder einer autoimmunen Komponente; und Kombinationen daraus.

## Revendications

1. Composé de formule (I), 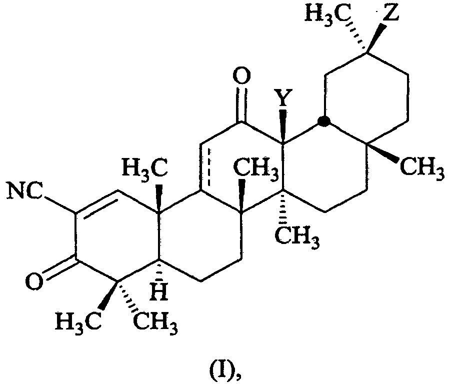 ou l'un de ses sels pharmaceutiquement acceptables, dans lequel
----- représente une liaison simple ou double ;
Y représente -OR¹ ; et
Z représente -CO₂H, -CO₂R², -C(O)NR³R⁴, -C(O)G¹, -NR³C(O)R⁵, -NR³S(O)₂R⁵, -CH₂NR³C(O)R⁵, -CH₂NR³S(O)₂R⁵, -NR³R⁶, ou -CH₂NR³R⁶ ;
ou
Y représente un atome d'hydrogène ; et
Z représente -C(O)NR³R¹⁴, -C(O)G¹, -NR³C(O)R⁵, -NR³S(O)₂R⁵, -CH₂NR³C(O)R⁵, -CH₂NR³S(O)₂R⁵, -NR³R⁶, ou -CH₂NR³R⁶;
R¹ représente un atome d'hydrogène, un groupe alkyle en C₁ à C₆, halogénoalkyle en C₁ à C₆, -CH₂CR²a=CR^{2b}R^{2c}, -CH₂G^{2a}, -G^{2a}, -CH²G^{2b}, -G^{2b}, ou C(O)R^{1a};
R² représente un groupe alkyle en C₁ à C₆, halogénoalkyle en C₁ à C₆, -CH₂CR^{2a}=CR^{2b}R^{2c}, -CH₂G^{2a}, -G^{2a}, -CH₂G^{2b}, ou -G^{2b};
R³ représente un atome d'hydrogène, un groupe alkyle en C₁ à C₆, halogénoalkyle en C₁ à C₆, -CH₂CR^{2a}=CR^{2b}R^{2c}, -CH₂G^{2a}, -G^{2a}, -CH²G^{2b}, ou -G^{2b} ;
R⁴ représente un atome d'hydrogène, un groupe alkyle en C₁ à C₈, alcényle en C₁ à C₈, alcynyle en C₁ à C₈, halogénoalkyle en C₁ à C₈, alcoxy en C₁ à C₆-alkyle en C₁ à C₆, di (alcoxy en C₁ à C₆)-alkyle en C₁ à C₆, cyanoalkyle en C₁ à C₆, hydroxyalkyle en C₁ à C₆, di(hydroxy)alkyle en C₁ à C₆, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, ou -SO₂R^{1a} ;
R⁵ représente un groupe alkyle en C₁ à C₈, alcényle en C₁ à C₈, alcynyle en C₁ à C₈, halogénoalkyle en C₁ à C₈, alcoxy en C₁ à C₆, alcoxy en C₁ à C₆-alkyle en C₁ à C₆, alcoxy en C₁ à C₆-alcoxy en C₁ à C₆-alkyle en C₁ à C₆, cyano, cyanoalkyle en C₁ à C₆, hydroxyalkyle en C₁ à C₆, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH- (CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -N(R^{1b})-(CR^{5a}R^{5b})ₙ-CN, -G^{5a}, -(CR^{5a}R^{5b})ₙ-G^{5a}, -G^{5b}, -O-G^{5b}, -N(R^{1b}) -G^{5b}, -(CR^{5a}R^{5b})ₙ-G^{5b}, ou -(CR^{5a}R^{5b})ₙ-NHC(O)G^{5b} ;
R⁶ représente un atome d'hydrogène, un groupe alkyle en C₁ à C₈, alcényle en C₁ à C₈, alcynyle en C₁ à C₈, halogénoalkyle en C₁ à C₈, alcoxy en C₁ à C₆-alkyle en C₁ à C₆, di(alcoxy en C₁ à C₆)-alkyle en C₁ à C₆, cyanoalkyle en C₁ à C₆, hydroxyalkyle en C₁ à C₆, di(hydroxy)alkyle en C₁ à C₆, -G^{4a}, -(CR^{4a}R^{4b})ₘ-G^{4a}, -(CR^{4a}R^{4b})ₘ-G^{4b}, -(CR^{4a}R^{4b})ₘ-C(O)OR², ou - (CR^{4a}R^{4b})ₘ-C(O)N(R³)₂ ;
R¹⁴ représente un groupe alcényle en C₁ à C₈, alcynyle en C₁ à C₈, halogénoalkyle en C₁ à C₈, alcoxy en C₁ à C₆-alkyle en C₁ à C₆, alcoxy en C₁ à C₆-alcoxy en C₁ à C₆-alkyle en C₁ à C₆, di(alcoxy en C₁ à C₆)-alkyle en C₁ à C₆, cyanoalkyle en C₁ à C₆, hydroxyalkyle en C₁ à C₆, di (hydroxy) alkyle en C₁ à C₆, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO²H, -(CR^{4a}R^{4b})ₘ-C(O)N(R³)₂, -SO₂R^{1a}, ou -(CR^{4a}R^{4b})ₘ-SO₂R^{1a} ;
R^{1a} représente indépendamment, à chaque occurrence, un groupe alkyle en C₁ à C₆, halogénoalkyle en C₁ à C₆, cycloalkyle en C₃ à C₈, ou cycloalkyl en C₃ à C₈-alkyle en C₁ à C₃ ;
R^{1b} représente indépendamment, à chaque occurrence, un atome d'hydrogène, un groupe alkyle en C₁ à C₆ ou halogénoalkyle en C₁ à C₆ ;
R^{2a}, R^{2b}, et R^{2c} représentent indépendamment un atome d'hydrogène, un groupe alkyle en C₁ à C₆ ou halogénoalkyle en C₁ à C₆ ;
R^{4a} et R^{4b} représentent indépendamment, à chaque occurrence, un atome d'hydrogène, un groupe alkyle en C₁ à C₆, ou halogénoalkyle en C₁ à C₆ ;
R^{4c} représente indépendamment, à chaque occurrence, un atome d'hydrogène, un groupe alkyle en C₁ à C₉, alcényle en C₂ à C₄, alcynyle en C₂ à C₄ ou halogénoalkyle en C₁ à C₄ ;
R^{4d} représente indépendamment, à chaque occurrence, un groupe alkyle en C₁ à C₄, alcényle en C₂ à C₄, alcynyle en C₂ à C₄ ou halogénoalkyle en C₁ à C₄ ;
R^{5a} et R^{5b} représentent indépendamment, à chaque occurrence, un atome d'hydrogène, un groupe alkyle en C₁ à C₆ ou halogénoalkyle en C₁ à C₆ ;
R^{5c} représente indépendamment, à chaque occurrence, un atome d'hydrogène, un groupe alkyle en C₁ à C₉, alcényle en C₂ à C₄, alcynyle en C₂ à C₄ ou halogénoalkyle en C₁ à C₄ ;
R^{5d} représente indépendamment, à chaque occurrence, un groupe alkyle en C₁ à C₄, alcényle en C₂ à C₄, alcynyle en C₂ à C₄ ou halogénoalkyle en C₁ à C₄ ;
G¹ représente un hétérocycle ou un groupe hétéroaryle contenant de l'azote, l'hétérocycle ou le groupe hétéroaryle étant fixé au groupe carbonyle de Z au niveau d'un atome d'azote et étant non substitué ou substitué par un ou plusieurs atomes d'halogène, groupes cyano, hydroxy, oxo, alcoxy en C₁ à C₆, alkyle en C₁ à C₈, alcényle en C₁ à C₈, alcynyle en C₁ à C₈, halogénoalkyle en C₁ à C₈, alcoxy en C₁ à C₆-alkyle en C₁ à C₆, cyanoalkyle en C₁ à C₆, hydroxyalkyle en C₁ à C₆, -CO₂R^{1a}, ou -C(O)N(R³)₂ ;
G^{2a} représente un groupe cycloalkyle en C₃ à C₈ ;
G^{2b} représente un groupe aryle ou hétéroaryle, le groupe aryle étant non substitué ou substitué par 1, 2, 3, 4 ou 5 groupes alkyle en C₁ à C₆, alcoxy en C₁ à C₆, cyano ou halogéno ;
G^{4a} représente un groupe cycloalkyle, cycloalcényle ou un hétérocycle, les groupes cycloalkyle, cycloalcényle, et l'hétérocycle étant non substitués ou substitués par un groupe alkyle en C₁ à C₄, alcényle en C₂ à C₄, alcynyle en C₂ à C₄, halogéno, cyano, oxo, -NO₂, -OR^{4c}, -(CR^{4a}R^{4b})ₘ-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{9d}), cyanoalkyle en C₁ à C₄, halogénoalkyle en C₁ à C₄, hydroxyalkyle en C₁ à C₄, -G^{a}, ou -(CR^{4a}R^{4b})ₘ-G^{a} ;
G^{4b} représente un groupe aryle ou hétéroaryle, les groupes aryle et hétéroaryle étant non substitués ou substitués par un groupe alkyle en C₁ à C₄, alcényle en C₂ à C₄, alcynyle en C₂ à C₄, halogéno, cyano, -NO₂, -OR^{4c}, -alkyl-OR^{4c}, -OC(O)R^{4d}, -OC(O)N(R^{4c})₂, -SR^{4c}, -S(O)R^{4d}, -S(O)₂R^{4d}, -S(O)₂N(R^{4c})₂, -C(O)R^{4d}, -C(O)OR^{4c}, -C(O)N(R^{4c})₂, -N(R^{4c})₂, -N(R^{4c})C(O)R^{4d}, -N(R^{4c})C(O)O(R^{4d}), -N(R^{4c})S(O)₂(R^{4d}), cyanoalkyle en C₁ à C₄, halogénoalkyle en C₁ à C₄, -G^{a} ou -(CR^{4a}R^{4b})ₘ-G^{a}, -O-G^{a} ;
G^{5a} représente un groupe cycloalkyle, cycloalcényle ou un hétérocycle, les groupes cycloalkyle, cycloalcényle, et l'hétérocycle étant non substitués ou substitués par un groupe alkyle en C₁ à C₄, alcényle en C₂ à C₄, alcynyle en C₂ à C₄, halogéno, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}, -N(R^{5c})C(O)O(R^{5d}), -N(R^{5c})S(O)₂(R^{5d}), cyanoalkyle en C₁ à C₄, halogénoalkyle en C₁ à C₄, -G^{a}, ou -(CR^{5a}R^{5b})ₙ-G^{a} ;
G^{5b} représente un groupe aryle ou hétéroaryle, les groupes aryle et hétéroaryle étant non substitués ou substitués par un groupe alkyle en C₁ à C₄, alcényle en C₂ à C₄, alcynyle en C₂ à C₄, halogéno, cyano, oxo, -NO₂, -OR^{5c}, -alkyl-OR^{5c}, -OC(O)R^{5d}, -OC(O)N(R^{5c})₂, -SR^{5c}, -S(O)R^{5d}, -S(O)₂R^{5d}, -S(O)₂N(R^{5c})₂, -C(O)R^{5d}, -C(O)OR^{5c}, -C(O)N(R^{5c})₂, -N(R^{5c})₂, -N(R^{5c})C(O)R^{5d}. -N(R^{5c})C(O)O(R^{5d}), -N(R^{5c})S(O)₂(R^{5d}), cyanoalkyle en C₁ à C₄, halogénoalkyle en C₁ à C₄, -G^{a}, -(CR^{5a}R^{5b})ₙ-G^{a}, ou -O-G^{a} ;
G^{5c} représente un groupe aryle ou hétéroaryle, les groupes aryle et hétéroaryle étant non substitués ou substitués par 1, 2, 3, 4 ou 5 groupes alkyle en C₁ à C₆, alcoxy en C₁ à C₆, cyano ou halogéno ;
G^{a} représente un groupe aryle ou hétéroaryle, les groupes aryle et hétéroaryle étant non substitués ou substitués par 1, 2, 3, 4 ou 5 groupes alkyle en C₁ à C₆, cyano ou halogéno ;
m, à chaque occurrence, vaut indépendamment 1, 2, 3, 4, ou 5 ; et
n, à chaque occurrence, vaut indépendamment 1, 2, 3, 4, ou 5.

2. Composé selon la revendication 1, ou l'un de ses sels, dans lequel Y représente -OR¹.

3. Composé selon la revendication 1, ou l'un de ses sels, dans lequel Y représente OH.

4. Composé selon la revendication 3, ou l'un de ses sels, dans lequel Z représente -CO₂H ou -CO₂R².

5. Composé selon la revendication 4, dans lequel R² représente un groupe alkyle en C₁ à C₆.

6. Composé selon la revendication 3, ou l'un de ses sels, dans lequel Z représente -C(O)NR³R⁴ ou -C(O)G¹.

7. Composé selon la revendication 6, dans lequel,
R² représente un groupe alkyle en C₁ à C₆ ;
R³ représente un atome d'hydrogène ou un groupe alkyle en C₁ à C₆ ;
R⁴ représente un atome d'hydrogène, un groupe alkyle en C₁ à C₈, alcynyle en C₁ à C₈, alcoxy en C₁ à C₆-alkyle en C₁ à C₆, di (alcoxy en C₁ à C₆)-alkyle en C₁ à C₆, cyanoalkyle en C₁ à C₆, hydroxyalkyle en C₁ à C₆, di(hydroxy) alkyle en C₁ à C₆, -(CR^{4a}R^{4b})ₘ-N(R³)₂, -(CR^{4a}R^{4b})ₘ-N(R³)C(O)R³, -G^{4a}, -(CR^{4a}R^{4b})ₘ-G^{4a}, -G^{4b}, -(CR^{4a}R^{4b})ₘ-G^{4b}, -(CR^{4a}R^{4b})ₘ-C(O)OR², -(CR^{4a}R^{4b})ₘ-CO₂H, -(CR^{4a}R^{4b})ₘ-C(O)N(R³)₂, -(CR^{4a}R^{4b})ₘ-C(O)N(OH)R³, ou -SO₂R^{1a} ;
R^{1a} représente indépendamment, à chaque occurrence, un groupe alkyle en C₁ à C₆ ;
R^{4a} et R^{4b} représentent indépendamment, à chaque occurrence, un atome d'hydrogène ou un groupe alkyle en C₁ à C₆ ;
R^{4c} représente indépendamment, à chaque occurrence, un atome d'hydrogène ou un groupe alkyle en C₁ à C₄ ;
R^{4d} représente indépendamment, à chaque occurrence, un groupe alkyle en C₁ à C₄ ou halogénoalkyle en C₁ à C₄ ;
G¹ représente un hétérocycle ou un groupe hétéroaryle contenant de l'azote, l'hétérocycle ou le groupe hétéroaryle étant fixé au groupe carbonyle de Z au niveau d'un atome d'azote et étant non substitué ou substitué par un ou plusieurs atomes d'halogène, groupes hydroxy, alcoxy en C₁ à C₆, alkyle en C₁ à C₈, halogénoalkyle en C₁ à C₈, hydroxyalkyle en C₁ à C₆, -CO₂R^{1a}, ou -C(O)N(R³)₂ ;
G^{4a} représente un groupe cycloalkyle ou un hétérocycle, le groupe cycloalkyle et l'hétérocycle étant non substitués ou substitués par un groupe alkyle en C₁ à C₄, halogéno, cyano, oxo, -OR^{4c}, -C(O)R^{4d}, -C(O)OR^{4c}, -C(O)N(R^{4c})₂, -N(R^{4c})C(O)R^{4d}, -N(R^{4c})C(O)O(R^{4d}), -N(R^{4c})S(O)₂(R^{4d}), halogénoalkyle en C₁ à C₄, hydroxyalkyle en C₁ à C₄ ou -(CR^{4a}R^{4b})ₘ-G^{a} ;
G^{4b} représente un groupe aryle ou hétéroaryle, les groupes aryle et hétéroaryle étant non substitués ou substitués par un groupe alkyle en C₁ à C₄, halogéno, cyano, -OR^{4c}, halogénoalkyle en C₁ à C₄ ou -O-G^{a} ;
G^{a} représente un groupe aryle, le groupe aryle étant non substitué ou substitué par 1, 2, 3, 4 ou 5 groupes alkyle en C₁ à C₆, cyano ou halogéno ; et
m, à chaque occurrence, vaut indépendamment 1, 2, 3 ou 4.

8. Composé selon la revendication 6, dans lequel,
R³ représente un atome d'hydrogène ou un groupe alkyle en C₁ à C₆ ;
R⁴ représente un groupe alkyle en C₁ à C₈, alcoxy en C₁ à C₆-alkyle en C₁ à C₆, di (alcoxy en C₁ à C₆)-alkyle en C₁ à C₆, cyanoalkyle en C₁ à C₆, hydroxyalkyle en C₁ à C₆, -G^{4a}, -(CR^{4a}R^{4b})ₘ-G^{4a}, -G^{4b} ou -(CR^{4a}R^{4b})ₘ-G^{4b} ;
R^{1a} représente indépendamment, à chaque occurrence, un groupe alkyle en C₁ à C₆ ;
R^{4a} et R^{4b} représentent indépendamment, à chaque occurrence, un atome d'hydrogène ou un groupe alkyle en C₁ à C₆ ;
R^{4c} représente indépendamment, à chaque occurrence, un groupe alkyle en C₁ à C₄ ;
G¹ représente un hétérocycle ou un groupe hétéroaryle contenant de l'azote, l'hétérocycle ou le groupe hétéroaryle étant fixé au groupe carbonyle de Z au niveau d'un atome d'azote et étant non substitué ou substitué par un ou plusieurs -CO₂R^{1a} ;
G^{4a} représente un groupe cycloalkyle ou un hétérocycle ;
G^{4b} représente un groupe aryle ou hétéroaryle, les groupes aryle et hétéroaryle étant non substitués ou substitués par un groupe alkyle en C₁ à C₄, ou -OR^{4c} ; et
m, à chaque occurrence, vaut indépendamment 1 ou 2.

9. Composé selon la revendication 3, ou l'un de ses sels, dans lequel Z représente -NR³C(O)R⁵, -NR³S(O)₂R⁵, -CH₂NR³C(O)R⁵, ou -CH₂NR³S(O)₂R⁵.

10. Composé selon la revendication 9, dans lequel
R² représente un groupe alkyle en C₁ à C₆ ;
R³ représente un atome d'hydrogène ou un groupe alkyle en C₁ à C₆ ;
R⁵ représente un groupe alkyle en C₁ à C₈, alcényle en C₁ à C₈, alcynyle en C₁ à C₈, halogénoalkyle en C₁ à C₈, alcoxy en C₁ à C₆, alcoxy en C₁ à C₆-alkyle en C₁ à C₆, alcoxy en C₁ à C₆-alcoxy en C₁ à C₆-alkyle en C₁ à C₆, cyano, cyanoalkyle en C₁ à C₆, hydroxyalkyle en C₁ à C₆, - (CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R³, -(CR^{5a}R^{5b})ₙ-N(R³)C(O)N(R³)₂, -(CR^{5a}R^{5b})ₙ-NHC(O)OR^{1a}, - (CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, -(CR^{5a}R^{5b})ₙ-SO₂R^{1a}, -(CR^{5a}R^{5b})ₙ-NHSO₂R^{1a}, -NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, ou -(CR^{5a}R^{5b})ₙ-NHC(O)G^{5b} ;
R^{1a} représente indépendamment, à chaque occurrence, un groupe alkyle en C₁ à C₆ ;
R^{1b} représente indépendamment, à chaque occurrence, un atome d'hydrogène ou un groupe alkyle en C₁ à C₆ ;
R^{5a} et R^{5b} représentent indépendamment, à chaque occurrence, un atome d'hydrogène ou un groupe alkyle en C₁ à C₆ ;
R^{5c} représente indépendamment, à chaque occurrence, un atome d'hydrogène ou un groupe alkyle en C₁ à C₄ ;
G^{5a} représente un groupe cycloalkyle ou un hétérocycle, le groupe cycloalkyle et l'hétérocycle étant non substitués ou substitués par un groupe alkyle en C₁ à C₄, oxo, -OR^{5c} ou -C(O)OR^{5c} ;
G^{5b} représente un groupe aryle ou hétéroaryle, les groupes aryle et hétéroaryle étant non substitués ou substitués par un groupe alkyle en C₁ à C₄, halogéno, -OR^{5c}, -C(O)N(R^{5c})₂ ou halogénoalkyle en C₁ à C₄ ;
G^{5c} représente un groupe aryle, le groupe aryle étant non substitué ou substitué par 1, 2, 3, 4 ou 5 groupes alkyle en C₁ à C₆, alcoxy en C₁ à C₆, cyano ou halogéno ; et
n, à chaque occurrence, vaut indépendamment 1, 2, 3 ou 4.

11. Composé selon la revendication 3, ou l'un de ses sels, dans lequel Z représente -NR³R⁶ ou -CH₂NR³R⁶.

12. Composé selon la revendication 1, ou l'un de ses sels, dans lequel Y représente H.

13. Composé selon la revendication 12, ou l'un de ses sels, dans lequel Z représente -C(O)NR³R¹⁴ ou -C(O)G¹.

14. Composé selon la revendication 13, dans lequel,
R² représente un groupe alkyle en C₁ à C₆ ;
R³ représente un atome d'hydrogène ou un groupe alkyle en C₁ à C₆ ;
R¹⁴ représente un groupe alcényle en C₁ à C₈, alcynyle en C₁ à C₈, halogénoalkyle en C₁ à C₈, alcoxy en C₁ à C₆-alkyle en C₁ à C₆, alcoxy en C₁ à C₆-alcoxy en C₁ à C₆-alkyle en C₁ à C₆, di (alcoxy en C₁ à C₆)-alkyle en C₁ à C₆, cyanoalkyle en C₁ à C₆, hydroxyalkyle en C₁ à C₆, di(hydroxy) alkyle en C₁ à C₆, -G^{4a}, -(CR^{4a}R^{4b})ₘ-G^{4a}, -G^{4b}, -(CR^{4a}R^{4b})ₘ-G^{4b}, - (CR^{4a}R^{4b})ₘ-C(O)OR², -(CR^{4a}R^{4b})ₘ-CO₂H, -(CR^{4a}R^{4b})ₘ-C(O)N(R³)₂, -SO₂R^{1a}, ou -(CR^{4a}R^{4b})ₘ-SO₂R^{1a} ;
R^{1a} représente indépendamment, à chaque occurrence, un groupe alkyle en C₁ à C₆, halogénoalkyle en C₁ à C₆, cycloalkyle en C₃ à C₈, ou cycloalkyl en C₃ à C₈-alkyle en C₁ à C₃ ;
R^{4a} et R^{4b} représentent indépendamment, à chaque occurrence, un atome d'hydrogène ou un groupe alkyle en C₁ à C₆ ;
R^{4c} représente indépendamment, à chaque occurrence, un atome d'hydrogène ou un groupe alkyle en C₁ à C₄ ;
R^{4d} représente indépendamment, à chaque occurrence, un groupe alkyle en C₁ à C₄ ou halogénoalkyle en C₁ à C₄ ;
G^{4a} représente un groupe cycloalkyle ou un hétérocycle, le groupe cycloalkyle et l'hétérocycle étant non substitués ou substitués par un groupe alkyle en C₁ à C₄, halogéno, cyano, oxo, -OR^{4c}, -C(O)R^{4d}, -C(O)OR^{4c}, -C(O)N(R^{4c})₂, -N(R^{4c})C(O)R^{4c}, -N(R^{4c})C(O)O(R^{4d}), -N(R^{4c})S(O)₂(R^{4d}), halogénoalkyle en C₁ à C₄, hydroxyalkyle en C₁ à C₄ ou -(CR^{4a}R^{4b})ₘ-G^{a} ;
G^{4b} représente un groupe aryle ou hétéroaryle, les groupes aryle et hétéroaryle étant non substitués ou substitués par un groupe alkyle en C₁ à C₄, halogéno, cyano, -O-G^{a} ou -OR^{4c} ;
G^{a} représente un groupe aryle, le groupe aryle étant non substitué ou substitué par 1, 2, 3, 4 ou 5 groupes alkyle en C₁ à C₆, cyano ou halogéno ; et
m, à chaque occurrence, vaut indépendamment 1, 2, 3 ou 4.

15. Composé selon la revendication 12, ou l'un de ses sels, dans lequel Z représente -NR³C(O)R⁵, -NR³S(O)₂R⁵, -CH₂NR³C(O)R⁵, ou -CH₂NR³S(O)₂R⁵.

16. Composé selon la revendication 15, dans lequel,
R² représente un groupe alkyle en C₁ à C₆ ;
R³ représente un atome d'hydrogène ou un groupe alkyle en C₁ à C₆ ;
R⁵ représente un groupe alkyle en C₁ à C₈, alcényle en C₁ à C₈, alcynyle en C₁ à C₈, halogénoalkyle en C₁ à C₈, alcoxy en C₁ à C₆, alcoxy en C₁ à C₆-alkyle en C₁ à C₆, alcoxy en C₁ à C₆-alcoxy en C₁ à C₆-alkyle en C₁ à C₆, cyano, cyanoalkyle en C₁ à C₆, hydroxyalkyle en C₁ à C₆, -(CR^{5a}R^{5b})ₙ-C(O)OR², -(CR^{5a}R^{5b})ₙ-N(R³)₂, -(CR^{5a}R^{5b})ₙ-C(O)N(R³)₂, -(CR^{5a}R^{5b})ₙ-N(R³)C(O)R₃, -(CR^{5a}R^{5b})ₙ-N(R³)C(O)N(R³)₂, -(CR^{5a}R^{5b})ₙ-NHC(O)OR^{1a}, -(CR^{5a}G^{5c})ₙ-NHC(O)OR^{1a}, -N(R^{1b})₂, -NH-(CR^{5a}R^{5b})ₙ-C(O)OR², -N(R^{1b})-(CR^{5a}R^{5b})ₙ-OR^{1a}, - (CR^{5a}R^{5b})ₙ-SO₂R^{1a}, -(CR^{5a}R^{5b})n-NHSO₂R^{1a}, -NHC(O)R^{1a}, -NHC(O)OR^{1a}, -G^{5a}, -(CR^{5a}R^{5b})ₙ-G^{5a}, -G^{5b}, -O-G^{5b}, -N(R^{1b})-G^{5b}, -(CR^{5a}R^{5b})ₙ-G^{5b}, ou -(CR^{5a}R^{5b})ₙ-NHC(O)G^{5b} ;
R^{1a} représente indépendamment, à chaque occurrence, un groupe alkyle en C₁ à C₆ ;
R^{1b} représente indépendamment, à chaque occurrence, un atome d'hydrogène ou un groupe alkyle en C₁ à C₆ ;
R^{5a} et R^{5b} représentent indépendamment, à chaque occurrence, un atome d'hydrogène ou un groupe alkyle en C₁ à C₆ ;
R^{5c} représente indépendamment, à chaque occurrence, un atome d'hydrogène, un groupe alkyle en C₁ à C₄ ou halogénoalkyle en C₁ à C₄ ;
G^{5a} représente un groupe cycloalkyle ou un hétérocycle, le groupe cycloalkyle et l'hétérocycle étant non substitués ou substitués par un groupe alkyle en C₁ à C₄, oxo, -OR^{5c} ou -C(O)OR^{5c} ;
G^{5b} représente un groupe aryle ou hétéroaryle, les groupes aryle et hétéroaryle étant non substitués ou substitués par un groupe alkyle en C₁ à C₄, halogéno, -OR^{5c}, -C(O)N(R^{5c})₂ ou halogénoalkyle en C₁ à C₄ ;
G^{5c} représente un groupe aryle, le groupe aryle étant non substitué ou substitué par 1, 2, 3, 4 ou 5 groupes alkyle en C₁ à C₆, alcoxy en C₁ à C₆, cyano ou halogéno ; et
n, à chaque occurrence, vaut indépendamment 1, 2, 3 ou 4.

17. Composé selon la revendication 12, ou l'un de ses sels, dans lequel Z représente -NR³R⁶ ou -CH₂NR³R⁶.

18. Composé selon la revendication 1, ou l'un de ses sels, le composé étant choisi dans le groupe constitué de :
le 11-cyano-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxylate de (2*S*,4a*S*,6a*S*,6b*R*,8a*R*,12a*S*,14a*S*,14b*R*)-méthyle ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-(1,3-oxazol-5-ylméthyl)-10,14-dioxo-1,2,3,4,4a,5,6,6a,6b,7,8,8a,9,10,12a,14,14a,14b-octadécahydropicène-2-carboxamide ;
l'acide (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxylique ;
le (2*S*,4a*S*,6a*S*,6b*R*,8a*R*,12a*S*,14a*S*,14b*R*)-11-cyano-14a-hydroxy-*N*-(2-hydroxyéthyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-*N-*(pyridazin-4-ylméthyl)-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-N,2,4a,6a,6b,9,9,12a-octaméthyl-10,14-dioxo-N-propyl-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-*N*-(2,2-diméthylpropyl)-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-*N-*(pyridazin-3-ylméthyl)-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-[(1-méthyl-1*H-*pyrazol-4-yl)méthyl]-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-[2-(morpholin-4-yl)éthyl]-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-(2-méthylbenzyl)-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-[(1-méthyl-1*H-*pyrazol-3-yl)méthyl]-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-*N*-(2-méthoxybenzyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-*N*-(2-hydroxybutyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (4a*R,*6a*R,*6b*S,*8a*S,*11*S,*12a*R,*12b*S,*14b*S*)-12b-hydroxy-11-(1*H*-imidazol-1-ylcarbonyl)-4,4,6a,6b,8a,11,14b-heptaméthyl-3,13-dioxo-3,4,4a,5,6,6a,6b,7,8,8a,9,10,11,12,12a,12b,13,14b-octadécahydropicène-2-carbonitrile ;
le (2*S*,4a*S*,6a*S*,6b*R*,8a*R*,12a*S*,14a*S*,14b*R*)-11-cyano-14a-hydroxy-*N*-(2-méthoxyéthyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-(3-méthylbutyl)-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-*N-*(tetrahydro-2*H*-pyran-4-yl)-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-*N*-(2-hydroxypropyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-*N*-[(1*S*)-1-phényléthyl]-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12aS,14a*S*,14b*R*)-11-cyano-*N-*cyclohexyl-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-*N*-benzyl-11-cyano-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-*N*-(1,3-diméthoxypropan-2-yl)-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le 1-{[(2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicén-2-yl]carbonyl}prolinate de méthyle ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-10,14-dioxo-*N*-propyl-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-*N*-(2-furylméthyl)-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-*N*-(4-méthoxyphényl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-*N*-(2-méthoxyéthyl)-*N*,2,4a,6a,6b,9,9,12a-octaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-*N-*(cyanométhyl)-14a-hydroxy-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-(1,2-oxazol-5-ylméthyl)-10,14-dioxo-1,2,3,4,4a,5,6,6a,6b,7,8,8a,9,10,12a,14,14a,14b-octadécahydropicène-2-carboxamide ;
le (4a*R*,6a*R*,6b*S*,8a*S*,11*S*,12a*R*,12b*S*,14b*S*)-12b-hydroxy-4,4,6a,6b,8a,11,14b-heptaméthyl-3,13-dioxo-11-(pyrrolidin-1-ylcarbonyl)-3,4,4a,5,6,6a,6b,7,8,8a,9,10,11,12,12a,12b,13,14b-octadécahydropicène-2-carbonitrile ;
le (2*S*,4a*S*,6a*S*,6b*R*,8a*R*,12a*S*,14a*S*,14b*R*)-11-cyano-14a-hydroxy-*N*-isobutyl-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*S*,6b*R*,8a*R*,12a*S*,14a*S*,14b*R*)-11-cyano-14a-hydroxy-2,4a,6a,6b,9,9,12a-heptaméthyl-*N*-(2-méthylbutyl)-10,14-dioxo-1,2,3,4,4a,5,6,6a,6b,7,8,8a,9,10,12a,14,14a,14b-octadécahydropicène-2-carboxamide ;
le (2*S*,4a*S*,6a*R*,6b*S*,8a*R*,12a*S*,14a*R*,14b*S*)-11-cyano-*N*-(2-méthoxyéthyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*R*,6b*S*,8a*R*,12a*S*,14a*R*,14b*S*)-11-cyano-*N*-(2-furylméthyl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*R*,6b*S*,8a*R*,12a*S*,14a*R*,14b*S*)-11-cyano-*N-*cyclohexyl-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*R*,6b*S*,8a*R*,12a*S*,14a*R*,14b*S*)-11-cyano-*N*-(1,3-diméthoxypropan-2-yl)-2,4a,6a,6b,9,9,12a-heptaméthyl-10,14-dioxo-1,2,3,4,4a,5,6,6a,6b,7,8,8a,9,10,12a,14,14a,14b-octadécahydropicène-2-carboxamide ;
le (2*S*,4a*S*,6a*R*,6b*S*,8a*R*,12a*S*,14a*R*,14b*S*)-11-cyano-2,4a,6a,6b,9,9,12a-heptaméthyl-*N*-[(1-méthyl-1*H*-pyrazol-4-yl)méthyl]-10,14-dioxo-1,2,3,4,4a,5,6,6a,6b,7,8,8a,9,10,12a,14,14a,14b-octadécahydropicène-2-carboxamide ; et
le (2*S*,4a*S*,6a*R*,6b*S*,8a*R*,12a*S*,14a*R*,14b*S*)-11-cyano-2,4a,6a,6b,9,9,12a-heptaméthyl-*N*-(1,3-oxazol-5-ylméthyl)-10,14-dioxo-1,2,3,4,4a,5,6,6a,6b,7,8,8a,9,10,12a,14,14a,14b-octadécahydropicène-2-carboxamide.

19. Composé, ou l'un de ses sels, selon l'une quelconque des revendications 1 à 18, le sel étant un sel pharmaceutiquement acceptable.

20. Composition pharmaceutique comprenant une quantité thérapeutiquement efficace d'un composé selon l'une quelconque des revendications 1 à 18, ou de l'un de ses sels pharmaceutiquement acceptables, en combinaison avec un support pharmaceutiquement acceptable.

21. Composé selon l'une quelconque des revendications 1 à 18, ou l'un de ses sels pharmaceutiquement acceptables, pour une utilisation dans un procédé de traitement d'une maladie avec une composante inflammatoire chez un sujet en ayant besoin.

22. Composé selon l'une quelconque des revendications 1 à 18, ou l'un de ses sels pharmaceutiquement acceptables, pour une utilisation dans un procédé de traitement d'une affection chez un sujet en ayant besoin, l'affection étant choisie dans le groupe constitué de maladies avec une composante inflammatoire ou une composante auto-immune ; et leurs combinaisons.
